(19) **Europäisches Patentamt**
**European Patent Office**
**Office européen des brevets**

(11) **EP 1 856 144 B1**

(12) **EUROPÄISCHE PATENTSCHRIFT**

(45) Veröffentlichungstag und Bekanntmachung des Hinweises auf die Patenterteilung:
**18.07.2012 Patentblatt 2012/29**

(21) Anmeldenummer: **06707398.1**

(22) Anmeldetag: **24.02.2006**

(51) Int Cl.:
**C07K 14/435** $^{(2006.01)}$    **G01N 33/50** $^{(2006.01)}$

(86) Internationale Anmeldenummer:
**PCT/EP2006/001960**

(87) Internationale Veröffentlichungsnummer:
**WO 2006/089801 (31.08.2006 Gazette 2006/35)**

(54) **VERFAHREN ZUM REDOX-POTENTIAL-ABHÄNGIGEN NACHWEIS VON TARGETMOLEKÜLEN DURCH WECHSELWIRKENDE POLYPEPTIDE**

METHOD FOR THE REDOX POTENTIAL-DEPENDENT DETECTION OF TARGET MOLECULES BY INTERACTIVE PEPTIDES

PROCEDE POUR DETECTER, EN FONCTION DU POTENTIEL REDOX, DES MOLECULES CIBLES AU MOYEN DE POLYPEPTIDES EN INTERACTION

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **25.02.2005 EP 05090045**

(43) Veröffentlichungstag der Anmeldung:
**21.11.2007 Patentblatt 2007/47**

(73) Patentinhaber: **Forschungsverbund Berlin e.V. 12489 Berlin (DE)**

(72) Erfinder:
• **FREUND, Christian 14055 Berlin (DE)**
• **ZIMMERMANN, Jürgen Jamaica Plain, MA 02130 (US)**

(74) Vertreter: **Klages, Christlieb et al Hertin Anwaltssozietät Kurfürstendamm 54/55 D-10707 Berlin-Charlottenburg (DE)**

(56) Entgegenhaltungen:
**WO-A-01/74858    WO-A-03/048323**

• **HEUER KATJA ET AL: "Structure of a helically extended SH3 domain of the T cell adapter protein ADAP" STRUCTURE (CAMBRIDGE), vol. 12, no. 4, April 2004 (2004-04), pages 603-610, XP002386000 ISSN: 0969-2126**
• **CHEN YAN ET AL: "Overexpressed human mitochondrial thioredoxin confers resistance to oxidant-induced apoptosis in human osteosarcoma cells" JOURNAL OF BIOLOGICAL CHEMISTRY, vol. 277, no. 36, 6 September 2002 (2002-09-06), pages 33242-33248, XP002386001 ISSN: 0021-9258**

EP 1 856 144 B1

SEQUENCE LISTING

<110> Forschungsverbund Berlin e.V.

<120> Verfahren zum Redox-Potential-abhaengigen Nachweis von Targetmolekuelen durch wechselwirkende Peptide

<130> XII 849-05

<160> 138

<170> PatentIn version 3.3

<210> 1
<211> 94
<212> PRT
<213> Homo sapiens

<400> 1

```
Glu Lys Lys Glu Gln Lys Glu Lys Glu Lys Lys Glu Gln Glu Ile Lys
1               5               10              15

Lys Lys Phe Lys Leu Thr Gly Pro Ile Gln Val Ile His Leu Ala Lys
            20              25              30

Ala Cys Cys Asp Val Lys Gly Gly Lys Asn Glu Leu Ser Phe Lys Gln
            35              40              45

Gly Glu Gln Ile Glu Ile Ile Arg Ile Thr Asp Asn Pro Glu Gly Lys
        50              55              60

Trp Leu Gly Arg Thr Ala Arg Gly Ser Tyr Gly Tyr Ile Lys Thr Thr
65              70              75              80

Ala Val Glu Ile Asp Tyr Asp Ser Leu Lys Leu Lys Lys Asp
                85              90
```

<210> 2
<211> 50
<212> PRT
<213> Homo sapiens

<400> 2

```
Glu Leu Val Gln Lys Phe Gln Val Tyr Tyr Leu Gly Asn Val Pro Val
1               5               10              15

Ala Lys Pro Val Gly Val Asp Val Ile Asn Gly Ala Leu Glu Ser Val
            20              25              30

Leu Ser Ser Ser Ser Arg Glu Gln Trp Thr Pro Ser His Val Ser Val
            35              40              45

Ala Pro
        50
```

<210> 3
<211> 50
<212> PRT
<213> Homo sapiens

<400> 3

Gly Leu Ser Leu Ser Val Pro Ala Thr Arg Gln Val Ile Ala Asn His
1                5                10               15

His Met Pro Ser Ile Ser Phe Ala Ser Gly Gly Asp Thr Asp Met Thr
            20               25               30

Asp Tyr Val Ala Tyr Val Ala Lys Asp Pro Ile Asn Gln Arg Ala Cys
        35               40               45

His Ile
    50

<210> 4
<211> 50
<212> PRT
<213> Homo sapiens

<400> 4

Met Thr Leu Glu Glu Leu Val Ala Cys Asp Asn Ala Ala Gln Lys Met
1                5                10               15

Gln Thr Val Thr Ala Ala Val Glu Glu Leu Leu Val Ala Ala Gln Arg
            20               25               30

Gln Asp Arg Leu Thr Val Gly Val Tyr Glu Ser Ala Lys Leu Met Asn
        35               40               45

Val Asp
    50

<210> 5
<211> 53
<212> PRT
<213> Homo sapiens

<400> 5

Glu Asn Glu Glu Ala Glu Gly Asp Glu Ile Tyr Glu Asp Leu Met Arg
1                5                10               15

Ser Glu Pro Val Ser Met Pro Pro Lys Met Thr Glu Tyr Asp Lys Arg
            20               25               30

Cys Cys Cys Leu Arg Glu Ile Gln Gln Thr Glu Glu Lys Tyr Thr Asp
        35               40               45

Thr Leu Leu Glu Arg
    50

<210> 6
<211> 50
<212> PRT
<213> Homo sapiens

<400> 6

```
Leu Glu Glu Glu Pro Ser Val Glu Thr Glu Asp Pro Ser Pro Glu Thr
1               5               10                  15

Phe Arg Gln Leu Phe Arg Leu Phe Cys Tyr Gln Glu Val Ala Gly Pro
            20              25                  30

Arg Glu Ala Leu Ser Arg Leu Trp Glu Leu Cys Cys Arg Trp Leu Arg
            35              40                  45

Pro Glu
        50
```

<210> 7
<211> 37
<212> PRT
<213> Homo sapiens

<400> 7

```
Gln Glu Leu Cys Arg Gln Leu Phe Arg Gln Phe Cys Tyr Gln Asp Ser
1               5               10                  15

Pro Gly Pro Arg Glu Ala Leu Ser Arg Leu Arg Glu Leu Cys Cys Gln
            20              25                  30

Trp Leu Lys Pro Glu
        35
```

<210> 8
<211> 37
<212> PRT
<213> Homo sapiens

<400> 8

```
Ser Glu Ala Cys Arg Gln Arg Phe Arg Gln Phe Cys Tyr Gly Asp Val
1               5               10                  15

His Gly Pro His Glu Ala Phe Ser Gln Leu Trp Glu Leu Cys Cys Arg
            20              25                  30

Trp Leu Arg Pro Glu
        35
```

<210> 9
<211> 39
<212> PRT

26

<213> Homo sapiens

<400> 9

Cys Glu Val Phe Arg Gln Arg Phe Arg Gln Phe Gln Tyr Arg Glu Ala
1               5                   10                  15

Ala Gly Pro His Glu Ala Phe Asn Lys Leu Trp Glu Leu Cys Cys Gln
            20                  25                  30

Trp Leu Lys Pro Lys Pro His
        35

<210> 10
<211> 49
<212> PRT
<213> Homo sapiens

<400> 10

Lys Gly Leu Glu Gly Met Ile Arg Lys Arg Ser Gly Gly His Arg Val
1               5                   10                  15

Pro Gly Leu Thr Cys Cys Gly Arg Asp Gln Val Cys Tyr Arg Trp Ser
            20                  25                  30

Lys Arg Trp Leu Trp Lys Asp Ser Phe Leu Leu Tyr Met Cys Leu Glu
        35                  40                  45

Thr

<210> 11
<211> 50
<212> PRT
<213> Homo sapiens

<400> 11

Lys Gly Ile Glu Gly Met Ile Met Lys Arg Ser Gly Gly His Arg Ile
1               5                   10                  15

Pro Gly Leu Asn Cys Cys Gly Gln Gly Arg Ala Cys Tyr Arg Trp Ser
            20                  25                  30

Lys Arg Trp Leu Ile Val Lys Asp Ser Phe Leu Leu Tyr Met Lys Pro
        35                  40                  45

Asp Ser
        50

<210> 12
<211> 50
<212> PRT

EP 1 856 144 B1

<213> Homo sapiens

<400> 12

```
Ile Leu Arg Val Gly Phe Val Leu Arg Ser Ala Arg Arg Ala Pro Cys
1               5                   10                  15

Cys Arg Ile Met Val Leu Cys Ser Asp Arg Ile Leu Phe Gly His Arg
            20                  25                  30

Gly Val Asn Leu Asp Gly Asn Phe Phe Thr Val His Ala Glu Phe Lys
            35                  40                  45

Leu Lys
        50
```

<210> 13
<211> 49
<212> PRT
<213> Homo sapiens

<400> 13

```
Asn Thr Met Cys Gly Tyr Leu Lys Arg Lys Leu Arg Asn Ser Ser Gly
1               5                   10                  15

Trp Gln Asp Leu Trp Trp Met Cys Cys His Thr Leu Tyr Phe Tyr Arg
            20                  25                  30

Asn His Asn Glu Arg Glu Pro Leu Ala His Leu Ser Leu Met Asp Tyr
            35                  40                  45

Gly
```

<210> 14
<211> 50
<212> PRT
<213> Homo sapiens

<400> 14

```
Gly Trp Lys Gln Cys Cys Val Arg Tyr Leu Leu Pro Trp Ser Lys Arg
1               5                   10                  15

Trp Leu Met Val Arg Asp Ser Phe Val Ala Tyr Met Asp His Arg Thr
            20                  25                  30

Glu Gln Ile Arg Met Val Leu Leu Met Asp Arg Asp Phe Lys Val Ala
            35                  40                  45

Ala Gly
        50
```

<210> 15
<211> 50
<212> PRT
<213> Homo sapiens

<400> 15

```
Phe Ile Met Glu Gly Thr Leu Thr Arg Val Gly Ala Lys His Glu Arg
1               5               10              15

His Ile Phe Leu Phe Asp Gly Leu Met Ile Cys Cys Lys Ser Asn His
            20              25              30

Gly Gln Pro Arg Leu Pro Gly Ala Ser Ser Ala Glu Tyr Arg Leu Lys
            35              40              45

Glu Lys
        50
```

<210> 16
<211> 49
<212> PRT
<213> Homo sapiens

<400> 16

```
Val Leu Lys Gln Gly Tyr Met Met Lys Lys Gly His Arg Arg Lys Asn
1               5               10              15

Trp Thr Glu Arg Trp Phe Val Leu Lys Pro Asn Ile Ile Ser Trp Val
            20              25              30

Ser Glu Asp Leu Lys Asp Lys Lys Gly Asp Ile Leu Leu Asp Glu Asn
            35              40              45

Cys
```

<210> 17
<211> 50
<212> PRT
<213> Homo sapiens

<400> 17

Val Leu Lys Ala Gly Tyr Leu Glu Lys Arg Arg Lys Asp His Ser Phe
1               5                   10                  15

Leu Gly Phe Glu Trp Gln Lys Arg Trp Cys Ala Leu Ser Lys Thr Val
              20                  25                  30

Phe Tyr Tyr Tyr Gly Ser Asp Lys Asp Lys Gln Gln Lys Gly Glu Phe
          35              40                  45

Ala Ile
    50

<210> 18
<211> 50
<212> PRT
<213> Homo sapiens

<400> 18

Ala Leu Glu Leu Gly Thr Val Met Thr Val Phe Ser Ala Arg Lys Ser

1               5                   10                  15

Thr Pro Glu Arg Arg Thr Val Gln Met Ile Met Glu Thr Arg Gln Val
              20                  25                  30

Ala Trp Ser Lys Thr Ala Asp Lys Ile Glu Gly Phe Leu Asp Ile Met
          35              40                  45

Glu Ile
    50

<210> 19
<211> 49
<212> PRT
<213> Homo sapiens

<400> 19

Leu Gln Tyr Phe Gly Gln Leu Leu Val Trp Asp Trp Asn Val Cys Lys
1               5                   10                  15

Ala Asp Ile Glu Arg Glu Tyr His Val Tyr Leu Phe Glu Lys Ile Leu
              20                  25                  30

Leu Cys Cys Lys Glu Met Ser Thr Leu Lys Arg Gln Ala Arg Ser Ile
          35              40                  45

Ser

<210> 20

<211> 50
<212> PRT
<213> Homo sapiens

<400> 20

```
Ala Leu Glu Leu Gly Thr Val Met Thr Val Phe Ser Phe Arg Lys Ser
1               5               10              15

Thr Pro Glu Arg Arg Thr Val Gln Val Ile Met Glu Thr Arg Gln Val
            20              25              30

Ala Trp Ser Lys Thr Ala Asp Lys Ile Glu Gly Phe Leu Asp Ile Met
            35              40              45

Glu Ile
    50
```

<210> 21
<211> 50
<212> PRT
<213> Homo sapiens

<400> 21

```
Phe Ile Met Glu Gly Thr Leu Thr Arg Val Gly Ala Lys His Glu Arg
1               5               10              15

His Ile Phe Leu Phe Asp Gly Leu Met Ile Cys Cys Lys Ser Asn His
            20              25              30

Gly Gln Pro Arg Leu Pro Gly Ala Ser Asn Ala Glu Tyr Arg Leu Lys
            35              40              45

Glu Lys
    50
```

<210> 22
<211> 50
<212> PRT
<213> Homo sapiens

<400> 22

Tyr Tyr Lys Glu Arg Leu Leu Tyr Leu Glu Glu Gly Gln Lys Asp Ser
1               5               10              15

Leu Ile Asp Ser Ser Arg Val Leu Cys Cys His Gly Glu Leu Lys Asn
        20              25              30

Asn Arg Gly Val Lys Leu His Val Phe Leu Phe Gln Glu Val Leu Val
        35              40              45

Ile Thr
    50

<210> 23
<211> 50
<212> PRT
<213> Homo sapiens

<400> 23

Arg Leu Cys Glu Gly Thr Leu Phe Arg Lys Ile Ser Ser Arg Arg Arg
1               5               10              15

Gln Asp Lys Leu Trp Phe Cys Cys Leu Ser Pro Asn His Lys Leu Leu
        20              25              30

Gln Tyr Gly Asp Met Glu Glu Gly Ala Ser Pro Pro Thr Leu Glu Ser
        35              40              45

Leu Pro
    50

<210> 24
<211> 50
<212> PRT
<213> Homo sapiens

<400> 24

Asp Thr Lys His Gly Met Met Lys Phe Arg Glu Asp Arg Ser Leu Leu
1               5               10              15

Gly Leu Gly Leu Pro Ser Gly Gly Phe His Asp Arg Tyr Phe Ile Leu
        20              25              30

Asn Ser Ser Cys Leu Arg Leu Tyr Lys Glu Val Arg Ser Gln Arg Pro
        35              40              45

Trp Ser
    50

<210> 25
<211> 54

<212> PRT
<213> Homo sapiens

<400> 25

```
Leu Ser Met Gly Asp Leu Leu Leu His Thr Thr Val Ile Trp Leu Asn
1               5                   10                  15

Pro Pro Ala Ser Leu Gly Lys Trp Lys Lys Glu Pro Glu Leu Ala Ala
            20                  25                  30

Phe Val Phe Lys Thr Ala Trp Leu Val Tyr Lys Asp Gly Ser Lys Gln
            35                  40                  45

Lys Ile Leu Trp Glu Gln
        50
```

<210> 26
<211> 50
<212> PRT
<213> Homo sapiens

<400> 26

```
Gly Val Lys Leu Glu Val Asn Glu Arg Ile Leu Gly Cys Cys Thr Ser
1               5                   10                  15

Leu Met Gln Ala Ile Gln Val Leu Ile Val Ala Ser Lys Asp Leu Gln
            20                  25                  30

Arg Glu Ile Val Glu Ser Gly Arg Gly Thr Ala Ser Pro Lys Glu Phe
            35                  40                  45

Tyr Ala
    50
```

<210> 27
<211> 50
<212> PRT
<213> Homo sapiens

<400> 27

Cys Pro Ser Pro Val Leu Asn Thr Pro Trp Ile Pro Phe Gln Asn Cys
1               5                   10                  15

Cys Tyr Asn Phe Ile Ile Thr Lys Asn Arg His Met Ala Thr Thr Gln
            20              25                  30

Asp Glu Val His Thr Lys Cys Gln Lys Leu Asn Pro Lys Ser His Ile
        35              40                  45

Leu Ser
    50

<210> 28
<211> 30
<212> PRT
<213> Homo sapiens

<400> 28

Glu Asp Ala Arg Ile Met Leu Ser Arg Ala Val Glu Cys Cys Pro Thr
1               5                   10                  15

Ser Val Glu Leu Trp Leu Ala Leu Ala Arg Leu Glu Thr Tyr
            20              25                  30

<210> 29
<211> 50
<212> PRT
<213> Homo sapiens

<400> 29

Arg Phe Ile Phe Asp Val Gly Thr Arg Leu Gly Leu His Tyr Asp Thr
1               5                   10                  15

Leu Ala Thr Gly Ile Ile Tyr Phe His Arg Phe Tyr Met Phe His Ser
            20              25                  30

Phe Lys Gln Phe Pro Arg Tyr Val Thr Gly Ala Cys Cys Leu Phe Leu
            35              40                  45

Ala Gly
    50

<210> 30
<211> 50
<212> PRT
<213> Homo sapiens

<400> 30

```
Asn Phe Leu Arg Arg Ile Ser Lys Ala Asp Asp Tyr Asp Val Gln Ser
1               5               10              15

Arg Thr Leu Gly Lys Tyr Leu Leu Glu Ile Thr Ile Val Asp Tyr Lys
            20              25              30

Phe Ile Gly Met Arg Pro Ser Leu Cys Cys Ala Ser Ala Met Tyr Leu
        35              40              45

Ala Arg
    50
```

<210> 31
<211> 50
<212> PRT
<213> Homo sapiens

<400> 31

```
Met Gln Ile Ile Asp Val Ala Ser Ile Leu Gly Leu Asp Cys Asp Ile
1               5               10              15

Ser Glu His Ala Phe Gln Leu Phe Arg Asp Cys Cys Ser Ala Thr Cys
            20              25              30

Leu Arg Asn Arg Ser Val Glu Ala Leu Ala Thr Ala Cys Leu Val Gln
        35              40              45

Ala Ile
    50
```

<210> 32
<211> 50
<212> PRT
<213> Homo sapiens

<400> 32

```
Cys Phe Met Arg Arg Phe Leu Lys Ala Ala Gln Ser Glu Lys Lys Leu
1               5               10              15

Glu Leu Leu Ser Phe Phe Met Ile Glu Leu Ser Leu Val Glu Tyr Glu
            20              25              30

Met Leu Gln Phe Cys Pro Ser Met Leu Ala Ala Ala Ile Tyr Thr
        35              40              45

Ala Gln
    50
```

<210> 33

<211> 50
<212> PRT
<213> Homo sapiens

<400> 33

```
Asp Phe Ile Met Tyr Cys His Thr Arg Leu Asn Leu Ser Thr Ser Thr
1               5                   10                  15

Leu Phe Leu Thr Phe Thr Ile Leu Asp Lys Tyr Ser Ser Arg Phe Ile
            20              25                  30

Ile Lys Ser Tyr Asn Tyr Gln Leu Leu Ser Leu Thr Ala Leu Trp Ile
            35              40                  45

Ser Ser
        50
```

<210> 34
<211> 50
<212> PRT
<213> Homo sapiens

<400> 34

```
Ile Arg Ile Arg Asn Leu Cys Glu Arg Ile Gln Tyr Met Glu Gln Thr
1               5                   10                  15

Glu Arg Val Tyr Asn Val Phe Lys Gln Ile Leu Asp Gln Gln Thr Thr
            20              25                  30

Leu Phe Phe Asn Arg His Met His Gln Leu Ile Leu Cys Cys Leu Tyr
            35              40                  45

Gly Val
        50
```

<210> 35
<211> 41
<212> PRT
<213> Homo sapiens

<400> 35

```
Met Gln Arg Glu Val Trp Met Ser Val Phe Arg Tyr Leu Ser Arg Arg
1               5                   10                  15

Glu Leu Cys Glu Cys Met Arg Val Cys Lys Thr Trp Tyr Lys Trp Cys
            20              25                  30

Cys Asp Lys Arg Leu Trp Thr Lys Ile
            35              40
```

<210> 36
<211> 50
<212> PRT
<213> Homo sapiens

<400> 36

Tyr Ser Lys Trp Tyr Phe Glu Val Met Val Asp Glu Val Thr Pro Phe
1               5               10              15

Leu Thr Ala Gln Ala Thr His Leu Arg Val Gly Trp Ala Leu Thr Glu
                20              25              30

Gly Tyr Thr Pro Tyr Pro Gly Ala Gly Glu Gly Trp Gly Gly Asn Gly
        35              40              45

Val Gly
    50

<210> 37
<211> 23
<212> PRT
<213> Homo sapiens

<400> 37

Trp Ala Ser Thr Glu Gly Tyr Ser Pro Tyr Pro Gly Gly Gly Glu Glu
1               5               10              15

Trp Gly Gly Asn Gly Val Gly
        20

<210> 38
<211> 50
<212> PRT
<213> Homo sapiens

<400> 38

Met Ala Gly Lys Ser Ser Leu Phe Lys Val Ile Leu Leu Gly Asp Gly
1               5               10              15

Gly Val Gly Lys Ser Ser Leu Met Asn Arg Tyr Val Thr Asn Lys Phe
                20              25              30

Asp Thr Gln Leu Phe His Thr Ile Gly Val Glu Phe Leu Asn Lys Asp
        35              40              45

Leu Glu
    50

<210> 39

<211> 50
<212> PRT
<213> Homo sapiens

<400> 39

```
Glu Phe Val Ala Ile Ala Asp Tyr Ala Ala Thr Asp Glu Thr Gln Leu
1               5                   10                  15

Ser Phe Leu Arg Gly Glu Lys Ile Leu Ile Leu Arg Gln Thr Thr Ala
            20                  25                  30

Asp Trp Trp Trp Gly Glu Arg Ala Gly Cys Cys Gly Tyr Ile Pro Ala
        35                  40                  45

Asn His
    50
```

<210> 40
<211> 50
<212> PRT
<213> Homo sapiens

<400> 40

```
His Arg Thr Leu Leu Tyr Gly His Ala Ile Leu Leu Arg His Ser Tyr
1               5                   10                  15

Ser Gly Met Tyr Leu Cys Cys Leu Ser Thr Ser Arg Ser Ser Thr Asp
            20                  25                  30

Lys Leu Ala Phe Asp Val Gly Leu Gln Glu Asp Thr Thr Gly Glu Ala
            35                  40                  45

Cys Trp
    50
```

<210> 41
<211> 50
<212> PRT
<213> Homo sapiens

<400> 41

Lys Met Phe Val Gly Gln Val Pro Arg Thr Trp Ser Glu Lys Asp Leu
1               5                   10                  15

Arg Glu Leu Phe Glu Gln Tyr Gly Ala Val Tyr Glu Ile Asn Val Leu
            20              25                  30

Arg Asp Arg Ser Gln Asn Pro Pro Gln Ser Lys Gly Cys Cys Phe Val
        35                  40                  45

Thr Phe
    50

<210> 42
<211> 50
<212> PRT
<213> Homo sapiens

<400> 42

Arg Ile Ile Val Glu Asn Leu Phe Tyr Pro Val Thr Leu Asp Val Leu
1               5                   10                  15

His Gln Ile Phe Ser Lys Phe Gly Thr Val Leu Lys Ile Ile Thr Phe
            20              25                  30

Thr Lys Asn Asn Gln Phe Gln Ala Leu Leu Gln Tyr Ala Asp Pro Val
        35                  40                  45

Ser Ala

50

<210> 43
<211> 50
<212> PRT
<213> Homo sapiens

<400> 43

Ser Val Ile Gly Gly Asn Leu Thr Gly Ile Phe Ile His Arg Val Thr
1               5                   10                  15

Pro Gly Ser Ala Ala Asp Gln Met Ala Leu Arg Pro Gly Thr Gln Ile
            20              25                  30

Val Met Val Asp Tyr Glu Ala Ser Glu Pro Leu Phe Lys Ala Val Leu
        35                  40                  45

Glu Asp
    50

<210> 44
<211> 50
<212> PRT
<213> Homo sapiens

<400> 44

```
His Asp Phe Gly Phe Ser Val Ser Asp Gly Leu Leu Glu Lys Gly Val
1               5                   10                  15

Tyr Val His Thr Val Arg Pro Asp Gly Pro Ala His Arg Gly Gly Leu
            20                  25                  30

Gln Pro Phe Asp Arg Val Leu Gln Val Asn His Val Arg Thr Arg Asp
            35                  40                  45

Phe Asp
        50
```

<210> 45
<211> 50
<212> PRT
<213> Homo sapiens

<400> 45

```
Glu Asp Phe Gly Phe Ser Val Ala Asp Gly Leu Leu Glu Lys Gly Val
1               5                   10                  15

Tyr Val Lys Asn Ile Arg Pro Ala Gly Pro Gly Asp Leu Gly Gly Leu
            20                  25                  30

Lys Pro Tyr Asp Arg Leu Leu Gln Val Asn His Val Arg Thr Arg Asp
            35                  40                  45

                            Phe Asp
                                50
```

<210> 46
<211> 48
<212> PRT
<213> Homo sapiens

<400> 46

```
Pro Ala Ile Gly Asp Ile Cys Cys Ala Gln Phe Ser Glu Asp Asp Gln
1               5                   10                  15

Trp Tyr Arg Ala Ser Val Leu Ala Tyr Ala Ser Glu Glu Ser Val Leu
            20                  25                  30

Val Gly Tyr Val Asp Tyr Gly Asn Phe Glu Ile Leu Ser Leu Met Arg
            35                  40                  45
```

<210> 47
<211> 50
<212> PRT
<213> Homo sapiens

<400> 47

Phe Lys Ala Glu Ile Gly Gln Pro Cys Cys Ala Phe Phe Ala Gly Asp
1               5               10              15

Gly Ser Trp Tyr Arg Ala Leu Val Lys Glu Ile Leu Pro Asn Gly His
            20              25              30

Val Lys Val His Phe Val Asp Tyr Gly Asn Ile Glu Glu Val Thr Ala
        35              40              45

Asp Glu
    50

<210> 48
<211> 49
<212> PRT
<213> Homo sapiens

<400> 48

Tyr Arg Pro Arg Ile Gly Asp Ala Cys Cys Ala Lys Tyr Thr Ser Asp
1               5               10              15

Asp Phe Trp Tyr Arg Ala Val Val Leu Gly Thr Ser Asp Thr Asp Val
            20              25              30

Glu Val Leu Tyr Ala Asp Tyr Gly Asn Ile Glu Thr Leu Pro Leu Cys
        35              40              45

Arg

<210> 49
<211> 50
<212> PRT
<213> Homo sapiens

<400> 49

Lys Asp Lys Leu Lys Met Glu Val Asp Gln Leu Lys Lys Glu Val Thr
1           5               10                15

Leu Glu Arg Met Leu Val Ser Lys Cys Cys Glu Glu Val Arg Asp Tyr
            20              25              30

Val Glu Glu Arg Ser Gly Glu Asp Pro Leu Val Lys Gly Ile Pro Glu
            35              40              45

Asp Lys
        50

<210> 50
<211> 50
<212> PRT
<213> Homo sapiens

<400> 50

Met Asp Asp Arg Glu Asp Leu Val Tyr Gln Ala Lys Leu Ala Glu Gln
1           5               10                15

Ala Glu Arg Tyr Asp Glu Met Val Glu Ser Met Lys Lys Val Ala Gly
            20              25              30

Met Asp Val Glu Leu Thr Val Glu Glu Arg Asn Leu Leu Ser Val Ala
            35              40              45

Tyr Lys
        50

<210> 51
<211> 50
<212> PRT
<213> Homo sapiens

<400> 51

Asn Thr Thr Leu Arg Cys Cys Leu Ser Pro Glu Glu Asn Ala Glu Asp
1           5               10                15

Met Glu Val Arg Trp Phe Gln Ser Gln Phe Ser Pro Ala Val Phe Val
            20              25              30

Tyr Lys Gly Gly Arg Glu Arg Thr Glu Glu Gln Lys Glu Glu Tyr Arg
            35              40              45

Gly Arg
        50

<210> 52
<211> 50

<212> PRT
<213> Homo sapiens

<400> 52

```
Ser Arg Ala Pro His Met Ile Arg Asp Arg Lys Tyr His Leu Lys Thr
1               5                   10              15

Tyr Arg Gln Cys Cys Val Gly Thr Glu Leu Val Asp Trp Met Met Gln
            20              25              30

Gln Thr Pro Cys Val His Ser Arg Thr Gln Ala Val Gly Met Trp Gln
            35              40              45

Val Leu
        50
```

<210> 53
<211> 50
<212> PRT
<213> Homo sapiens

<400> 53

```
Ala Thr Leu Thr Ile Leu His Gln Gln Thr Glu Ala Val Leu Gly Glu
1               5                   10              15

Cys Arg Val Arg Phe Leu Ser Phe Leu Ala Val Gly Arg Asp Val His
            20              25              30

Thr Phe Ala Phe Ile Met Ala Ala Gly Pro Ala Ser Phe Cys Cys His
            35              40              45

Met Phe
        50
```

<210> 54
<211> 50
<212> PRT
<213> Homo sapiens

<400> 54

Leu Glu Cys Cys Glu Gly Leu Ala Gln Ser Ile Ile Ser Thr Val Gly
1                   5                   10                  15

Gln Ala Phe Glu Leu Arg Phe Lys Gln Tyr Leu His Ser Pro Pro Lys
            20                  25                  30

Val Ala Leu Pro Pro Glu Arg Leu Ala Gly Pro Glu Glu Ser Ala Trp
        35                  40                  45

Gly Asp

50

<210> 55
<211> 49
<212> PRT
<213> Homo sapiens

<400> 55

Pro Asp Ser Trp Leu Cys Leu Leu Ala Ile Asp Glu Glu Glu Glu Asp
1                   5                   10                  15

Asp Ile Ala Leu Gln Ile His Phe Thr Leu Ile Gln Ser Phe Cys Cys
            20                  25                  30

Asp Asn Asp Ile Asn Ile Val Arg Val Ser Gly Met Gln Arg Leu Ala
        35                  40                  45

Gln

<210> 56
<211> 53
<212> PRT
<213> Homo sapiens

<400> 56

Gly Ser Ile Gln Gln His Phe Leu Lys Pro Leu Gln Arg Phe Leu Lys
1                   5                   10                  15

Pro Gln Asp Ile Glu Ile Ile Phe Ile Asn Ile Glu Asp Leu Leu Arg
            20                  25                  30

Val His Thr His Phe Leu Lys Glu Met Lys Glu Ala Leu Gly Thr Pro
        35                  40                  45

Gly Ala Leu Glu Arg
        50

<210> 57

<211> 50
<212> PRT
<213> Homo sapiens

<400> 57

```
Leu Arg Thr Lys Glu Gln Ile Leu Glu Leu Leu Val Leu Glu Gln Phe
1               5               10                  15

Leu Thr Val Leu Pro Gly Glu Ile Gln Ala Arg Val Arg Glu Gln Gln
            20              25                  30

Pro Glu Ser Gly Glu Glu Ala Val Val Leu Val Glu Gly Leu Gln Arg
            35              40                  45

Lys Pro
        50
```

<210> 58
<211> 50
<212> PRT
<213> Homo sapiens

<400> 58

```
Ile His Thr Lys Glu Gln Ile Leu Glu Leu Leu Val Leu Glu Gln Phe
1               5               10                  15

Leu Thr Ile Leu Pro Gly Asp Leu Gln Ala Trp Val His Glu His Tyr
            20              25                  30

Pro Glu Ser Gly Glu Glu Ala Val Thr Ile Leu Glu Asp Leu Glu Arg
            35                  40              45

Gly Thr
        50
```

<210> 59
<211> 50
<212> PRT
<213> Homo sapiens

<400> 59

```
Leu Arg Thr Lys Glu Gln Ile Leu Glu Leu Leu Val Leu Glu Gln Phe
1               5               10              15

Leu Thr Val Leu Pro Gly Glu Ile Gln Gly Trp Val Arg Glu Gln His
            20              25              30

Pro Gly Ser Gly Glu Glu Ala Val Ala Leu Val Glu Asp Leu Gln Lys
        35              40              45

Gln Pro
    50
```

<210> 60
<211> 50
<212> PRT
<213> Homo sapiens

<400> 60

```
Met Arg Ser Lys Glu Gln Ile Leu Glu Leu Leu Val Leu Glu Gln Phe
1               5               10              15

Leu Thr Ile Leu Pro Thr Glu Ile Glu Thr Trp Val Arg Glu His Cys
            20              25              30

Pro Glu Asn Arg Glu Arg Val Val Ser Leu Ile Glu Asp Leu Gln Arg
        35              40              45

Glu Leu
    50
```

<210> 61
<211> 50
<212> PRT
<213> Homo sapiens

<400> 61

```
Gly Ala Ile Ser Phe Val Gln Leu Phe Asp Pro Gly Phe Glu Val Gln
1               5               10              15

Val Gly Lys Arg Ser Thr Glu Ala Arg His Gly Val Arg Ile Asp Thr
            20              25              30

Ser His Arg Ser Leu Ile Leu Lys Cys Ser Ser Tyr Arg Gln Ala Arg
        35              40              45

Trp Trp
    50
```

<210> 62
<211> 50
<212> PRT

<213> Homo sapiens

<400> 62

Gly Ala Ile Ala Phe Val Leu Leu Val Asp Lys Glu Phe Lys Ile Lys
1               5                   10                  15

Val Gly Lys Lys Glu Thr Glu Thr Lys Tyr Gly Ile Arg Ile Asp Asn
                20                  25                  30

Leu Ser Arg Thr Leu Ile Leu Lys Cys Asn Ser Tyr Arg His Ala Arg
            35                  40                  45

Trp Trp
        50

<210> 63
<211> 50
<212> PRT
<213> Homo sapiens

<400> 63

Gly Met Met Ile Asp Glu Gly Asp Thr Tyr Lys Val Met Asp Gly Glu
1               5                   10                  15

Asn Asn Val Ile Thr Leu His Asn Ala Asp Ile Ser Ile Val Phe Ala
                20                  25                  30

Ala Pro Asp Arg Ala Ser Trp Ile Glu Asp Ile Thr Glu Ala Ile Lys
            35                  40                  45

Asn Ala
        50

<210> 64
<211> 50
<212> PRT
<213> Homo sapiens

<400> 64

Val Gly Leu Pro Thr Val Ala Asp Lys Ile Asp Asn His Glu Asn Cys
1               5                   10                  15

Phe Lys Leu Phe Tyr Gly Ser His Thr Tyr Phe Phe Arg Thr Asp Ser
            20                  25                  30

Tyr Tyr Phe Phe Glu Arg Trp Val Asp Ser Ile Phe Gln Ala Ala Ile
            35                  40                  45

Ser Arg
        50

<210> 65
<211> 50
<212> PRT
<213> Homo sapiens

<400> 65

Gly Lys Glu Thr Glu Gly Ile Pro Thr Gly Leu Ile Ile Thr Asn Ser
1               5                   10                  15

Gln His Glu Leu His Leu Lys Cys Arg Arg Leu Gln Asp Thr Ala Thr
            20                  25                  30

Trp Lys Tyr Ile Ile Glu Gln Ala Met Gly Gly Ile Gly Asn Thr Trp
            35                  40                  45

Leu Gln
        50

<210> 66
<211> 50
<212> PRT
<213> Homo sapiens

<400> 66

Phe Phe Met Arg Lys Val Gln Ile Asn Asp Lys Asp Asp Thr Ser Glu
1               5                   10                  15

Tyr Lys His Ala Phe Glu Ile Ile Leu Lys Asp Gly Asn Ser Val Ile
            20                  25                  30

Phe Ser Ala Lys Ser Ala Glu Glu Lys Asn Asn Trp Met Ala Ala Leu

                    35              40                      45

Ile Ser
        50

<210> 67
<211> 48
<212> PRT
<213> Homo sapiens

<400> 67

Cys Val Glu Ser Leu Pro Asp Lys Asp Gly Lys Lys Cys Leu Phe Leu
1               5               10              15

Val Lys Cys Phe Asp Lys Thr Phe Glu Ile Ser Ala Ser Asp Lys Lys
            20              25              30

Lys Lys Gln Glu Trp Ile Gln Ala Ile His Ser Thr Ile His Leu Leu
        35              40              45

<210> 68
<211> 50
<212> PRT
<213> Homo sapiens

<400> 68

Asp Gly Tyr Ser Val Arg Met Asn Asn Thr Leu Arg Lys Asp Gly Lys
1               5               10              15

Lys Asp Cys Cys Phe Glu Ile Ser Ala Pro Asp Lys Arg Ile Tyr Gln
            20              25              30

Phe Thr Ala Ala Ser Pro Lys Asp Ala Glu Glu Trp Val Gln Gln Leu
        35              40              45

Lys Phe
    50

<210> 69
<211> 50
<212> PRT
<213> Homo sapiens

<400> 69

Lys Glu Ile Arg Pro Gly Lys Asn Ser Lys Asp Phe Glu Arg Ala Lys
1               5               10              15

Ala Val Arg His Lys Ala Asp Cys Cys Phe Thr Ile Phe Tyr Gly Thr
            20              25              30

Gln Phe Val Leu Ser Thr Leu Ser Leu Ala Thr Asp Ser Lys Glu Asp
        35              40              45

                            Ala Val
                            50

<210> 70
<211> 49
<212> PRT
<213> Homo sapiens

<400> 70

Met Asn Lys Lys Thr Lys Arg Leu Asp Ser Leu Gln Leu Lys Gly Arg
1               5                   10                  15

Ile Leu Thr Ser Asn Ile Thr Thr Trp Pro Asn His His Met Gly Ser
            20                  25                  30

Tyr Ala Ile Gln Ile Phe Trp Arg Gly Asp Pro Gln His Glu Ser Phe
            35                  40                  45

Ile

<210> 71
<211> 50
<212> PRT
<213> Homo sapiens

<400> 71

Lys Glu Ile Arg Pro Gly Lys Asn Ser Lys Asp Phe Glu Arg Ala Lys
1               5                   10                  15

Ala Val Arg Gln Lys Glu Asp Cys Cys Phe Thr Ile Leu Tyr Gly Thr
            20                  25                  30

Gln Phe Val Leu Ser Thr Leu Ser Leu Ala Ala Asp Ser Lys Glu Asp
            35                  40                  45

Ala Val
        50

<210> 72
<211> 50
<212> PRT
<213> Homo sapiens

<400> 72

Phe Phe Met Arg Lys Val Gln Ile Asn Asp Lys Asp Asp Thr Asn Glu
1               5                   10                  15

Tyr Lys His Ala Phe Glu Ile Ile Leu Lys Asp Glu Asn Ser Val Ile
            20                  25                  30

Phe Ser Ala Lys Ser Ala Glu Glu Lys Asn Asn Trp Met Ala Ala Leu
            35                  40                  45

Ile Ser
    50

<210> 73
<211> 50
<212> PRT
<213> Homo sapiens

<400> 73

```
Arg Ala Val Thr His Asn Glu Gln Leu Cys Tyr Gln Leu Tyr Arg Gln
1               5                   10                  15

Pro Ile Pro Val Lys Asp Leu Leu Leu Glu Asp Leu Gln Asp Gly Glu
            20                  25                  30

Val Arg Leu Gly Gly Ser Leu Arg Gly Ala Phe Ser Asn Asn Glu Arg
            35                  40                  45

Ile Lys
        50
```

<210> 74
<211> 50
<212> PRT
<213> Homo sapiens

<400> 74

```
Glu Gln Leu Pro Val Ala Asp Met Arg Ala Leu Leu Thr Gly Lys Asp
1               5                   10                  15

Cys Pro His Val Arg Glu Lys Gly Ser Gly Lys Gln Asn Lys Asp Leu
            20                  25                  30

Tyr Glu Leu Ala Phe Ser Ile Ser Tyr Asp Arg Gly Glu Glu Glu Ala
            35                  40                  45

Tyr Leu
        50
```

<210> 75
<211> 49
<212> PRT
<213> Homo sapiens

<400> 75

```
Gly Ala Pro Glu Thr Ser His Arg Pro Glu Lys Glu Trp Pro Ile Lys
1               5                   10                  15

Ser Leu Lys Val Tyr Leu Gly Val Lys Lys Lys Leu Arg Pro Pro Thr
            20                  25                  30

Cys Trp Gly Phe Thr Trp His Glu Thr Glu Lys His Glu Lys Gln Gln
            35                  40                  45

Trp
```

<210> 76

<210> 50

<212> PRT

<213> Homo sapiens

<400> 76

```
Lys Lys Leu Val Gly Ser His Arg Leu Ser Ile Tyr Glu Asp Trp Asp
1               5                   10                  15

Pro Phe Arg Phe Arg His Met Ile Pro Thr Glu Ala Leu Gln Val Arg
            20                  25                  30

Ala Leu Ala Ser Ala Asp Ala Glu Ala Asn Ala Val Cys Glu Ile Val
            35                  40                  45

His Val
        50
```

<210> 77

<211> 50

<212> PRT

<213> Homo sapiens

<400> 77

```
Lys Asn Ser Arg Trp Thr Glu Gly Leu Ile Ser Ala Ser Lys Ala Val
1               5                   10                  15

Gly Trp Gly Ala Thr Val Met Val Asp Ala Ala Asp Leu Val Val Gln
            20                  25                  30

Gly Arg Gly Lys Phe Glu Glu Leu Met Val Cys Ser His Glu Ile Ala
            35                  40                  45

Ala Ser
        50
```

<210> 78

<211> 50

<212> PRT

<213> Homo sapiens

<400> 78

```
Ile Arg Asp Glu Lys Glu Asn Asn Phe Val Leu Glu Gln Leu Leu Tyr
1               5                   10                  15

Phe Asn Tyr Met Ala Ser Trp Val Met Leu Gly Ile Thr Tyr Arg Asn
            20                  25                  30

Asn Ser Leu Met Trp Phe Asp Lys Thr Pro Leu Ser Tyr Thr His Trp
```

35      40      45

Arg Ala
50

<210> 79
<211> 45
<212> PRT
<213> Homo sapiens

<400> 79

Lys Val Glu Glu Thr Pro Lys Lys Cys Lys Asp Ile Ile Lys Thr Ala
1      5      10      15

Arg Ser Leu Leu Asn Asp Val Gln Phe Gly Gln Phe Gly Asp Asp Pro
    20      25      30

Lys Glu Glu Val Met Val Leu Glu Arg Ile Leu Leu Gln
    35      40      45

<210> 80
<211> 34
<212> PRT
<213> Homo sapiens

<400> 80

Leu Ile Leu Gly Lys Leu Pro Val Trp Asn Gly Asn Leu Ile His Tyr
1      5      10      15

Ser Gly Gly Tyr Arg Ile Ser Asp Met Arg Glu Cys Ile Glu Leu Met
    20      25      30

Phe Gln

<210> 81
<211> 32
<212> PRT
<213> Homo sapiens

<400> 81

Arg Glu Ala Gln Glu Pro Arg Thr Leu Gln Glu Ile Ser Ile Ala Ala
1      5      10      15

Asn Val Gln Gln Lys Glu Ile Gly Lys Tyr Ile Lys Ile Leu Gly Glu
    20      25      30

<210> 82
<211> 33
<212> PRT
<213> Homo sapiens

<400> 82

Cys Thr Ile Asn Gly Phe Lys Ser Trp Asn Lys Cys Cys Glu Leu His

1 5 10 15

Thr Arg Tyr Ser Glu Glu His Leu Met Val Cys Ser Arg Met Met Val
20 25 30

Glu

<210> 83
<211> 37
<212> PRT
<213> Homo sapiens

<400> 83

Lys Phe Trp Asp Ser Lys Asn Arg Met Ala Thr Leu Lys Val Leu Gln
1 5 10 15

Asn Leu Cys Cys Asn Gln Tyr Ser Ile Lys Gln Phe Thr Thr Met Glu
20 25 30

Met His Leu Phe Lys
35

<210> 84
<211> 50
<212> PRT
<213> Homo sapiens

<400> 84

Ala Lys Val Cys Gln Leu Glu Leu Ser Phe Arg Glu Ile Leu Asn Asn
1 5 10 15

Tyr Lys Lys Glu Ala Gln Cys Lys Pro Glu Val Phe Leu Ser Ile Tyr
20 25 30

Ile Gly Ser Arg Asn His Asn Gly Val Leu Ile Ser Arg His Val Asp
35 40 45

Ile Ile
50

<210> 85
<217> 50
<212> PRT
<213> Homo sapiens

<400> 85

Asp Asp Leu Tyr Ser Tyr Gly Phe Asp Gly Leu His Leu Trp Thr Gly
1 5 10 15

His Val Ala Arg Pro Val Thr Ser Pro Gly Gln His Leu Leu Ala Pro
20 25 30

Glu Asp Val Ile Ser Cys Cys Leu Asp Leu Ser Val Pro Ser Ile Ser
35 40 45

Phe Arg
50

<210> 86
<211> 50
<212> PRT
<213> Homo sapiens

<400> 86

Asp Asp Leu Phe Ser Tyr Gly Phe Asp Gly Leu His Leu Trp Ser Gly
1 5 10 15

Cys Ile Ala Arg Thr Val Ser Ser Pro Asn Gln His Leu Leu Arg Thr
20 25 30

Asp Asp Val Ile Ser Cys Cys Leu Asp Leu Ser Ala Pro Ser Ile Ser
35 40 45

Phe Arg
50

<210> 87
<211> 50
<212> PRT
<213> Homo sapiens

<400> 87

Val Asp Gly His Phe Val Thr Met Gln Ile Trp Asp Thr Ala Gly Gln
1 5 10 15

Glu Arg Phe Arg Ser Leu Arg Thr Pro Phe Tyr Arg Gly Ser Asp Cys
20 25 30

Cys Leu Leu Thr Phe Ser Val Asp Asp Ser Gln Ser Phe Gln Asn Leu
35 40 45

Ser Asn
50

<210> 88
<211> 5

<212> PRT
<213> Homo sapiens

<400> 88

```
Val Gly Lys His Val
1               5
```

<210> 89
<211> 6
<212> PRT
<213> Homo sapiens

<400> 89

```
Trp Thr Ile His Pro Ala
1               5
```

<210> 90
<211> 29
<212> PRT
<213> Homo sapiens

<400> 90

```
Tyr Thr Arg Lys Ala Ala Leu Glu Ala Gln Asn Ala Leu His Asn Met
1               5                   10                  15

Lys Val Leu Pro Gly Met His His Pro Ile Gln Met Lys
            20                  25
```

<210> 91
<211> 20
<212> PRT
<213> Homo sapiens

<400> 91

```
Gln His Ala Lys Leu Ser Leu Asp Gly Gln Asn Ile Tyr Asn Ala Cys
1               5                   10                  15

Cys Thr Leu Arg
            20
```

<210> 92
<211> 25
<212> PRT
<213> Homo sapiens

<400> 92

```
Thr Thr Leu Glu Glu Ala Val Gly Leu Leu Arg Arg Val Asp Gly Phe
1               5                   10                  15

Cys Cys Leu Ser Val Lys Val Asn Thr
            20                  25
```

<210> 93
<211> 23
<212> PRT
<213> Homo sapiens

<400> 93

```
Cys Cys Leu Ala Val Pro Leu Leu Ala Glu Ala Gly Asp Val Leu Glu
1               5                   10                  15

Leu Ile Ile Ser Arg Lys Pro
            20
```

<210> 94
<211> 23
<212> PRT
<213> Homo sapiens

<400> 94

```
Cys Cys Leu Val Val Pro Leu Ile Ala Glu Ser Gly Asn Lys Leu Asp
1               5                   10                  15

Leu Val Ile Ser Arg Asn Pro
            20
```

<210> 95
<211> 9
<212> PRT
<213> Homo sapiens

<400> 95

```
Leu Cys Pro Ile Ile Pro Lys Leu Leu
1               5
```

<210> 96
<211> 9
<212> PRT
<213> Homo sapiens

<400> 96

```
Leu Arg Met Ile Ser Ser Thr Phe Leu
1               5
```

<210> 97
<211> 9

57

<212> PRT
<213> Homo sapiens

<400> 97

```
Val Gln Pro Ile Thr Ser Ser His Leu
1               5
```

<210> 98
<211> 13
<212> PRT
<213> Homo sapiens

<400> 98

```
Asn Pro Phe Lys Glu Leu Lys Gly Gly Cys Val Ile Ser
1               5               10
```

<210> 99
<211> 50
<212> PRT
<213> Homo sapiens

<400> 99

```
Asn Val Ile Gly Ala Arg Arg Ala Ser Trp Arg Ile Ile Ser Ser Ile
1               5               10              15

Glu Gln Lys Glu Glu Asn Lys Gly Gly Glu Asp Lys Leu Lys Met Ile
            20              25              30

Arg Glu Tyr Arg Gln Met Val Glu Thr Glu Leu Lys Leu Ile Cys Cys
            35              40              45

Asp Ile
        50
```

<210> 100
<211> 31
<212> PRT
<213> Homo sapiens

<400> 100

```
Thr Thr Phe Val Ser Lys Asp Ser Arg Gly Ser Val Ala Leu Ile Ile
1               5               10              15

His Asn Val Thr Ala Glu Asp Asn Gly Ile Tyr Gln Cys Tyr Phe
            20              25              30
```

<210> 101
<211> 26
<212> PRT

<213> Homo sapiens

<400> 101

```
Leu Glu Asp Gly Val Leu Asn His Val Asp Gln Glu His His Phe Gln
1               5                10                15

Asp Lys Tyr Leu Phe Tyr Arg Phe Leu Asp
            20              25
```

<210> 102
<211> 30
<212> PRT
<213> Homo sapiens

<400> 102

```
Trp Cys Glu Pro Asn Ala Ala Ser Leu Ser Glu Ala Val Gln Ala Ala
1               5                10                15

Cys Met Leu Arg Tyr Gln Lys Cys Leu Asp Ala Arg Ser Gln
            20              25              30
```

<210> 103
<211> 50
<212> PRT
<213> Homo sapiens

<400> 103

```
Glu Glu Asp Ser Leu Glu His Asn Tyr Tyr Asn Ser Ile Pro Gly Lys
1               5                10                15

Glu Pro Pro Leu Gly Gly Leu Val Asp Ser Arg Leu Ala Leu Thr Gln
            20              25              30

Pro Cys Ala Leu Thr Ala Leu Asp Gln Gly Pro Ser Pro Ser Leu Arg
            35              40              45

Asp Ala
    50
```

<210> 104
<211> 50
<212> PRT
<213> Homo sapiens

<400> 104

```
Leu Leu Gly Glu Pro Ala Glu Thr Gln Gly Thr Thr Glu Ala Arg Asp
1               5                   10              15

Leu His Cys Leu Leu Val Thr Met Pro His Thr Asp Ala Trp Lys Ser
            20              25              30

His Gly Leu Val Glu Val Ala Ser Tyr Cys Glu Glu Ser Arg Gly Asn
            35              40              45

Asn Gln
        50
```

<210> 105
<211> 50
<212> PRT
<213> Homo sapiens

<400> 105

```
Ala Asn Leu Tyr Gln Val Phe Ile Lys Tyr Lys Glu Arg Phe Leu Val
1               5                   10              15

Tyr Gly Arg Tyr Cys Ser Gln Val Glu Ser Ala Ser Lys His Leu Asp
            20              25              30

Arg Val Ala Ala Ala Arg Glu Asp Val Gln Met Lys Leu Glu Glu Cys
            35              40              45

Ser Gln
        50
```

<210> 106
<211> 7
<212> PRT
<213> Homo sapiens

<400> 106

```
Arg Lys His Arg Gln Arg Gly
        1               5
```

<210> 107
<211> 26
<212> PRT
<213> Homo sapiens

<400> 107

```
Asp Glu Ala Val Leu Gln Val Gln Ala His Glu His Gly Gln Glu Ile
1               5                   10                      15

Phe Gln Lys Lys Val Ser Pro Pro Gly Pro
            20                  25
```

<210> 108
<211> 18
<212> PRT
<213> Homo sapiens

<400> 108

```
Val Lys Ala Trp Arg Gln Asp Val Pro Ser Glu Glu Ala Glu Pro Glu
1               5                   10                      15

Ala Ala
```

<210> 109
<211> 22
<212> PRT
<213> Homo sapiens

<400> 109

```
Glu Ile Pro Glu Gln Gln Val Asp Met His Asp Ile Leu Leu Glu Glu
1               5                   10                      15

Leu Ala Pro Val Gly Thr
            20
```

<210> 110
<211> 11
<212> PRT
<213> Homo sapiens

<400> 110

```
Ala Gln Glu Ile Thr Glu Leu Ala Gln Gly Pro
1               5                   10
```

<210> 111
<211> 11
<212> PRT
<213> Homo sapiens

<400> 111

```
Gly Gly Ala Ile Glu Glu Phe Ile Gln Lys His
1               5                   10
```

<210> 112

<211> 11
<212> PRT
<213> Homo sapiens

<400> 112

```
        Pro His Arg Phe Ser Ser Ser Phe Pro Val Arg
        1               5               10
```

<210> 113
<211> 5
<212> PRT
<213> Homo sapiens

<400> 113

```
        Leu Gln Tyr Arg Ser
        1               5
```

<210> 114
<211> 5
<212> PRT
<213> Homo sapiens

<400> 114

```
        Val Leu Gln Asp Met
        1               5
```

<210> 115
<211> 13
<212> PRT
<213> Homo sapiens

<400> 115

```
        Lys Trp Leu Ser Gly Leu Lys Ile Leu His Gln Glu Ala
        1               5                   10
```

<210> 116
<211> 23
<212> PRT
<213> Homo sapiens

<400> 116

```
        Leu Lys Leu Arg Asn Glu Glu Ser His Lys Leu Trp Met Ser Val Leu
        1               5               10              15

        Asn Arg Leu Leu Trp Lys Asn
                    20
```

<210> 117
<211> 13

<212> PRT
<213> Homo sapiens

<400> 117

```
Asn Trp Leu Ser Gly Leu Lys Ile Leu His Gln Glu Ala
1                   5                   10
```

<210> 118
<211> 5
<212> PRT
<213> Homo sapiens

<400> 118

```
Leu Gln Tyr Arg Ser
1               5
```

<210> 119
<211> 40
<212> PRT
<213> Homo sapiens

<400> 119

```
Asn Phe Phe Arg Val Ser Phe Lys Asn Gly Ser Gln Ser Gln Thr His
1               5                   10                  15

Ser Leu Gln Ala Asn Asp Thr Phe Asn Lys Gln Gln Trp Leu Asn Cys
            20                  25                  30

Ile Arg Gln Ala Lys Glu Thr Val
            35                  40
```

<210> 120
<211> 24
<212> PRT
<213> Homo sapiens

<400> 120

```
Asn Phe Ile Ala Pro Ser Lys Arg Glu Phe Tyr Leu Trp Thr Asp Gly
1               5                   10                  15

Leu Ser Ala Leu Leu Gly Ser Pro
            20
```

<210> 121
<211> 24
<212> PRT
<213> Homo sapiens

<400> 121

```
Tyr Leu Cys Cys Asp Thr Gln Met Glu Leu Arg Glu Trp Phe Ala Thr
1               5                   10              15

Phe Leu Phe Val Gln His Asp Gly
            20
```

<210> 122
<211> 36
<212> PRT
<213> Homo sapiens

<400> 122

```
Lys Ser Glu Ser Glu Gly Arg Pro Glu Arg Val Phe His Leu Cys Cys
1               5                   10              15

Ser Ser Pro Glu Ser Arg Lys Asp Phe Leu Lys Ala Val His Ser Ile
            20                  25                  30

Leu Arg Asp Lys
            35
```

<210> 123
<211> 49
<212> PRT
<213> Homo sapiens

<400> 123

```
Thr Ala Gln Leu Val Ala Ala Ser Lys Val Lys Ala Asp Lys Asp Ser
1               5                   10              15

Pro Asn Leu Ala Gln Leu Gln Gln Ala Ser Arg Gly Val Asn Gln Ala
            20                  25                  30

Thr Ala Gly Val Val Ala Ser Thr Ile Ser Gly Lys Gln Ile Glu Glu
            35                  40                  45

Thr
```

<210> 124
<211> 43
<212> PRT
<213> Homo sapiens

<400> 124

```
Gly Arg Pro Thr Ile Lys Asn Glu Lys Phe Leu Ala Gly Leu Ser Thr
1               5               10              15

Asp Gly Phe Trp Asp Ile Gln Thr Phe Lys Val Ile Glu Glu Ala Val
            20              25              30

Tyr Phe His Gln His Ser Ile Leu Ala Cys Lys
        35              40
```

<210> 125
<211> 8
<212> PRT
<213> Homo sapiens

<400> 125

```
Thr Phe Tyr Asn Glu Val Phe Val
1               5
```

<210> 126
<211> 38
<212> PRT
<213> Homo sapiens

<400> 126

```
Ile Asn Gly Cys Pro Val Gln Gly Val Phe Glu Ser Phe Asn Leu Asp
1               5               10              15

Gly Leu Phe Phe Pro Val Val Ser Phe Ser Ala Gly Val Lys Val Arg
            20              25              30

Phe Leu Leu Gly Gly His
        35
```

<210> 127
<211> 39
<212> PRT
<213> Homo sapiens

<400> 127

```
Ile Asn Gly Gln Pro Val Gln Gly Met Phe Glu Asn Phe Asn Ile Asp
1               5               10              15

Gly Leu Phe Phe Pro Val Val Ser Phe Ser Ala Gly Ile Lys Val Arg
            20              25              30

Phe Leu Leu Gly Gly Arg His
        35
```

<210> 128
<211> 50
<212> PRT
<213> Homo sapiens

<400> 128

```
Trp Lys Lys Glu Phe Ile Tyr Tyr Ala Asp Val Lys Glu Pro Glu Ser
1               5               10              15

Phe Pro Phe Val Ile Leu Gly Asn Lys Ile Asp Ile Ser Glu Arg Gln
            20              25              30

Val Ser Thr Glu Glu Ala Gln Ala Trp Cys Arg Asp Asn Gly Asp Tyr
            35              40              45

Pro Tyr
        50
```

<210> 129
<211> 50
<212> PRT
<213> Homo sapiens <400> 129

```
Leu Asp Val Leu Asp Lys His Leu Ile Pro Ala Ala Asn Thr Gly Glu
1               5               10              15

Ser Lys Val Phe Tyr Tyr Lys Met Lys Gly Asp Tyr His Arg Tyr Leu
            20              25              30

Ala Glu Phe Ala Thr Gly Asn Asp Arg Lys Glu Ala Ala Glu Asn Ser
            35              40              45

Leu Val
        50
```

<210> 130
<211> 10
<212> PRT
<213> Homo sapiens

<400> 130

```
Trp Val Pro Tyr Ile Ser Leu Gln Glu Arg
1               5               10
```

<210> 131
<211> 50
<212> PRT
<213> Homo sapiens

<400> 131

```
Arg Ala Asn Asn Gly Arg Phe Thr Leu Arg Asp Leu Leu Met Val Pro
1               5                   10                  15
```

```
Met Gln Arg Val Leu Lys Tyr His Leu Leu Leu Gln Glu Leu Val Lys
            20                  25                  30
```

```
His Thr Gln Glu Ala Met Glu Lys Glu Asn Leu Arg Leu Ala Leu Asp
            35                  40                  45
```

```
Ala Met
    50
```

<210> 132
<211> 14
<212> PRT
<213> Homo sapiens

<400> 132

```
Cys Ser Leu Pro Trp Asp Val Gly Ser Thr Gly Thr Ala Pro
1               5                   10
```

<210> 133
<211> 49
<212> PRT
<213> Homo sapiens

<400> 133

```
Asp Asn Met Asp Phe Ser Ser Met Thr Leu Thr Gln Ile Lys Arg Gln
1               5                   10                  15
```

```
Glu Met Asp Ser Gln Val Arg Val Leu Glu Leu Glu Asn Glu Leu Gln
            20                  25                  30
```

```
Lys Glu Arg Gln Lys Leu Gly Glu Leu Arg Lys Lys His Tyr Glu Leu
            35                  40                  45
```

```
Ala
```

<210> 134
<211> 25
<212> PRT
<213> Homo sapiens

<400> 134

```
Phe Glu Thr Ser Ala Lys Asp Ala Thr Asn Val Ala Ala Ala Phe Glu
1               5                   10                  15
```

```
Glu Ala Val Arg Arg Val Leu Ala Thr
            20                  25
```

<210> 135
<211> 50
<212> PRT
<213> Homo sapiens

<400> 135

Ala Tyr Lys Ala Ala Ser Asp Ile Ala Met Thr Glu Leu Pro Pro Thr
1               5                   10                  15

His Pro Ile Arg Leu Gly Leu Ala Leu Asn Phe Ser Val Phe Tyr Tyr
            20                  25                  30

Glu Ile Leu Asn Ser Pro Asp Arg Ala Cys Arg Leu Ala Lys Ala Ala
            35                  40                  45

Phe Asp
    50

<210> 136
<211> 17
<212> PRT
<213> Homo sapiens

<400> 136

Arg Asp Leu Ala Gln Cys Val Asn Glu Val Lys Arg Asp Asn Glu Thr
1               5                   10                  15

Leu

<210> 137
<211> 45
<212> PRT
<213> Homo sapiens

<400> 137

Asp Ala Ile Ala Glu Leu Asp Thr Leu Ser Glu Glu Ser Tyr Lys Asp
1               5                   10                  15

Ser Thr Leu Ile Met Gln Leu Leu Arg Asp Asn Leu Thr Leu Trp Thr
            20                  25                  30

Ser Asp Met Gln Gly Asp Gly Glu Glu Gln Asn Lys Glu
            35                  40                  45

<210> 138
<211> 82
<212> PRT
<213> Homo sapiens

<400> 138

```
Ser Phe Ser His Leu Glu Gly Leu Leu Gln Glu Ala Gly Pro Ser Glu
1              5                  10          15

Ala Cys Cys Val Arg Asp Val Thr Glu Pro Gly Ala Leu Arg Met Glu
            20              25              30

Thr Gly Asp Pro Ile Thr Val Ile Glu Gly Ser Ser Ser Phe His Ser
        35              40              45

Pro Asp Ser Thr Ile Trp Lys Gly Gln Asn Gly Arg Thr Phe Lys Val
    50              55              60

Gly Ser Phe Pro Ala Ser Ala Val Thr Leu Ala Asp Ala Gly Gly Leu
65              70              75              80

Pro Ala
```

## Beschreibung

Die Erfindung betrifft bevorzugt eine Aminosäuresequenz EKKEQKEKEK KEQEIKKKFK LTGPIQVIHL AKACCDVKGG KNELSFKQGE QIEIIRITDN PEGKWLGRTA RGSYGYIKTT AVEIDYDSLK LKKD (= SEQ ID Nr. 1), eine Proteinerkennungsdomäne umfassend diese Aminosäuresequenz und die Verwendung der Aminosäuresequenz oder der Proteinerkennungsdomäne zur Identifizierung von Redox-abhängigen Protein-Protein- oder Protein-Lipid-Wechselwirkungen und ein Verfahren zur Detektion von Target-Molekülen (Moleküle, die von SEQ ID Nr. 1 gebunden werden, wobei sich die Angabe SEQ ID Nr. 1 auf oben genannte Aminosäuresequenz bezieht) in Abhängigkeit vom Redoxpotential; die Erfindung betrifft zudem die Verwendung der Aminosäuresequenz als Marker zur Messung des Redoxpotentials in Zellen; weiterhin betrifft die Erfindung die Verwendung der Aminsäuresequenz für den Transport und die Redox-abhängige Freisetzung von an die Seitenkette der Cysteine von SEQ ID Nr. 1 gebundenen Molekülen; die Erfindung betrifft auch die Verwendung der Aminosäuresequenz zur Herstellung einer Aminosäuresequenz-/Polypeptidbibliothek; weiterhin betrifft die Erfindung einen Kit, der die Aminosäuresequenz bzw. die Proteinerkennungsdomäne umfasst; ausserdem betrifft die Erfindung Antikörper gegen Aminosäuresequenzen von SEQ ID Nr. 1 oder der Polypeptidbibliothek, sowie die Verwendung der Aminosäuresequenz in pharmazeutischen Formulierungen.

Oxidativer Stress und ein verändertes Redox-Gleichgewicht in Organismen, insbesondere in Zellen, sind ein wichtiger Indikator und wesentlicher Risikofaktor für zahlreiche Krankheits- und Alterungsprozesse. So ist beispielsweise beschrieben, dass bei altersbedingten neurodegenerativen Erkrankungen, wie z.B. Alzheimer, reaktive Sauerstoffspezien eine herausragende Bedeutung spielen. Weiterhin ist bekannt, dass viele entzündungshemmende Medikamente z.B. für Autoimmunerkrankungen als Antioxidantien wirken. Chronisch entzündliche Erkrankungen gehen oft mit einer Dysfunktion Redox-abhängiger-Prozesse einher, z.B. rheumatoide Arthritis, Hautkrankheiten wie Psoriasis und Hautkrebs, Multiple Sklerose, Tuberkulose und chronische Lungen- und Atemwegserkrankungen wie etwa Asthma pheumoconiosis, nasale Polypen und Lungen-Fibrose.

Es ist außerdem im Stand der Technik offenbart, dass in vielen Tumoren ein prooxidatives Milieu vorherrscht, welches die entarteten Zellen vor extrazellulären Zelltod-Signalen schützt.

Auch in HIV-infizierten Individuen kommt es zum systemischen oxidativen Stress. Daher stellt die Redox-Kontrolle eine wichtige therapeutische Strategie bei HIV-Erkrankten dar. Oxidativer Stress ist auch ein Indikator bei chronischen Nierenleiden.

Hieraus folgt, dass bei allen genannten Erkrankungen Redox-sensitive Inhibitoren als pharmazeutische Mittel zur Diagnose oder Therapie einsetzbar wären, sofern sie zur Verfügung stünden. Aber trotz der Fülle an Beispielen, die die Bedeutung von Redoxvorgängen in Krankheitsprozessen belegen, gibt es bisher keine spezifisch wirkenden Medikamente, die in ihrer Wirkung sensitiv für den Redoxzustand eines Gewebes sind und nur unter pathologischen Redox-Bedingungen wirken. Dies liegt zum einen an der noch mangelnden Kenntnis und Charakterisierung der am Redox-Signaling beteiligten Komponenten, aber auch an der Schwierigkeit, Redox-abhängige Makromoleküle wie Peptide, Proteine aber auch Lipid- bzw. Kohlenhydratstrukturen spezifisch zu inhibieren.

Aufgabe der Erfindung war es daher, ein Polypeptid bzw. eine Aminosäuresequenz bereitzustellen, mit dem Targetmoleküle, deren Inhibition Redox-Potential-abhängig schaltbar ist, detektiert werden können und die weiterhin zur Identifizierung von Redox-abhängigen Protein-Protein- oder Protein-Lipid-Wechselwirkungen eingesetzt werden können; Aufgabe der Erfindung war es weiterhin, Sonden oder Marker bereitzustellen, die mit den Targetmolekülen spezifisch wechselwirken.

Die Erfindung löst dieses Problem durch die Bereitstellung eines Polypeptids umfassend ein Cys-Cys-Motiv aufweisend ein reversibel einstellbares Redox-Potential im Bereich von -400 mV bis +200 mV, bevorzugt im Bereich -300 mV bis 0 mV, noch mehr bevorzugt im Bereich -300 mV bis -150 mV einsetzbar insbesondere zur Detektion von Redox-abhängigen Protein-Protein- oder Protein-Lipid-Wechselwirkungen und/oder zur Detektion von Molekülen, die in Abhängigkeit vom Redox-Potential von dem Polypeptid gebunden werden können, bestehend aus der Aminosäuresequenz gemäß SEQ ID Nr. 1. Erfindungsgegenstand sind demgemäß insbesondere die offenbarten Peptide bzw. Polypeptide oder Aminosäuresequenzen sowie ihre Verwendung. Die o. g. Verwendungsmöglichkeiten sollen weniger zweckgebundene Stoffe offenbaren, sondern zunächst eine einzige allgemeine erfinderische Idee, die zwischen den offenbarten Peptiden einen technischen Zusammenhang herstellt. Die offenbarten Peptide zeigen gemeinsame Eigenschaften bzw. Wirkungen. Der funktionelle Zusammenhang ist das reversibel einstellbare Redox-Potential im Bereich von -400 mV bis +200 mV, einsetzbar insbesondere zur Detektion von Redox-abhängigen Protein-Protein- oder Protein-Lipid-Wechselwirkungen und/oder zur Detektion von Molekülen, die in Abhängigkeit vom Redox-Potential von dem Polypeptid gebunden werden. Selbstverständlich ist aber auch die Verwendung der Peptide in der Medizin beansprucht (zweckgebundener Stoffanspruch für die erste medizinische Indikation), wie auch die Verwendung in der Forschung und für weitere medizinische Anwendungen.

Die Erfindung löst das o. g. Problem in einer bevorzugten Ausführungsform, bevorzugt durch die Bereitstellung einer Aminosäuresequenz EKKEQKEKEK KEQEIKKKFK LTGPIQVIHL AKACCDVKGG KNELSFKQGE QIEIIRITDN PEGKWLGRTA RGSYGYIKTT AVEIDYDSLK LKKD (SEQ ID Nr. 1) oder einer Nukleinsäuresequenz, die für die genannte Aminosäuresequenz codiert.

Vorteilhafte Ausgestaltungsformen der Erfindung ergeben sich aus den Unteransprüchen.

Die Erfindung soll im folgenden an Hand von vorteilhaften Ausführungsformen und verschiedenen Aspekten der erfindungsgemäßen technischen Lehre näher erläutert werden, wobei nach Darstellung der erfindungsgemäßen Erzeugnisse - d.h. der Moleküle, die synonym auch als Aminosäuresequenzen, als Peptide oder Proteine oder einer Mischung dieser bezeichnet werden können - bevorzugte erfindungsgemäße Verfahren und Anwendungen beschrieben werden: (i) zunächst werden die erfindungsgemäße Aminosäuresequenz SEQ ID Nr. 1, (ii) die räumliche Struktur der Aminosäuresequenz gemäß SEQ ID Nr. 1 und (iii) Erkennungsmoleküle, die gegen diese gerichtet sind, näher erläutert, (iv) folgend werden Herstellungsverfahren der Aminosäuresequenz gemäß SEQ ID Nr. 1 beschrieben, (v) anschließend werden Verfahren zur Gewinnung bevorzugter Modifikationen und Abwandlungen (Muteine) der Aminosäuresequenz gemäß SEQ ID Nr. 1 offenbart, (vi) sowie ein Nachweis- und die Gewinnungsverfahren von modifizierten Aminosäuresequenzen gemäß SEQ ID Nr. 1, die an definierte Targetmoleküle binden, (vii) und abschließend werden vorteilhafte Verwendungen der Aminosäuresequenz gemäß SEQ ID Nr. 1 und der davon abgeleiteten Muteine sowie (viii) noch einmal Vorteile der erfindungsgemäßen Lehre erläutert.

(i) Neben SEQ ID Nr. 1 ist auch eine funktionsanaloge Struktur, wie z. B. SF SHLEGLLQEA GPSEACCVRD VTEPGALRME TGDPITVIEG SSSFHSPDST IWKGQNGRTF KVGSFPASAV TLADAGGLPA bevorzugt. Der Begriff "im wesentlichen" (oder funktionsanaloge Struktur) heißt im Sinne der Erfindung, dass insbesondere die Aminosäuresequenz aus der Sequenzabfolge EKKEQKEKEK KEQEIKKKFK LTGPIQVIHL AKACCDVKGG KNELSFKQGE QIEIIRITDN PEGKWLGRTA RGSYGYIKTT AVEIDYDSLK LKKD besteht, es aber nicht ausgeschlossen ist, dass die Sequenzabfolge eine weitere Aminosäure oder eine andere Struktur (Lipid- und/oder Zuckerrest) umfasst. Auch wenn der erfindungsgemäße Stoff aus der Aminosäuresequenz EKKEQKEKEK KEQEIKKKFK LTGPIQVIHL AKACCDVKGG KNELSFKQGE QIEIIRITDN PEGKWLGRTA RGSYGYIKTT AVEIDYDSLK LKKD besteht, so ist dem Fachmann bekannt, dass er Änderungen durch Additionen, Deletionen oder Substitutionen vornehmen kann, ohne dass das Polypeptid im wesentlichen verändert wird. Nicht wesentlich verändert ist die modifizierte Aminosäuresequenz, wenn sie die gleiche Funktion erfüllt wie die Sequenz EKKEQKEKEK KEQEIKKKFK LTGPIQVIHL AKACCDVKGG KNELSFKQGE QIEIIRITDN PEGKWLGRTA RGSYGYIKTT AVEIDYDSLK LKKD und zwar im wesentlichen auf dieselbe Art und Weise, wobei sie hierbei zum selben Ergebnis führt. D. h. die modifizierte Aminosäuresequenz entspricht im Sinne der Erfindung im wesentlichen insbesondere der Sequenz EKKEQKEKEK KEQEIKKKFK LTGPIQVIHL AKACCDVKGG KNELSFKQGE QIEIIRITDN PEGKWLGRTA RGSYGYIKTT AVEIDYDSLK LKKD (SEQ ID Nr. 1), wenn ihre Anwendung keine wesentliche Auswirkung auf die Lösung des erfindungsgemäßen Problems hat und wenn dies für den Fachmann offensichtlich ist, wobei der Fachmann hierbei erkennt, dass die Erfinder nicht die Absicht haben, ihre Lehre auf den Wortlaut der Ansprüche - d. h. auf die Sequenz gemäß SEQ ID Nr. 1 zu beschränken.

Die Aminosäuresequenz oder das Polypeptid gemäß SEQ ID Nr. 1 zeigt überraschende Eigenschaften in Bezug auf sein Verhalten unter entweder reduzierenden oder oxidierenden Bedingungen. Insbesondere NMR-spektroskopische Untersuchungen zeigen, dass die beiden benachbarten Cysteine von SEQ ID Nr. 1 reversibel eine intramolekulare Disulfidbrücke bilden können. Das Polypeptid oder Protein liegt dabei in beiden Formen als Monomer vor, wobei sich die Struktur der durch SEQ ID Nr. 1 definierten Aminosäuresequenz unter reduzierenden oder oxidierenden Bedingungen signifikant ändert. Mittels Dithiothreitol (reduzierend) oder Wasserstoffperoxid (oxidierend) kann der Redox-Zustand eindeutig festgelegt werden, da keine weiteren Cysteine ausserhalb der benachbarten Cysteine in SEQ ID Nr. 1 vorkommen. Auch reduziertes und oxidiertes Glutathion, der intrazelluläre Redox-Puffer, eignet sich zur Einstellung einer der beiden Konformationen, es sind aber beliebige Redox-Puffer-Systeme zur Einstellung des Redox-Gleichgewichts der Aminosäuresequenz nach SEQ ID Nr. 1 denkbar. Vorteilhafte Eigenschaften der Aminosäuresequenz nach SEQ ID Nr. 1 sind die sehr gute Löslichkeit, die relativ hohe Temperaturstabilität und die geringe Tendenz zur Aggregation.

Der Gegenstand der Erfindung ist auch eine Proteinerkennungsdomäne, die die Aminosäuresequenz bzw. die von dieser abgeleiteten Mutanten oder Muteine umfasst. Die Proteinerkennungsdomäne kann durch Standardverfahren der Biochemie bzw. Genetik in funktionsanaloge Derivate und strukturhomologe Makromoleküle so umgewandelt werden, dass diese besonders effizient mit Targetmolekülen wechselwirken, deren Inhibition Redox-Potential-abhängig modifizierbar ist. Ein Target im Sinne der Erfindung sind z.B. Zucker, RNA, DNA, Aminosäuren, Vitamine, second messenger, insbesondere Proteine oder Lipide.

(ii) Die Struktur der Aminosäuresequenz gemäß SEQ ID Nr. 1: Durch die Strukturaufklärung wurde eine Domäne identifiziert, die zu einer Familie von Protein-Interaktionsdomänen gehört. Vorteilhafterweise kann durch geeignete Mutationen die Domänen so verändert werden, dass sie als Bindungspartner für beliebige andere Zielmoleküle fungieren kann. Überraschend konnte gezeigt werden, dass die Bindung abhängig vom Redox-Potential (der Umgebung) ist. Es können daher spezifische Zielmoleküle einer Variante der Domäne entwickelt werden, die in der einen Redoxform an das Zielmolekül binden und in der anderen nicht.

Durch NMR-spektroskopische Methoden wurde die dreidimensionale Struktur der Domäne bzw. der Aminosäuresequenz nach SEQ ID Nr. 1 aufgeklärt. Es stellte sich heraus, dass die Domäne durch $H_2O_2$ bzw. Dithiothreitol reversibel in eine oxidierte bzw. reduzierte Form gebracht werden kann. Beide Formen unterscheiden sich strukturell deutlich voneinander. Die strukturelle Umwandlung beruht auf der Oxidation bzw. Reduktion der beiden Cysteine in der Sequenz. Diese strukturelle Umwandlung stellt einen wichtigen Aspekt der Erfindung dar. Die Erfindung betrifft daher auch Peptidstrukturen, die mindestens zwei benachbarte Cysteine aufweisen, die sich Redox-abhängig umwandeln können.

Durch analytische Ultrazentrifugation und NMR-Methoden konnte gezeigt werde, dass sowohl die oxidierte als auch die reduzierte Form der Domäne - also der Aminosäuresequenz nach SEQ ID Nr. 1 - als Monomer vorliegt. Es handelt sich also um eine intramolekulare Reaktion, bei der zwischen den beiden benachbarten Cysteinen eine Disulfidbrücke gebildet wird; es kommt bevorzugt nicht zur Bildung von Dimeren etc. durch Bildung von intermolekularen Disulfidbrücken. Jedoch kann die Disulfidbrücke Bestandteil einer spezfischen Protein-Interaktionsfläche mit einem anderen Polypetid sein. Die Domäne bindet an Sequenzen in anderen Proteinen, und an saure Lipide, wie sie z.B. in biologischen Membranen vorkommen. Dabei sind die verschiedenen Domänen der erfindungsgemäßen Peptide jeweils spezifisch in ihrer Bindung für bestimmte Sequenzen, ähnlich wie ein bestimmter Antikörper spezifisch ist.

Die Strukturen der reduzierten und oxidierten Form der erfindungsgemäßen Aminosäuresequenzen, beruhen auf NOE-Restraints, die in Tabelle 3 zusammengefasst sind. Das Charakteristische der Struktur ist eine N-terminale alpha-Helix, die gegen ein beta-Faltblatt gepackt ist und über diverse hydrophobe Kontakte wechselwirkt. Insbesondere befinden sich zwei Cysteine direkt benachbart an den Positionen 34 und 35 dieser Struktur der Aminosäuresequenz gemäß SEQ ID Nr. 1 und können in der oxidierten Variante eine intramolekulare Disulfidbrücke bilden. Diese Eigenschaft und die Veränderungen in der räumlichen Anordnung der Atome infolge des Cysteinyl-Cystein-Ringschlusses werden durch die beiden dreidimensionalen Strukturen, die auch Gegenstand der Erfindung sind, offenbart. Ein weiterer Aspekt der Erfindung ist auch das Ensemble der 3D-dimensionalen Koordinaten (siehe Beispiele). Elektronische Repräsentationen dieser Koordinaten oder schematische Repräsentationen, welche die Sekundärstrukturelemente und den Verlauf der Kette (Rückgrat-Konformation) enthalten, sind ein weiterer wesentlicher Aspekt der Erfindung. Dies bezieht sich auch auf die elektronische Repräsentation der Struktur eines Muteins, welches eine ähnliche dreidimensionale Faltung wie die Struktur des Polypeptids gemäß SEQ ID Nr. 1 aufweist.

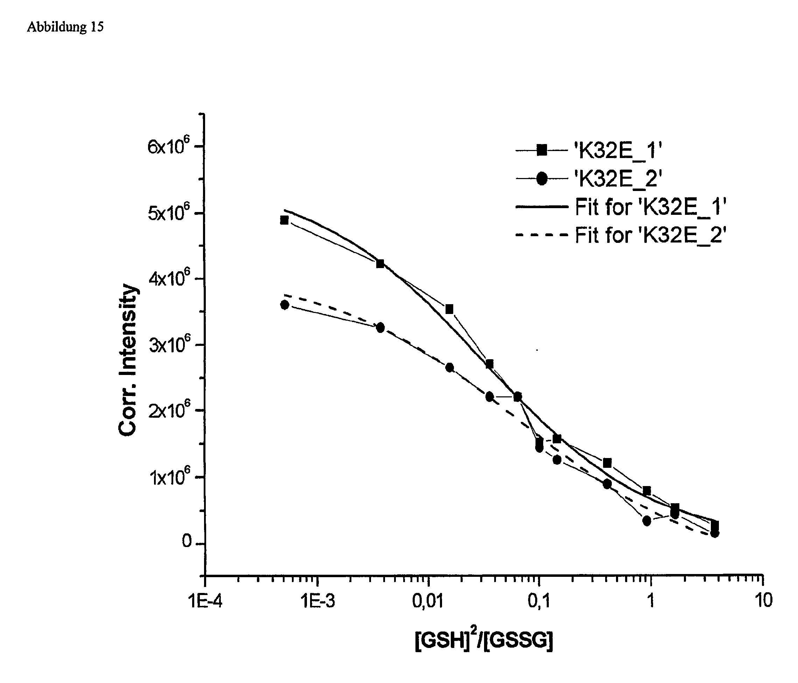

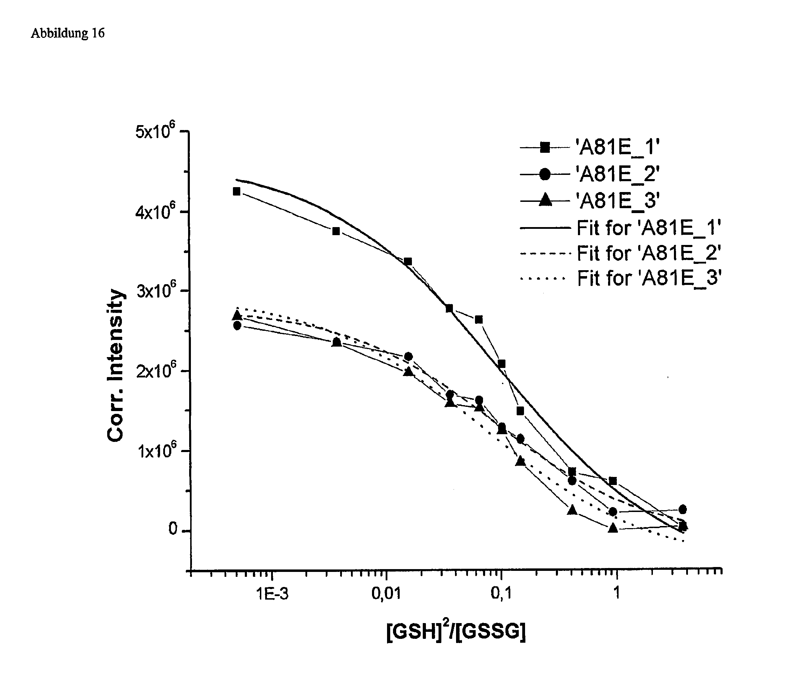

Vorteilhafterweise erlauben die Strukturen gemäß der Erfindung die Anwendung der Methode der rationalen Mutagenese, um das Redox-Potential der Domäne zu verändern. Insbesondere, aber nicht ausschliesslich, werden die Positionen 18, 20, 32, 38 bis 43, 47, 48, 50, 59 bis 64, 78, 79, 80 und/oder 81, insbesondere 32, 48, gemäß der erfindungsgemäßen Struktur gegen eine der zwanzig natürlichen Aminosäuren ausgetauscht, die dem Fachmann bekannt sind; das Redox-Potential kann z. B. über die NMR-Spektroskopie (wie in den Beispielen angeführt) bestimmt werden. D. h. jede der Positionen 32, 38 bis 43, 47, 48, 50, 59 bis 64, 78, 79, 80 und/oder 81 kann mit folgenden Aminosäuren besetzt werden: Gly, Ala, Val, Leu, Ile, Phe, Tyr, Trp, Pro, Asp, Glu, Asn, Gln, Ser, Thr, Cys, Met, Lys, Arg oder His. Dadurch werden Varianten des erfindungsgemäßen Proteins erzeugt, die ein spezifisches Redox-Potential besitzen und als Scaffold-Protein verwendet werden können (s. Abb. 15 und 16 und Beispielteil).

Ebenso dient die erfindungsgemäße Struktur als Grundlage für das Einführen von Mutationen, die zu grösseren "Cavities" führen, z.B. unter zu Hilfenahme des Programms VOIDOO (Uppsala Software Factory). Dadurch können gezielt Bindungstaschen, z.B. für Fluorophore, Xenon oder Lipide in das Protein eingeführt werden. Ausserdem erlaubt die Kenntnis der Struktur die Anwendung einer Methode zur Randomisierung von Aminosäuren, welche die Grundlage für die neuen Bindungseigenschaften von Sequenzen nach SEQ ID Nr. 1 oder den Muteinen bildet. Auch das Auffinden von kleinen organischen Molekülen, die an bestimmte Oberflächenstrukturen (wie z.B. hydrophobe Taschen oder elektrostatisch positiv oder negativ geladene Bereiche) binden, wird durch die erfindungsgemäße Struktur vorteilhafterweise ermöglicht.

(iii) Bevorzugte Modifikationen und Abwandlungen der Aminosäuresequenz gemäß SEQ ID Nr. 1 : Es ist selbstverständlich möglich, gezielte Abwandlungen und Modifikationen der Aminosäuresequenz vorzunehmen, um eine verbesserte Funktion zu erhalten. Derartig gezielt veränderte Aminosäuresequenzen werden im Sinne der Erfindung als Muteine (siehe oben) bezeichnet. Die erfindungsgemäße Aminosäuresequenz bzw. deren Muteine können durch geeigneten Austausch, Deletion oder Addition von weiteren Aminosäuren oder durch Einfügung eines Glykosidrestes weiterhin modifiziert werden, ohne dass die Fähigkeit der Aminosäuresequenz, mit einem Targetmolekül, dessen Inhibition Redox-Potential-abhängig schlatbar ist, zu interagieren, wesentlich beeinträchtigt ist. Derartige Aminosäuresequenzen, insbesondere Muteine, werden im Sinne der Erfindung auch als funktionsanaloge Derivate oder Strukturhomologe bezeichnet. Es kann jedoch auch bevorzugt sein, dass Muteine nicht-schaltbar inhibieren. Die Muteine sind insbesondere als Redox-sensitive Inhibitoren einsetzbar. Im Sinne der Erfindung ist jedes von der erfindungsgemäßen Aminosäuresequenz (bevorzugt SEQ ID Nr. 1) abgeleitete Polypeptid, welches nur im oxidierten oder reduzierten Zustand an die Target-Moleküle bindet, ein Mutein. Der Redoxzustand des Inhibitors ist sensitiv für die aktuelle Umgebung. Selbstverständlich ist die Redox-Schaltbarkeit keine notwendige Eigenschaft der Muteine. Die Muteine können auch bindende Varianten sein, die krankheitsrelevante Targets nicht Redox-abhängig (also sowohl in reduzierter als auch in oxidierter Form) binden. Auch solche Muteine sind - mit einer nicht-redox-abhängigen Erkennung - bevorzugte Marker oder Sonden der Analyse oder therapeutische Mittel zur Behandlung der oben genannten Krankheiten.

Die Muteine können auch als funktionsanaloge Sequenzen bezeichnet werden. Funktionsanaloge Sequenzen sind im Sinne der Erfindung jene Sequenzen, die der Fachmann als gleichwirkend identifizieren kann. Selbstverständlich ist es möglich, dass der Fachmann aufgrund seiner Kenntnis der im Menschen gefundenen Nukleinsäuremoleküle auch in Versuchstieren Analoge und Homologe aufgrund von Homologie- oder Analogie-Untersuchungen detektiert, mit denen Redox-Vorgänge untersucht werden können.

Funktionsanalog bedeutet insbesondere, dass die abgewandelten Strukturhomologen oder Derivate wie die Aminosäuresequenz gemäß SEQ ID Nr.1 die Fähigkeit besitzen, mit Targetmolekülen messbar zu interagieren bzw. zur Identifizierung von Redox-abhängigen Protein-Protein- oder Protein-Lipid-Wechselwirkungen eingesetzt zu werden. Das heißt, die Aminosäuresequenzen bzw. die Muteine werden durch den Austausch, die Deletion oder die Addition von Aminosäuren oder durch Hinzufügen bzw. Variationen von Zuckerresten nicht so beeinträchtigt, dass ihr Bindungsverhalten hierdurch so stark nachlässt, dass die erfindungsgemäßen Aufgaben nicht mehr gelöst werden könnten.

Für die Aminosäuresequenz nach SEQ ID Nr. 1 können z.B. Aminosäureaustausche auf der Basis verschiedener Kriterien gemacht werden, die Hydrophobizität, Ladungseigenschaften, Polarität, Größe, die Anwesenheit einer funktionellen Gruppe (z.B. -NH2-Gruppe, aromatischer Charakter) umfassen. Die Zuordnung der Aminosäuren in bestimmte Gruppen wird der geübten Fachkraft gut möglich sein, weitere angemessene Aminosäureaustausche sind z.B. in Bowie et al. (Science 247; 1306-1310 (1990)) zu finden. Z.B. können konservative Aminosäure-Austausche gemacht werden in Bezug auf Aminosäuren, welche verwandte Seitenketten besitzen. Die natürlichen Aminosäuren werden so in vier Gruppen eingeteilt, innerhalb derer Aminosäurensubstitutionen insbesondere in der Sequenz EKKEQKEKEK KEQEIKKKFK LTGPIQVIHL AKACCDVKGG KNELSFKQGE QIEIIRITDN PEGKWLGRTA RGSYGYIKTT AVEIDYDSLK LKKD oder SF SHLEGLLQEA GPSEACCVRD VTEPGALRME TGDPITVIEG SSSFHSPDST IWKGQNGRTF KVGSFPASAV TLADAGGLPA bevorzugt sind: (1) saure Aminosäuren: Apsartat und Glutamat. (2) Basische Aminosäuren: Lysin, Arginin und Histidin, (3) nicht-polar: Alanin, Valin, Leucin, Isoleucin, Prolin, Phenylalanin, Methionin, Tryptophan, (4) ungeladen polare Aminosäuren: Glycin, Asparagin, Glutamin, Cystein, Serin, Threonin und Tyrosin. Phenylalanin, Tryptophan und Tyrosin werden manchmal auch als Aminosäuren mit aromatischen Seitenketten als Gruppe definiert. Es ist z.B. zu erwarten, das ein isolierter Austausch von Leucin zu Isoleucin, von Aspartat zu Glutamat, von Threonin zu Serin oder ein ähnlicher konservativer isolierter Austausch nicht zu einer wesentlichen Veränderung in der Aktivität oder Bindungseigenschaft der Sequenz gemäß SEQ ID Nr. 1 führen wird.

Bevorzugte Aminosäuresequenzen - insbesondere Peptide oder Proteine - im Sinne der Erfindung sind die, die ein Cys-Cys-Motiv enthalten, welches ein reversibel einstellbares Redox-Potential im Bereich von -400 bis +200 mV besitzt, bevorzugt im Bereich -300 mV bis 0 mV, noch mehr bevorzugt im Bereich -300 mV bis -150 mV, also alle Proteine mit einem Cys-Cys-Motiv, wie gemäß SEQ ID Nr. 1 offenbart. Diese Peptide können bevorzugt an den direkt benachbarten Positionen w-x-Cys-Cys-y-z (w, x, y, z: eine beliebige der zwanzig natürlichen Aminosäuren) durch eine der 20 natürlich vorkommenden Aminosäuren ersetzt sein und ein verändertes Redoxpotential mit Werten zwischen -400 und +200 mV aufweisen.

(iv) Erkennnungsmoleküle, die gegen die erfindungsgemäßen Moleküle gerichtet sind: Die Erfindung betrifft auch ein Erkennnungsmolekül, das gegen das Nukleinsäuremolekül, den Vektor, die Wirtszelle und/oder das Polypeptid - die Aminosäuresequenz - gerichtet sind. Erkennungssubstanzen im Sinne der Erfindung sind Moleküle, die mit den genannten Strukturen wie Nukleinsäuremolekülen oder -sequenzen, Vektoren, Wirtszellen und/oder Polypeptiden bzw. deren Fragmenten wechselwirken können; insbesondere so wechselwirken, dass eine Detektion dieser Strukturen möglich ist. Die Erkennungssubstanzen können insbesondere spezifische Nukleinsäuren sein, die an das erfindungsgemäße Nukleinsäuremolekülen binden, aber auch Antikörper, Fluoreszenzmarker, markierte Kohlenhydrate oder Lipide, Antisense-Konstrukte, cDNA oder mRNA-Moleküle bzw. deren Fragmente. Es ist selbstverständlich auch möglich, dass die Erkennungssubstanzen nicht Proteine oder Nukleinsäuren bzw. Antikörper sind, sondern gegen diese gerichtete Antikörper. Die Erkennungssubstanzen können in solch einem Fall insbesondere sekundäre Antikörper sein. In einer besonderen Ausführungsform der Erfindung sind die Erkennungsmoleküle Antikörper, Antikörperfragmente und/oder Antisensekonstrukte, z.B. RNA-Interferenzmoleküle. Die Erfindung betrifft also auch Antikörper, welche bevorzugt die Sequenz gemäß SEQ ID Nr. 1, die Muteine oder Fragmente dieser Sequenz binden. Zum Beispiel bezieht sich die Erfindung auf polyklonale oder monoklonale Antikörper, einschliesslich nicht-humaner und humaner Antikörper, chimäre Antikörper, humanisierte Antikörper oder Fragmente von Antikörpern, die nach Immunisierung, durch Hybridomazellen oder durch Phage Display oder durch Ribosome Display gewonnen wurden (Current Protocols in Immunology, John Wiley & Sons, N. Y. (1994;) EP application 173,494 (Morrison); International Patent Application WO86/01533; Chem. Rev. 2001, 101, 3205-3218, Ronald H. Hoess, Protein Design and Phage Display);(Hanes & Plückthun, Proc. Nat. Acad. Sci. 94, 4937-4942 (1997); Roberts & Szostak 94, 12297-12302 (1997)). Techniken zur Gewinnung von Immunogenizität gegen das Protein oder Polypeptid umfassen die Konjugation an eine Träger-Substanz oder ein Träger-Molekül, die dem Fachmann wohlbekannt sind. Das Protein kann zudem in Anwesenheit eines Adjuvans für die Immunisierung verwendet werden. Standard ELISA oder Immuno-Assays mit dem Protein der SEQ ID Nr. 1 können benutzt werden um die Menge an Antikörper festzustellen. Auch kann der Antikörper (das Antikörperfragment) mit einem weiteren Molekül verknüpft werden (z.B. ein Fluorophor), welches die bessere Detektion des Antikörpers erlaubt.

(v) Herstellungsverfahren zur Generierung der Aminosäuresequenz bevorzugt gemäß SEQ ID Nr. 1:

Bei der erfindungsgemäßen Aminosäuresequenz kann es sich beispielsweise um rekombinant hergestellte Produkte handeln. Diese Produkte können durch genetische Manipulationen hergestellt werden, bei denen die codierende Nukleinsäure für das benötigte Produkt gewöhnlich mit Hilfe eines Plasmids oder eines viralen Vektors in einen geeigneten Mikroorganismus oder in eine geeignete Zelllinie eingeführt wird. In der Zelle wird die Nukleinsäure exprimiert und in die Aminosäuresequenz translatiert. Die gewünschte Aminosäuresequenz kann aus den Zellen durch Extraktion und Reinigung gewonnen werden. Die Proteine und Polypeptide der erfindungsgemäßen Anwendung können durch eine Reihe von Prozeßen isoliert und gereinigt werden. Diese umfassen, aber sind nicht begrenzt auf, Anionen oder Kationenaustausch-Chromatographie, High Performance Liquid Chromatography (HPLC), Ni-basierte Chromatographie His-getagter Proteine, Streptavidin-Tag basierte Reinigung. Die spezielle Methode zur Reinigung hängen von den speziellen Eigenschaften des Polypeptids und der Wirtszelle ab und sind für die geübte Fachkraft direkt ersichtlich.

Die Transfektion eines Vektors, der die Aminosäuresequenz insbesondere gemäß SEQ ID Nr. 1 oder eines der Muteine enthält, kann in prokaryotische oder eukaryotische Zellen erfolgen. Zellen, die transfiziert werden können umfassen, aber sind nicht limitiert auf, Bakterien-Zellen (wie etwa E.coli K12-Stämme, Streptomyces, Pseudomonaden, Serratia marcescens und Salmonella typhimurium, Insektenzellen (wie z.B. Sf9-Zellen) einschliesslich Drosophila-Zellen, Pilze wie etwa Hefe (S.cerevisiae, S.pombe, Pichia pastoris), Pflanzenzellen und Säugerzellen wie z.B. Thymocyten, primäre Zellen oder Zellkulturen wie Jurkat, CHO-Zellen oder COS-Zellen.

Die Expressionsrate in E.coli BL21 (DE3) ist vorteilhafterweise sehr groß (typischerweise 20 mg gereinigtes Protein pro 1 Liter Bakterienkultur). Als Konsequenz dieser ungewöhnlichen Eigenschaft können auch Muteine der Aminosäuresequenz bevorzugt nach SEQ ID Nr. 1 erzeugt werden, die ebenfalls stabil exprimiert werden und die ein verändertes Redoxpotential aufweisen (s. Beispiele). Eine weitere Eigenschaft der Aminosäuresequenz nach SEQ ID Nr. 1 besteht in der Bindung saurer Lipide, wie Phosphoinositolen. Weiterhin kann die Sequenz gemäß SEQ ID Nr. 1 an Position 86 in einem in vitro Kinase-Versuch phosphoryliert werden und auch in vivo kann diese Phosphorylierung stattfinden. Dadurch wird die Möglichkeit gegeben, die Domäne über phosphospezifische Antikörper oder SH2-Domänen in Kontakt zu bringen und damit analytische nachweisen oder diese zu Reinigungszwecken zu verwenden. Weiterhin können Aminosäuresequenzen nach SEQ ID Nr. 1 oder entsprechende Muteine Vorläufermoleküle aktiver Moleküle sein, d.h. aus diesen Vorläufermoleküle können durch Spaltung aktive Aminosäuresequenzen nach SEQ ID Nr. 1 oder die entsprechenden aktiven Muteine erzeugt werden.

Alternativ kann das Polypeptid bevorzugt der SEQ ID Nr. 1 oder die entsprechenden Muteine auch durch in vitro Translation hergestellt werden. Dabei werden prokayrotische oder eukaryotische Zellextrakte für die Translation einer cDNAgelesenen mRNA in Polypeptid-Sequenzen verwendet. Eine weitere Alternative zur Herstellung des Polypeptids besteht in der chemischen Synthese von insbesondere SEQ ID Nr. 1.

Die Sequenz der bevorzugten erfindungsgemäßen Aminosäuresequenz SEQ ID Nr. 1 - d.h. EKKEQKEKEK KEQEIKKKFK LTGPIQVIHL AKACCDVKGG KNELSFKQGE QIEIIRITDN PEGKWLGRTA RGSYGYIKTT AVEIDYDSLK LKKD - kann z. B. als rekombinantes Peptid oder als ungetagtes oder z.B. $His_6$-getagtes Protein in dem Bakterium E. coli exprimiert werden.

Die erfindungsgemäße Aminosäuresequenz kann z.B. mit einer Membran-Translokations-Sequenz (MTS) auf der Ebene der DNA verknüpft werden, um bevorzugt Zellmembran-permeabel zu werden. Dadurch wird die Protein-Trasnduktion ermöglicht, d.h. die erfindungsgemäße Aminosäuresequenz kann vorteilhafterweise in Zellen und Gewebe eindringen (Behrens et al. Curr Gene Ther. 2003 Oct;3(5):486-94; Rojas et al., Nat Biotechnol. 16, 370-375 (1998)).

(vi) Bevorzugte Methoden zur Herstellung von Modifikationen und Abwandlungen der Aminosäuresequenz gemäß SEQ ID Nr. 1: In einer bevorzugten Ausführungsform der Erfindung kann die Modifikation des erfindungsgemäßen Polypeptides mit verschiedenen Methoden erfolgen, die insbesondere eine gezielte oder eine zufällige Modifikation ermöglichen. Dem Fachmann sind hierzu verschiedene Methoden bekannt. Dabei können gezielt Nukleotide ausgetauscht werden, so dass eine gewünschte Aminosäuresequenz oder Mutante erzeugt wird. Bevorzugt können randomisierende Methoden (z.B. error-prone-PCR, Verwendung degenerierter Oligonukleotide in der PCR oder bei der Gensynthese, DNA-shuffling, UV-Strahlung) eingesetzt werden, um eine Vielfalt von Muteinen der Aminosäuresequenz gemäß SEQ ID Nr. 1 zu erzeugen. Die Amplifizierung, Klonierung, Ligation etc. der entstehenden Nukleotid-Sequenzen erfolgt insbesondere mit molekularbiologischen Methoden.

Bei besonders bevorzugten Methoden zur Herstellung von Muteinen werden zunächst Variationen einer Ausgangs-DNA oder - RNA erzeugt. Das Ergebnis dieser - mit molekularbiologischen Mitteln umgesetzten - kombinatorischen Methoden ist eine Mischung (Bibliothek) verschiedener Moleküle, die bevorzugt Peptide (als Peptid-/Polypeptid-Bibliothek), Proteine oder Nukleinsäuren (als Protein- oder Nukleinsäure-Bibliothek) sein können. Es haben sich im Stand der Technik verschiedene Methoden zur Generierung dieser Mischung etabliert. Die Mischung kann als Bibliothek verschiedener Moleküle in-vivo, d.h. in Organismen wie z.B. Bakterien, oder in-vitro, beispielsweise in einem Reagenzglas mit Hilfe von Enzymen oder isolierten biologischen Komponenten erzeugt werden.

Als Basis für die Erzeugung der Mischung verschiedener Moleküle dient z.B. entweder die DNA oder RNA in verschiedenen Variationen. Je nach Technik wird diese Variation dann in Proteine übersetzt oder als RNA bzw. DNA getestet. Hierzu dienen spezielle - dem Fachmann bekannte - Selektionssysteme, also Methoden, welche die Proteine oder die DNA bzw. RNA mit den gewünschten Eigenschaften selektieren. Insbesondere sei hier neben dem Phage Display das Ribosome Display (Hanes & Plückthun, Proc. Nat. Acad. Sci. 94, 4937-4942 (1997); Roberts & Szostak 94, 12297-12302 (1997)) erwähnt. Die selektierten Moleküle werden in einem weiteren Schritt angereichert und gegebenenfalls im Detail (Sequenzabfolge, Redoxpotential, pI u.ä.) analysiert. Eine bevorzugte - mit molekularbiologischen Techniken - umgesetzte kombinatorische Methode ist die Phage-Display-Technologie. Hierzu werden bevorzugt Bakteriophagen, d. h. Viren, die Bakterien infizieren, eingesetzt, die an ihrer Oberfläche Proteine exprimieren, die gewisse Veränderungen, beispielsweise zusätzliche Segmente in bestimmten Sequenzbereichen, tolerieren. Dabei werden vor allem Protein-Fusionen des GenIII- oder des GenVIII-Oberflächenproteins verwendet, so dass ein beliebiges Polypeptid auf der Oberfläche des Phagen als Fusionsprotein exprimiert wird. Die Bereitstellung der Bakteriophagen, die kombinatorischen Möglichkeiten der Phage-Display-Technologie, der Aufbau einer Phagen-Bibliothek und ihre Vermehrung sowie das Screening der gewünschten Phagen ist in der Standardliteratur ausreichend beschrieben, so dass diesbezüglich auf die entsprechende Literatur verwiesen werden kann (z. B. Chem. Rev. 2001, 101, 3205-3218, Ronald H. Hoess, Protein Design and Phage Display).

Eine weitere bekannte kombinatorische Methode ist die kombinatorische Mutagenese. Bei dieser Technik erfolgt die Variation der Aminosäuren indirekt auf der DANN-Ebene. Das Verfahren der kombinatorischen Mutagenese, insbesondere das Screening nach Aktivität, bzw. das Screening nach physikalischen Eigenschaften bzw. die Techniken der zirkulären Permutation sind dem Fachmann bekannt.

Weitere bevorzugte kombinatorische Möglichkeiten ergeben sich aus in-vitro-Systemen zur Proteinbiosynthese und der gesteuerten Evolution. Hierzu zählen die Ribosom-Display-Technologie sowie verschiedene Techniken des DNAshufflings.

Selbstverständlich kann die Polypeptid-Bibliothek nicht nur unter Verwendung der Aminosäuresequenz gemäß SEQ ID Nr. 1 bereitgestellt werden. Bevorzugte Sequenzen sind auch die in Tabelle 4 genannten, welche CC-Motive als Teil einer gefalteten Bindungsdomäne enthalten.

(vi) Nachweis- und Gewinnungsverfahren von an Targetmolekülen bindenden Abwandlungen und Modifikationen der Aminosäuresequenz gemäß SEQ ID Nr. 1: Bevorzugt wird durch Phage Display eine Bibliothek von Varianten der Aminosäuresequenz gemäß SEQ ID Nr. 1 hergestellt. Auf Grundlage der aufgeklärten Struktur werden die Reste 32, 36-43, 47, 48, 50, 59-64, 78, 80 und/oder 81, bevorzugt 32, 37, 48 und/oder 81 (Nummerierung in Bezug auf die oben stehende Sequenz) ausgewählt, die in dieser Bibliothek im Prinzip je durch alle zwanzig natürliche Aminosäuren ersetzt werden. Dies entspricht bei 17 variierten Resten einer theoretischen Größe der Bibliothek von ca. $4 * 10^{24}$ Varianten. Bevorzugt ist es, mindestens $1 * 10^8$ Varianten in der Bibliothek zu haben. Diese Bibliothek wird im Weiteren für das Screening verwendet.

Gegenstand der Erfindung ist demgemäss also auch ein in-vitro Verfahren - bei dem die erfindungsgemäße Peptidbibliothek zum Einsatz kommt - zur Detektion und Gewinnung von an Targetmolekülen bindenden Polypeptiden umfassend folgende Schritte

a) Immobilisierung eines potentiellen Targetmoleküls auf einem Träger,
b) Inkontaktbringen von Peptiden mit der o. g. Polypeptid-Bibliothek mit den immobilisierten Targetmolekülen,
c) Eliminieren der nicht an die Targetmoleküle gebundenen Polypeptide,
d) Eluieren der gebundenen Polypeptide unter stringenten Bedingungen, wobei die bindenden Polypeptide gewonnen werden.

Ein Ziel- oder Targetmolekül (z.B. ein Protein, eine Nukleinsäure, ein Zucker oder ein Lipid) wird identifiziert, dessen Funktion beeinflusst werden soll. Von besonderem Interesse sind Target-Moleküle, die eine Rolle im Redox-Signaling spielen, z.B. NFkB, Phosphatasen (SHP-2), Transkriptionsfaktoren, Lipide. Bevorzugt sind auch Target-Moleküle, die nur im oxidierten oder reduzierten Zustand von einer Sequenz gemäß SEQ ID Nr. 1 gebunden werden und unterschiedliche Fluoreszenzeigenschaften im gebundenen versus nicht-gebundenem Zustand besitzen. Vorteilhafterweise wird der Einsatz Redox-Potential-abhängiger Fluoreszenzmarker in der Zelle dadurch ermöglicht.

Das Targetmolekül wird insbesondere auf einer Säule immobilisiert, die Phagenbibliothek wird über die Säule gegeben. Wenn die Bibliothek geeignete Varianten zur Bindung enthält, binden diese an das Targetmolekül, alle anderen Phagen werden direkt von der Säule gespült. Die gebundenen Phagen können anschließend unter strikteren Bedingungen (pH-Wert = 2.2) ebenfalls von der Säule gespült werden. Man erhält so eine Auswahl der ursprünglichen Bibliothek. Die enthaltenen Phagen werden vermehrt und wiederum über die Säule gegeben. Dieser Zyklus wird z.B. 5-10 Mal wiederholt. Dabei wird die Anzahl der Phagen und mithin der Varianten immer kleiner. Schließlich werden die Phagen sequenziert. Dadurch kann im Detail ermittelt werden, welche Varianten der Domäne zur Bindung an das Targetmolekül geeignet sind.

Das Screening der Bibliothek wird zunächst unter oxidierenden/reduzierenden Bedingungen durchgeführt. Nach einigen Zyklen der Auswahl wird das Screening unter den entgegengesetzten Bedingungen fortgesetzt, dabei werden jetzt die Phagen ausgewählt, die nicht binden, also sofort von der Säule eluieren. So erhält man Varianten der Domäne, die nur unter reduzierenden oder oxidierenden Bedingungen an das Zielmolekül binden.

Wichtige zelluläre Target- oder Zielstrukturen, wie etwa die Proteine, die bei der HIV-Infektion eine vermittelnde Rolle spielen, können beispielsweise mittels der erfindungsgemäßen molekularen Bibliothek gescreent werden. Das Screening der Bibliothek mit molekularen Targets, die krankheitsrelevant sind und von denen gezeigt wurde, dass sie eine wichtige Rolle in Redox-abhängigen Prozessen spielen, ist selbstverständlich nicht auf Proteine, die Infektionen - wie beispielsweise HIV-Infektion - vermitteln, beschränkt, sondern betrifft auch Proteine, die bei der Tumorentstehung, bei allergischen Krankheiten und bei Autoimmun-Erkrankungen eine entscheidende Rolle spielen. Beispiele sind die Transkriptionsfaktoren $NF_kB$ und AP-1, die in den Luft-Kanälen asthmatischer Patienten auf Grund einer veränderten Redox-Balance stimuliert werden. $NF_kB$ wird auch durch Angiotensin II stimuliert, welches oxidativen Stress, z.B. bei chronischen Nierenleiden, auslösen kann.

Weiterhin sind Redox-sensitive Phosphatasen als Zielmoleküle bevorzugt, da sie ebenfalls krankheitsrelevant sind. Der geringe Effekt "unspezifischer" Anti-Oxidantien als Medikamente in klinischen Studien wird darauf zurückgeführt, dass es bisher keine spezifischen und sensitiven Bio-Marker gibt, die den Redox-Phänotyp von kardiovaskulären Krankheiten beschreiben.

In einer bevorzugten Ausführungsform wird das Verfahren unter reduzierenden, insbesondere in Gegenwart von Dithiothreitol oder oxidierenden, insbesondere in Gegenwart von Wasserstoffperoxid, Bedingungen durchgeführt.

In einer weiteren bevorzugten Ausführungsform ist die Polypeptid-Bibliothek eine Phagen-Bibliothek.

In einer weiteren bevorzugten Ausführungsform wird das detektierte bindende Polypeptid, welches bevorzugt aus der Phagen-Bibliothek vermehrt wird, mit einem pharmazeutisch akzeptablen Träger formuliert.

Die Erfindung betrifft demgemäß auch ein Verfahren zur Herstellung einer pharmazeutischen Formulierung umfassend das erfindungsgemäße Verfahren, bevorzugt in einer Form bei der das detektierte bindende Polypeptid im einem pharmazeutisch akzeptablen Träger formuliert wird und weiterhin das In-Kontakt-Bringen der identifizierten bindenden Polypeptide mit einem pharmazeutisch akzeptablen Träger.

Die Erfindung betrifft weiterhin die Verwendung des erfindungsgemäßen Nukleinsäuremoleküls oder der erfindungsgemäßen Polypeptide zusammen mit einer Membran-Translokationssequenz oder einem GFP-Konstrukt zur Identifizierung von Redox-abhängigen Protein-Protein- oder Protein-Lipid-Wechselwirkungen.

Die Erfindung betrifft auch ein in-vitro Verfahren zur Detektion von Targetmolekülen, deren Inhibition Redox-Potential-abhängig schaltbar ist, umfassend die Schritte:

- Bereitstellung einer Probe umfassend mindestens einen Kandidaten des Targetmoleküls,
- In-Kontakt-Bringen eines der erfindungsgemäßen Polypeptide oder der erfindungsgemäßen Polypeptid-Bibliothek mit der Probe und
- Detektion der Wechselwirkung, insbesondere der Bindung zwischen dem Targetmolekül und dem Polypeptid.

In einer bevorzugten Ausführungsform der Erfindung wird dieses Verfahren so durchgeführt, dass die Polypeptid-Bibliothek mittels Phage-Display-Methode unter oxidierenden oder reduzierenden Bedingungen hergestellt wurde.

In einer weiteren bevorzugten Ausführungsform umfasst dieses Verfahren weiterhin die Formulierung des detektierten Targetmoleküls in einer pharmazeutisch akzeptablen Form.

Die Erfindung betrifft auch die Verwendung des detektierten und bindenden Polypeptids oder Targetmoleküls als pharmazeutisches Mittel zur Behandlung von Krankheiten, die mit oxidativem Stress oder der Veränderung des Redox-Potentials von Zellen assoziiert sind.

(vii) Weitere vorteilhafte Verwendungen der Aminosäuresequenz gemäß SEQ ID Nr. 1 und der davon abgeleiteten Muteine: In einer bevorzugten Ausgestaltung der Erfindung kann die Aminosäuresequenz nach SEQ ID Nr. 1 oder deren Muteine als Köder-Proteine in einem Yeast-two-hybrid-System oder three-hybrid-System (z.B. U.S. Patent-Nummer 5, 283,317; Zervos et al. (1993), Cell 72: 223-232; Madura et al. (1993), J. Biol. Chem. 268: 12046-12054; Bartel et al. (1993), Biotechniques 14:920-924; Iwabuchi et al. (1993) Oncogene 8; 1693-1696; and Brent WO94/10300) verwendet werden, um andere Proteine zu identifizieren, die mit dem Protein der SEQ ID Nr. 1 oder deren Muteine interagieren und ggf. die Aktivität modulieren. Solche bindenden Proteine können auch in den Prozessen, z.B. der Signaltransduktion in Zellen, an denen das Peptid gemäß SEQ ID Nr. 1 beteiligt ist beteiligt sein. Eine weitere vorteilhafte Ausgestaltung der Erfindung ist ein Screening zur Identifizierung von Modulatoren (z.B. antisense-Moleküle, Polypeptide, Peptidomimetica, kleine Moleküle), welche an Nukleinsäuresequenzen, Polypeptide oder Proteine, die aus SEQ ID Nr. 1 oder deren Varianten bestehen oder hervorgehen, binden oder deren Aktivität, Expression oder andere Eigenschaften verändern.

Eine weitere vorteilhafte Ausgestaltung betrifft einen Assay, in welchem die Muteine mit unterschiedlichem Redox-Potential an ein Träger-Material gebunden oder in Kontakt gebracht werden (z.B. im Plattenformat), wobei die Cysteine der Muteine mit einem Marker-Moleküle (z.B. ein Fluorphor, ein Farbstoff, ein radioaktives Molekül) kovalent verknüpft sind. Dieses Marker-Molekül bleibt bevorzugt unter Bedingungen, die im Vergleich zur Cystein-Marker-Bindung (bevorzugt eine Schwefel-Schwefel-Bindung) oxidierend sind an das Mutein gebunden. Eine Änderung des Redox-Potentials durch eine Lösung mit verändertem Redox-Potential, z.B. ein zelluläres Lysat, oder durch eine Lösung mit verändertem pH-Wert oder einer Lösung mit einer Kombination aus verändertem Redox-Potential und pH-Wert, führt dann zur Freisetzung des Marker-Moleküls. Dabei kann das Marker-Molekül nur entweder im freien oder aber in an dem Peptid nach SEQ ID Nr. 1/Mutein-gebundenen Zustand eine bestimmte Eigenschaft zeigen, die dann zur Messung des Redox-Potentials der Lösung (z.B. des Zell-Lysates) geeignet ist. So kann etwa ein nur in freiem Zustand fluoreszierendes Molekül durch Inkubation mit einer Lösung, deren Redox-Potential bestimmt werden soll, freigesetzt werden. Der Anteil freien Fluorophors steht dann im Verhältnis zum Redox-Potential der Lösung. Möglich ist aber auch, dass das unter bestimmten Redox-Potential-Bedingungen freigesetzte Marker-Molekül in Wasch-Schritten entfernt wird und der Anteil des weiter gebundenen Marker-Moleküls bestimmt wird. Durch den Einsatz von SEQ ID Nr. 1 und deren Muteinen, mit einem über ein weites Spektrum variierenden Redox-Potential wird es möglich sein, durch dieses Verfahren das Redox-Potential einer Vielzahl verschiedener Lösungen, z.B. Zell-Kulturen, Seren, Körperflüssigkeiten, zu bestimmen.

In einer weiteren erfindungsgemäßen Anwendung wird eine Lipid-Modifikation der beiden Cystein-Reste des SEQ ID Nr. 1 oder der Muteine vorgenommen: Die kovalente Verknüpfung der Redox-aktiven Cysteine mit fluoreszierenden Myristoyl- oder Palmitoyl-Lipiden wird als chemische Reaktion durchgeführt. Da die Aminosäuresequenz nach SEQ ID Nr. 1 schon selbst eine Affinität zu negativ geladenen Membranen hat, führt dies zu einer erhöhten Bindung an Membranen. Wird das Protein in Zellen eingeführt (etwa durch Mikroinjektion) ist eine Membran-Rekrutierung zu erwarten. Kommt es jedoch durch Stimulation der Zellen oder durch chemisch induzierten oxidativen Stress zu einer Veränderung des intrazellulären Redox-Potentials, so wird die Lipid-Gruppe abgespalten und die Aminosäuresequenz nach SEQ ID Nr. 1 wird von der Membran abgelöst werden wobei sich die Fluoreszenz des fluoreszierenden Lipids ändert. Die Änderung des Redoxpotentials führt also zu einer Redox-abhängigen Änderung der Fluoreszenz an der Membran. Alternativ kann das Protein zusätzlich zur Lipid-Modifkation auch Fluoreszenz-markiert werden und die Änderung des Redox-Potentials sollte dann zu einer Abnahme der Fluoreszenz an der Membran führen.

In einer weiteren erfindungsgemäßen Anwendung wird eine Methode zur Verfügung gestellt, bei der die unterschiedlichen Muteine mit variierendem Redox-Potential spezifisch durch bestimmte Antikörper oder deren Antigen-bindende Fragmente erkannt werden. Insbesondere stellt die Methode auch Antikörper zur Verfügung, die nur entweder die reduzierte oder oxidierte Form des jeweiligen Muteins (oder von SEQ ID Nr. 1 selbst) erkennt. Die Methoden zur Gewinnung der Antikörper oder deren Fragmente durch Immunisierungstechniken oder durch in vitro Methdoen (z.B. Phage Display, Ribosome Display) sind dem kundigen Fachmann bekannt und können nach publizierten Protokollen ausgeführt werden. Die Mutein- und Redox-spezifischen Antikörper können in ELISA oder RIA-Assays eingesetzt werden oder aber in Immunopräzipitationsexperimenten und auf Protein-Chips verwendet werden. Dadurch kann der Redox-Zustand von SEQ ID Nr. 1 und den Muteinen in beliebigen Lösungen (deren Redox-Potential bestimmt werden soll, z.B. zellulären Lysaten) durch die Antikörper und nachfolgende Detektionsmethoden (wie z.B. die Verwendung eines sekundären, Enzym-gekoppelten Antikörpers) nachgewiesen werden.

In einer weiteren vorteilhaften Ausgestaltung der Erfindung werden Zellen mit der DNA des jeweiligen Muteins (in einem Vektor, mit regulatorischen Sequenzen versehen oder als Fusionsprotein) transfiziert. Durch die Nachweisreaktion mit dem Mutein- und Redox-spezifischen Antikörper kann dann der Bereich des Redoxpotentials bestimmt werden. Dabei ist der Bereich durch die Unterschiede der Redox-Potentiale der Muteine und die Spezifität der Antikörper gegeben: Als Beispiel nehme man die Reaktion eines Antikörpers mit einem Mutein, das oxidiert vorliegt, während ein anderes Mutein noch vorwiegend durch reduziertes Mutein erkennenden Antikörper nachgewiesen wird. Besitzt ersteres Mutein etwa ein Redox-Potential von -250 mV, das zweite ein Redox-Potential von -240 mV, so liegt der wahre Wert des Redox-Potentials der Lösung zwischen -240 und -250 mV. Durch Mikroinjektion oder andere Verfahren zur intrazellulären Applikation des Antikörpers kann so auch das intrazelluläre Redox-Potential von Zellen abgeschätzt werden. Wird der Mutein-spezifische Antikörper mit einem Antikörper verbunden (bispezifischer Antikörper oder bispezifisches Antikörperfragment), der eine andere Spezifität besitzt und z.B. ein mit einem bestimmten Zell-Kompartment-assoziiertem Antigen wechselwirkt, so kann in dieser besonderen Variante des Verfahrens das Redox-Potential in bestimmten Kompartmenten der Zelle (z.B. dem Endoplasmatischen Retikulum, dem Zellkern, der Plasmamembran)gemessen werden.

Die Erfindung stellt in einer weiteren vorteilhaften Ausgestaltungsform auch eine Methode zur Verfügung, um biologisch nützliche Agenzien herzustellen, wobei ausgehend von der Analyse von Strukturen der Aminosäuresequenz gemäß SEQ ID Nr. 1 solche Agenzien identifiziert werden. Solche Unter-Strukturen oder Substrukturen können z.B. Epitope für die Herstellung von Antikörpern sein und die Methode umfasst die Entwicklung von Antikörpern gegen das Epitop. Die Substrukturen können auch Mutationsstellen beinhalten, welche die Bindungseigenschaften und die biologische Aktivität des Proteins verändern und die Methode enthält das Verfahren zur Einführung dieser Mutationen. Die Substruktur kann auch einen Verknüpfungspunkt für Anbindung einer seperaten chemischen Gruppe, wie z.B. ein Peptid, ein Polypeptid, ein Festkörpermaterial ("beads", Gele, chromatographische Medien, Chips, Platten, Glasträger), einen Linker und einen Marker (z.B. ein direkter Marker, etwa ein Fluorophor, oder einen indirekten Marker wie z.B. Biotin als Teil eines spezifischen Bindungspaars beinhalten.

In einer weiteren erfindungsgemäßen Anwendung wird insbesondere aufbauend auf einer strukturbasierten Analyse eine Methode zur Verfügung gestellt, um mögliche Metallkoordinierende Aminosäuren an bestimmten Positionen einzuführen (z.B. Cystein, Histidin, Glutamat).

In einer weiteren Anwendung der Erfindung wird eine Methode zur Verfügung gestellt, die eine effiziente Zuordnung der NMR-Resonanzsignale der Muteine ermöglicht. Insbesondere für Muteine, deren dreidimensionale Struktur nicht grundlegend gegenüber der Aminosäuresequenz gemäß SEQ ID Nr. 1 verändert ist, erlaubt der Vergleich mit dem charakterisierten Protein gemäß SEQ ID Nr. 1 ein schnelles Verfahren der Resonanzzuordnung. Aufgrund dieser Resonanzzuordnung kann eine Epitop-Kartierung von Bindungspartnern der Muteine vorgenommen werden. Das Screening von Substanzbibliotheken führt so zu einer Identifizierung der jeweiligen Bindungsstelle. Aufgrund eines Homologiemodeling kann die Bindungsstelle auch als dreidimensionales Modell realistisch wiedergegeben werden.

In einer anderen erfindungsgemäßen Anwendung wird eine Methode zur Verfügung gestellt, welche die Entwicklung von Liganden (Bindungspartnern) und molekularen "Scaffolds" ermöglicht. Aufbauend auf der Struktur und den NMR-Zuordnungen ist es möglich, die Struktur eines Muteinscaffold-Komplexes aufzuklären und die chemischen Strukturen zu identifizieren, die, wenn sie verändert werden, die Bindungsaffinität, die Bindungs-Spezifität oder beides zwischen Mutein und molekularem Scaffold verändern; und die als Grundlage für die Synthese eines Liganden dienen, der eine geänderte Bindungsaffinität, Bindungsspezifität oder beides besitzt. Mit molekularem "Scaffold" ist ein Kernmolekül gemeint, an welches eine oder mehrere chemische Gruppen kovalent verknüpft werden können. Die chemischen Gruppen umfassen, sind aber nicht begrenzt auf, Hydroxyl-, Methyl-, Nitro-, Carboxyl-Gruppen. Molekulare Scaffolds binden an mindestens ein Zielmolekül (z.B. das Polypeptid gemäß SEQ ID Nr. 1 und den Liganden für SEQ ID Nr. 1 oder ein Mutein), und dieses Zielmoleküle kann bevorzugt ein Protein oder Enzym sein. Vorteilhafte Eigenschaften eines "Scaffolds" umfassen die Bindung an ein Zielmolekül-Bindungstasche, so dass ein oder mehrere Substituenten des "Scaffolds" in einer Bindungstasche des Zielmoleküls binden; "Scaffolds" besitzen bevorzugt chemisch veränderbare Gruppen, insbesondere durch chemische Synthese, so dass kombinatorische Bibliotheken erzeugt werden können; "Scaffolds besitzen bevorzugt Positionen, an welche andere chemische Gruppen angeknüpft werden können, die nicht mit der Bindung des "Scaffolds" an das Zielmolekül (z.B. dem Protein bzw. Peptid gemäß SEQ ID Nr. 1) interferieren, so dass das "Scaffold" oder Mitglieder der "Scaffold"-abgeleiteten Bibliothek modifiziert werden können, so dass der "Scaffold"-Zielmolekül-Komplex vorteilhafte Eigenschaften besitzt (z.B. aktiver Transport in Zellen oder bestimmte Organe oder die Möglichkeit den Liganden an ein chromatographisches Material für die weitere Analyse zu binden).

(viii) Vorteile der Erfindung: Es ist überraschend, dass die anmeldungsgemäßen Aminosäuresequenzen, deren Muteine bzw. Proteinerkennungsdomänen die diese umfassen, eingesetzt werden können, um spezifisch Redox-abhängige Ziel- bzw. Targetmoleküle zu detektieren bzw. zu generieren.

Ebenso stellt die Erfindung eine Methode zur Verfügung, um Antikörper oder deren Antigen-bindende Fragmente nur gegen entweder die oxidierte oder reduzierte Form des Proteins über Tier-Immunisierungen, Hybridomazell-Linien oder Phage Display zu erhalten.

Die anmeldungsgemäßen Sequenzen und Domänen bzw. die mit diesen gewonnenen Mitglieder einer Polypeptid-Bibliothek können überraschenderweise als intra- und extrazelluläre Marker in der wissenschaftlichen Analyse aber auch in der Diagnose und Therapie von Krankheiten, die mit oxidativem Stress bzw. mit einem veränderten Redox-Gleichgewicht in den Zellen assoziiert sind, eingesetzt werden. Insbesondere können zelluläre Veränderungen, die mit pathologischen Veränderungen des Redox-Gleichgewichts und mit oxidativen Modifikationen von Proteinen einhergehen, zum einen detektiert aber zum anderen auch moduliert - d.h. gezielt verändert - werden.

Hierbei ist es beispielsweise möglich, dass in einem ersten Schritt eine molekulare Bibliothek auf Basis der erfindungsgemäßen Aminosäuresequenz bzw. der erfindungsgemäßen Proteinerkennungsdomäne, die auch als Redox-Scaffolds bezeichnet werden können, erzeugt werden. Diese Bibliothek kann dann mit einer Reihe klinisch relevanter Targetmoleküle gescreent bzw. durchmustert werden, so dass die Inhibition der Targetmoleküle in-vitro bzw. in-vivo detektiert und getestet werden kann. Ausgehend von den gewonnen Ergebnissen können dann in weitergehenden klinischen Studien im humanen Bereich zur Effizienz und Einsetzbarkeit die aus der molekularen Bibliothek isolierten rekombinanten Peptide oder Proteine getestet und als pharmazeutische Mittel entwickelt werden. Selbstverständlich ist es auch möglich, die gewonnenen Proteine oder Peptide als analytisches Werkzeug für die in-vitro und in-vivo Analyse zellulärer Redoxvorgänge zu verwenden.

Neben der linearen Sequenzabfolge der erfindungsgemäßen Aminosäuresequenz stellt insbesondere die räumliche Struktur derselben einen wesentlichen Aspekt der Erfindung dar. Die Struktur ist die rationale Grundlage für die Erzeugung einer molekularen Bibliothek.

Die vorteilhaften Eigenschaften der erfindungsgemäßen Polypeptide sind die Basis dafür, dass eine molekulare Bibliothek erzeugt werden kann, die eine Vielzahl von $10^8$ bis $10^{10}$ verschiedenen Muteine der Aminosäuresequenz gemäß SEQ ID Nr. 1 enthält.

Der anmeldungsgemäßen Lehre liegt demgemäß die Kenntnis zugrunde, dass Proteine oder Peptide eine herausragende Rolle in allen biologischen Systemen spielen. Als zelluläre Exekutive können sie dabei entweder eine enzymatische Funktion wahrnehmen, eine Rolle als Struktur- oder Stützprotein spielen oder aber über vielfältige Wechselwirkungen mit DNA, Lipiden, Zuckermolekülen oder anderen Proteinen regulatorische Funktionen übernehmen. Auf Grund dieser großen Bedeutung von Proteinen ist es in Wissenschaft und Klinik entscheidend, molekulare Marker zur Untersuchung von Proteinen zur Hand zu haben. Die anmeldungsgemäßen Aminosäuresequenzen bzw. Proteinerkennungsdomänen oder die mit Hilfe dieser gewonnenen Mitglieder einer Polypeptid-Bibliothek können als solche Marker in den verschiedensten Bereichen der Medizin, Biologie oder Chemie eingesetzt werden, etwa bei mikroskopischen Untersuchung von Proteinen, bei der Identifizierung von Proteinen aus größeren Komplexen oder als Bindungsmoleküle in der Protein-Chip-Technologie. Da bekannt ist, dass eine sehr große Anzahl von Proteinvermittelten Prozessen über Mechanismen gesteuert wird, die vom Redox-Potential innerhalb der Zelle abhängig sind, können die erfindungsgemäßen Aminosäuresequenzen bzw. Proteinerkennungsdomänen vorteilhafterweise als molekulare Sonden für die Untersuchung und Steuerung dieser Mechanismen verwendet werden. Weil oxidativer Stress ein wichtiger Faktor bei der Entstehung von Tumoren ist und einen wesentlichen epigenetischen Faktor im Alterungsprozess darstellt, können die erfindungsgemäßen Erzeugnisse selbstverständlich auch als molekulare Sonden innerhalb dieser Prozesse eingesetzt werden.

Die anmeldungsgemäße Lehre zeichnet sich durch folgende Merkmale aus:

- Abkehr vom technisch Üblichen
- neue Aufgabenstellung
- Vorliegen eines seit langem ungelösten dringenden Bedürfnisses für die Lösung des mit der Erfindung gelösten Problems
- bisheriges vergebliches Bemühen der Fachwelt
- Einfachheit einer Lösung, insbesondere weil sie kompliziertere Lehren ersetzt
- Entwicklung der wissenschaftlichen Technik ging in eine andere Richtung
- entwicklungsstraffende Leistung
- Fehlvorstellungen der Fachwelt über die Lösung des entsprechenden Problems (Vorurteil)
- technischer Fortschritt, wie z. B.: Verbesserung, Leistungssteigerung, Verbilligung, Ersparnis an Zeit, Material, Arbeitsstufen, Kosten oder schwer beschaffbaren Rohstoffen, erhöhte Zuverlässigkeit, Beseitigung von Fehlern, Qualitätshebung, Wartungsfreiheit, größere Effektivität, höhere Ausbeute, Vermehrung der technischen Möglichkeiten, Bereitstellung eines weiteren (nicht notwendig besseren) Mittels, Eröffnung eines zweiten (nicht notwendigerweise besseren) Weges, Eröffnung eines neuen Gebietes, erstmalige Lösung einer Aufgabe, Reservemittel, Alternativen, Möglichkeit der Rationalisierung, Automatisierung oder Miniaturisierung oder Bereicherung des Arzneimittelschatzes
- glücklicher Griff (aus einer Vielzahl von Möglichkeiten wurde eine bestimmte gewählt, deren Ergebnis nicht vorausgesagt werden konnte)
- Irrtum in Entgegenhaltungen

- junges Gebiet der Technik
- Kombinationserfindung, d.h. mehrere bekannte Elemente werden zu einer Kombination zusammengeführt, die einen überraschenden Effekt aufweist
- Lizenzvergabe
- Lob der Fachwelt und/oder
- wirtschaftlicher Erfolg.

Auch die Informationsverarbeitung gesunder Zellen wird in vielfacherweise durch Oxidations- oder Reduktionsprozesse (Redox-Gleichgewichte) reguliert. Dabei wirken die Redox-abhängigen Änderungen auf der Ebene von Modifikationen von Nukleinsäuren, Lipiden, Zuckern und insbesondere auch von Proteinen. Insbesondere die Charakterisierung der Veränderung von Proteinen - mittels der erfindungsgemäßen molekularen Sonden - durch die Änderung des Redox-Potentials und die Verfügbarkeit reaktiver Sauerstoffspezies (ROS) ermöglicht die Detektion, aber auch die Modifikation einer Vielzahl biologisch interessanter und medizinisch relevanter ROS-modifizierter Proteine. Die erfindungsgemäßen molekularen Sonden sind daher Marker, die das Redox-Potential in lebenden Zellen, Geweben oder Organismen zum einen charakterisieren und zum anderen modulieren können. Die erfindungsgemäßen Peptide können somit als Sonden bzw. Marker in der Analyse eingesetzt werden, um Redox-sensitive Wechselwirkungen auf Zelloberflächen oder innerhalb der Zellen zu verfolgen. Aus der Analyse der Redox-modifizierten Proteine ergibt sich auch die Möglichkeit, therapeutische Proteine zu detektieren, die krankheitsrelevante Redox-Potentialabhängige Mechanismen unterbinden können. Insbesondere Proteinwirkstoffe, welche spezifisch die bei der Oxidation veränderten pathologischen Mechanismen unterbinden können, sind mit den anmeldungsgemäßen Aminosäuresequenzen, insbesondere mit den Mitgliedern der Polypeptid-Bibliothek, detektier- und modifizierbar. Solche spezifischen Protein-Biologicals waren bisher im Stand der Technik noch nicht bekannt. Die erfindungsgemäßen molekularen Sonden, d.h. die erfindungsgemäßen Aminosäuresequenzen bzw. Peptide, insbesondere die Polypeptid-Bibliothek und deren Muteine aber auch die Proteinerkennungsdomänen sowie die funktionsanalogen Derivate und strukturhomologen Makromoleküle, weisen zahlreiche vorteilhafte Eigenschaften für den Einsatz als molekulare Marker/Sonden auf. Sie sind z.B. sehr gut wasserlöslich, thermisch stabil und tendieren nicht zur Aggregation. Weiterhin sind sie in beiden Zuständen (oxidiert, reduziert) löslich und daher in der gesamten Breite ihrer Anwendung löslich schaltbar. Sie sind sehr klein und können demgemäß gut durch Gewebe dringen. Da sie humanen Ursprungs sind, können sie in Menschen eingesetzt werden, ohne dass es zur Immunreaktion kommt. Insbesondere ist es vorteilhaft, dass ihr Redox-Zustand reversibel regulierbar ist. Das heißt, dass zwei unterschiedliche Zustände der erfindungsgemäßen Aminosäuresequenzen, Peptide oder Proteine existieren, die sich in ihrer Struktur unterscheiden und es daher erlauben, die molekulare Markierung oder therapeutische Inhibition entweder im reduzierten oder im oxidierten Zustand vorzunehmen. Des Weiteren hat sich gezeigt, dass die entsprechenden Aminosäuresequenzen sehr gut in E.coli exprimierbar sind. Die gewonnenen erfindungsgemäßen Aminosäuresequenzen sind über ein einfaches Reinigungsschema gut zu isolieren.

Eine erfindungsgemäße Anwendung ist die kovalente Verknüpfung von der Aminosäuresequenz gemäß SEQ ID Nr. 1 über einen Linker (peptidisch oder nicht-peptidisch) mit einem "caged" compound, welcher Xenon, ein Fluorophor oder andere als Sonden in der Biologie und Medizin einsetzbare Marker-Atome oder Moleküle enthält. Hyperpolarisiertes Xenon stellt als Gas ein sehr vielverprechendes und in der Medizin auch schon eingesetztes Agens zur Detektion von magentischen Resonanz-Signalen in der NMR-Spektroskopie und im MR-Imaging dar. Im Falle des hpyerpolarisierten Xenons kann die Konformationsänderung zwischen oxidiertem und reduziertem Zustand über eine Änderung des Xenon-Signals, welches entweder über die kovalente Verknüpfung des caged compounds oder aber direkt ans Protein gebunden wird, detektiert werden (Spence et al., J. Am. Chem. Soc. 126, 15287-15294 (2004); Lowery et al., Angew. Chem. Int. Ed. 43, 6320-6322 (2004)). Im letzteren Fall werden Mutationen einer hydrophoben Aminosäure, etwa Methionin, Leucin, Valin, Isoleucin, Phenylalanin, Tyrosin oder Tryptophan durch Alanin vorgenommen und eine Oligo-Tyrosin-Sequenz an den C-Terminus des Proteins anhängt.

Entsprechend der erfindungsgemäßen Anwendung kann ein auf ein spezielles Target-Molekül (wie z.B. NFkB, SHP-2) gerichtetes -Mutein eine Methode zur Verfügung stellen, bei der das Mutein in bestimmte Zellen und Gewebe eingeschleust, bzw. dort freigesetzt wird. Dazu stehen dem Fachmann verschiedene Methoden zur Verfügung, z.B. die Einbettung des Muteins in Liposomen oder Polymer-Materialien mit anschließender Zell-spezifischer Erkennung (z.B. durch Antikörper-Moleküle) und Freisetzung des Muteins.

Die erfindungsgemäße Anwendung umfasst pharmakologische Zusammensetzungen, welche das Polypeptid SEQ ID Nr. 1 oder ein Mutein oder ein Apoprotein umfassen. So kann z.B. das durch SEQ ID Nr. 1 oder eines der Muteine definierte Polypeptid mit einem physiologisch verträglichen Medium gemischt werden. Solche Medien umfassen aber sind nicht beschränkt auf, wässrige Lösungen, Salzlösungen, Polyole (z.B. Glycerol, Propylen-Glykol, flüssiges PolyethylenGlykol) und Dextrose-Lösungen. Die optimale Konzentration der aktiven Komponenten können empirisch entsprechend dem Fachmann wohl bekannten Methoden bestimmt werden und hängen von der jeweiligen gewünschten Zusammensetzung der Pharmaka ab. Methoden zur Applikation des exogenen Polypeptids (insbesondere von SEQ ID Nr. 1 und dessen Muteinen) an die Stelle der krankheitsrelevanten Behandlung umfassen, aber sind nicht beschränkt auf intradermale, intramuskuläre, intraperitoneale, intravenöse, subkutane, orale und intranasale Anwendungen. Andere Methoden können die Gentherapie, wiederbeladbare biologisch abbaubare Partikel, virale Vektoren, nackte DNA und Polymere umfassen. Die pharmazeutische Formulierung dieser Erfindung kann auch als Teil einer kombinierten Therapie mit anderen Agenzien verabreicht werden. Die Sequenz gemäß SEQ ID Nr. 1 oder der Muteine kann auch durch die Methoden der Gentherapie oder ex vivo in Zellen für die Expression in einem Wirbeltier (einschließlich Homo sapiens) eingeführt werden.

Die Erfindung betrifft auch einen Kit, der die erfindungsgemäßen Verbindungen umfasst, gegebenenfalls mit einer Information zum Kombinieren der Inhalte des Kits. Die Informationen zum Kombinieren der Inhalte des Kits betreffen die Verwendung des Kits zur Prophylaxe und/oder Therapie von Erkrankungen. Die Informationen können sich beispielsweise Die Erfindung betrifft auch ein Vaccin umfassend das erfindungsgemäße Polypeptid, die erfindungsgemäße Polypeptid-Bibliothek und/oder die erfindungsgemäße Proteinerkennungsdomäne, gegebenenfalls zusammen mit einem pharmazeutisch verträglichen Träger.

auch auf ein Therapieschema beziehen, d. h. auf ein konkretes Injektions- oder Applikationsschema, auf die zu verabreichende Dosis oder anderes. Die Informationen hierzu müssen nicht direkt im Kit befindlich sein, sondern im Kit kann auch eine Internetadresse vorhanden sein, die den Anwender auf Informationen im Internet weiterleitet, die sich auf eine Therapieschema oder anderes beziehen.

Im Folgenden soll die Erfindung an Hand von Beispielen näher erläutert werden, ohne auf diese Beispiele beschränkt zu sein.

Protein-Expression:

Die Sequenz der hSH3-1-Domäne des humanen ADAP-Proteins (SwissProt 015117, Aminosäuren 485 bis 579) wurde in den Vektor pET24d (Novagen) über die NcoI und XhoI-Restriktions-Schnittstellen subkloniert. Das Konstrukt enthält am N-Terminus zusätzlich die Aminosäure Methionin, und am C-Terminus die Aminosäuren Leucin und Glutamat und einen hexa-Histidin-tag.

Das Plasmid mit der ADAP-hSH3-1-Domäne wurde in den *E. coli* Stamm BL21 (DE3) (Invitrogen) transformiert. Eine Vorkultur wurde in 5 ml LB-Medium, das 34 mg/ml Kanamycin enthielt, bei 37 °C, 185 rpm für 12 Stunden inkubiert. Anschliessend wurde entweder eine 1 Liter LB-Hauptkultur so überimpft, dass eine $OD_{600}$ von 0.05 in der neuen Kultur gemessen wurde (Expression nicht-isotopenmarkierten Proteins), oder 0.5 ml dieser Vorkultur wurden in 100 ml $^{15}$N- oder $^{15}$N/$^{13}$C-markiertes Minimalmedium verdünnt. Das definierte Minimalmedium enthält 60 mM $K_2HPO_4$, 11 mM $KH_2PO_4$, pH = 7.4, 2 μM Natriumcitrat, 0.8 % (w/v) Glukose ($^{13}$C-markiert für die Expression $^{13}$C-markierten Proteins), 1 mM $MgSO_4$, 0.75 g/l $NH_4Cl$ $^{15}$N-markiert für $^{15}$N-markiertes Protein) und 34 mg/ml Kanamycin. Im Falle des Wachstums in LB-Medium wurde die Kultur bei einer $OD_{600}$ von 0.5 mit 1mM IPTG versetzt und für weitere 3 Stunden wachsen gelassen. Dann wurde die Kultur bei 4° C, 6000 rpm für 20 Minuten zentrifugiert. Das Zell-Pellet wurde bei -70° C gelagert.

Im Falle des Wachstums in Minimalmedium wurde die 100 ml-Kultur bei Erreichen einer $OD_{600}$ von 2.0 (nach ca. 12h) mit weiteren 900 ml frischen Mediums verdünnt und weiter bei 37° C und 185 rpm inkubiert. Bei einer $OD_{600}$ von 0.5 wurden die Zellen mit IPTG versetzt (1mM End-Konzentration im Medium), für weitere 4.5 Stunden inkubiert und bei 4° C, 6000 rpm für 20 Minuten zentrifugiert. Das Zell-Pellet wurde bei -70° C gelagert.

Das Zell-Pellet wurde in 20 ml NiHiTrap-Puffer A (enthaltend: 20 mM NaPPi, pH = 7.4, 500 mM NaCl, 20 mM Imidazol) resuspendiert und auf Eis mit Ultraschall zehn mal bei einer Leistung von maximal 120W für 30 Sekunden behandelt, wobei zwischen jedem der Ultraschall-Schritte eine Minute gewartet wurde. Das Zell-Lysat wurde dann für 30 Minuten bei 4° C und 24500 rpm zentrifugiert. Der Überstand wurde durch einen 0.2 μm Spritzen-Filter passiert und auf eine NiHiTrap-Säule (5 ml Volumen, Pharmacia) aufgetragen. Nach dem Waschen mit 20 mM $NaPP_i$, pH = 7.4, 500 mM NaCl, 50 mM Imidazol (8 Säulenvolumen) wurde zur Elution ein linearer Gradient von 10% bis 100% NiHiTrap Puffer B (enthält 20 mM $NaPP_i$, pH = 7.4, 500 mM NaCl, 400 mM Imidazol) gefahren, mit insgesamt 28 Säulenvolumen. Die hSH3-1-Domäne eluierte in den Fraktionen 26 - 34 bei ca. 35% Puffer B. Die Detektion des eluierten Proteins erfolgte über SDS-PAGE und Coomassie-Blau-Färbung. Die relevanten Fraktionen wurden vereinigt und auf eine Protein-Konzentration von 1 mM mittels Centriprep-Zentrifugationsfiltern (MW-cutoff: 3 kDa) eingeengt. Für die NMR-Messungen fand ein Puffer-Austausch gegen 50 mM $NaPP_i$, pH = 7.0, 150 mM NaCl, 0.05 % $NaN_3$-Lösung statt. Um das Protein in die reduzierte Form zu bringen, wurde zwischen 0.2 und 4 mM DTT zugegeben, zur Überführung in die oxidierte Form wurden zwischen 0.2 und 4 mM $H_2O_2$ zu der Proteinlösung zugegeben.

Expression von GST-hSH3-1: Die Aminosäuren 490-579 des ADAP-Proteins (SwissProt 015117) wurden über BamH1/Xhol in den Vektor pGEX4-T1 kloniert und das Plasmid anschließend in *E. coli* BL21 (DE3) transformiert. Die Expression erfolgte wie weiter oben für das His-verknüpfte Protein beschrieben. Zur Aufreinigung wurde das Zell-Pellet in 1xPBS (140 mM NaCl, 2,7 mM KCl, 10 mM $Na_2HPO_4$, 1,8 mM $KH_2PO_4$ pH = 7.3) resuspendiert und ultraschallbehandelt (s.o.) und der filtrierte Überstand auf eine Glutathion-Sepharose Säule (GSTrap 5 ml, Pharmacia) gegeben. Das Protein wurde über einen Schritt-Gradienten (5 SV 12,5%, 5 SV 25%, 2 SV 100% Puffer B, 50 mM Tris pH = 8.0, 40 mM Glutathion red.) eluiert. Die Domäne eluierte in den Fraktionen 10 - 18 bei 25 % Puffer B. Die Detektion des eluierten Proteins erfolgte über SDS-PAGE und Coomassie-Blau-Färbung. Die relevanten Fraktionen wurden vereinigt und gegen 1xPBS dialysiert. Um den GST-Tag abzutrennen, wurden je 25 mg Protein mit 50 U Thrombin (Merck Bioscience) über Nacht bei 8° C inkubiert. Die Probe wurde dann mittels einer Vivaspin-Zentrifugeneinheit (MWC 3 kDa) auf 2 ml konzentriert und über eine Gelfiltrationssäule (Superdex 75, Pharmacia) getrennt. Die hSH3-1-Domäne eluierte nach ca 80 ml. Die Detektion der Proteine erfolgte wie oben beschrieben. Im Folgenden wurde die Domäne wie das his-verknüpfte Protein behandelt.

NMR-Spektroskopie

Zur Aufklärung der Struktur der ADAP hSH3-1-Domäne wurden eine Reihe von NMR-spektroskopischen Experimenten durchgeführt. Alle Experimente wurden bei einer Probentemperatur von 300 K mit einer Proteinkonzentration von 0,2 - 1,5 mM gemessen. Die Spektren wurden an einem Bruker Avance 600-Spektrometer mit Cryo-Probenkopf oder an einem Bruker DMX600-Spektrometer mit Triple-Resonanz-Probenkopf durchgeführt.

Für die Rückgrat-Resonanz-Zuordnung wurde ein Standard-Paar von CBCANNH/CBCACONNH-Spektren (Grzesiek et al., J. Magn. Res. 1992, 99, 201; Grzesiek et al., J. Am. Chem. Soc. 1992, 114, 6291) mit einer einheitlich $^{15}$N/$^{13}$C-markierten Probe aufgenommen und es wurde jeweils ein nahezu komplettes Rückgrat-Assignment für die oxidierte und reduzierte Form der ADAP hSH3-1-Domäne erhalten. Die Reste 1-11 und 91-102 in beiden Formen sind in den Spektren nicht sichtbar, vermutlich aufgrund von erhöhter Flexibilität und damit verbundener geänderter Relaxationseigenschaften dieser Reste. Für die Seitenketten-Zuordnung wurde insbesondere das HCCH-TOCSY-Spektrum verwendet. Es konnten mehr als 95% der Seitenketten-Resonanzen sowohl für die reduzierte als auch für die oxidierte Form des Proteins zugeordnet werden. Ausgehend von der fast vollständigen Zuordnung der Kern-Resonanzen der ADAP-hSH3-1-Domäne wurden anschließend die Signale in den $^{15}$N-NOESY-HSQC, $^{13}$C-HMQC-NOESY und 2D-$^1$H-NOESY-Spektren zugeordnet, welche die zur Strukturberechnung erforderlichen Abstandsinformationen liefern (s. u.) Verwendete Software: TOPSPIN (Bruker, Rheinstetten, Deutschland); ANSIG 3.3, P. Kraulis, ANSIG: A Program for the Assignment of Protein 1H 2D NMR spectra by Interactive Graphics, J. Magn. Reson. (1989) v 24, pp 627-633)

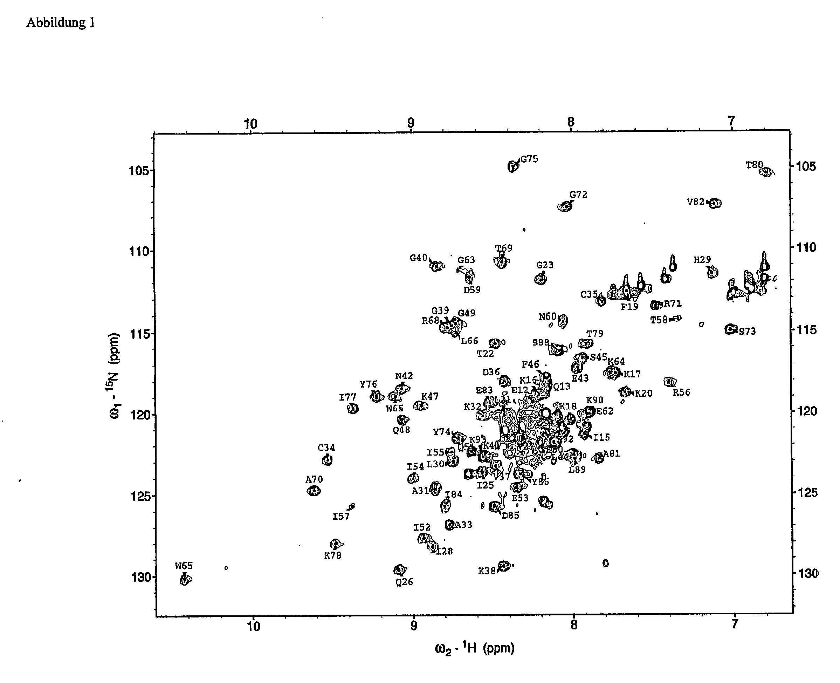

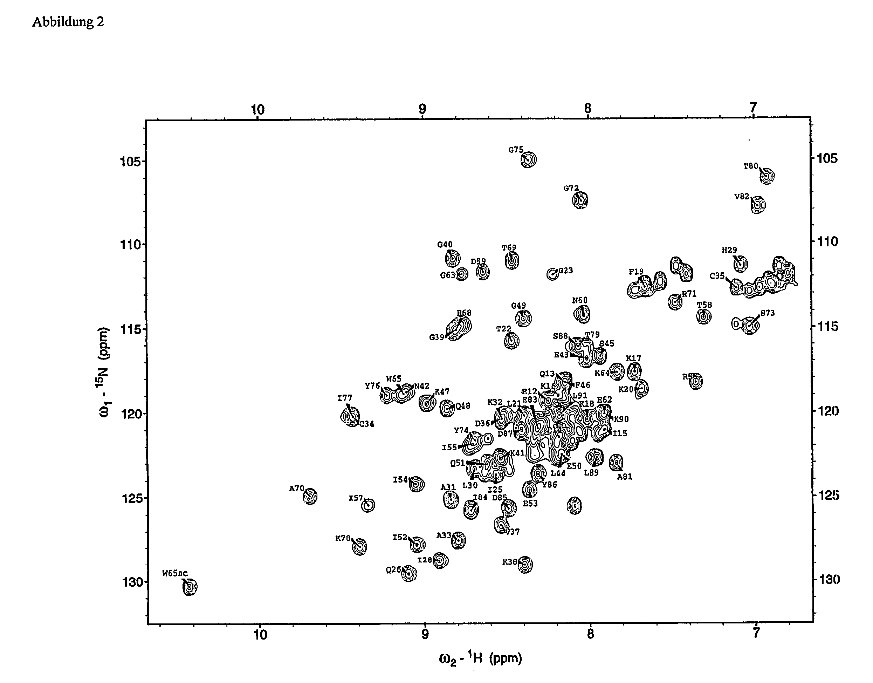

Die Rückgratzuordnung ist in Abb. 1 und 2 am Beispiel des $^{15}$N-$^1$H-Korrelationsspektrums ($^{15}$N-HSQC) dargestellt. Jedes Signal in diesem zweidimensionalen Experiment stammt von einer bestimmten Rückgrat-NH-Gruppe des Proteins (ausgenommen die NH2-Gruppen von Asparagin und Glutamin und die NH-Gruppe des Tryptophan). Die NH-Gruppe der Seitenkette des Tryptophan-Restes 65 ist mit der Erweiterung sc (side chain) gekennzeichnet.

Während beide Formen der ADAP hSH3-1-Domäne sich in Lösung ähnlich verhalten, was ihre Oligomerisation betrifft, zeigen die $^{15}$N-HSQC-Spektren der beiden Formen deutliche Unterschiede (s. Abb. 1 und 2). Dies deutet darauf hin, daß sie sich auch strukturell unterscheiden.

Die Unterschiede der beiden Spektren sind in Abb. xy dargestellt. Dazu wurde die kombinierte chemische Verschiebung für einen Rest nach Formel 2 (Hajduk et al., J. Med. Chem. 1997, 40, 3144) berechnet:

$$\Delta = |\Delta(\delta^1 H)| + 0,2 * |\Delta(\delta^{15}N)|$$

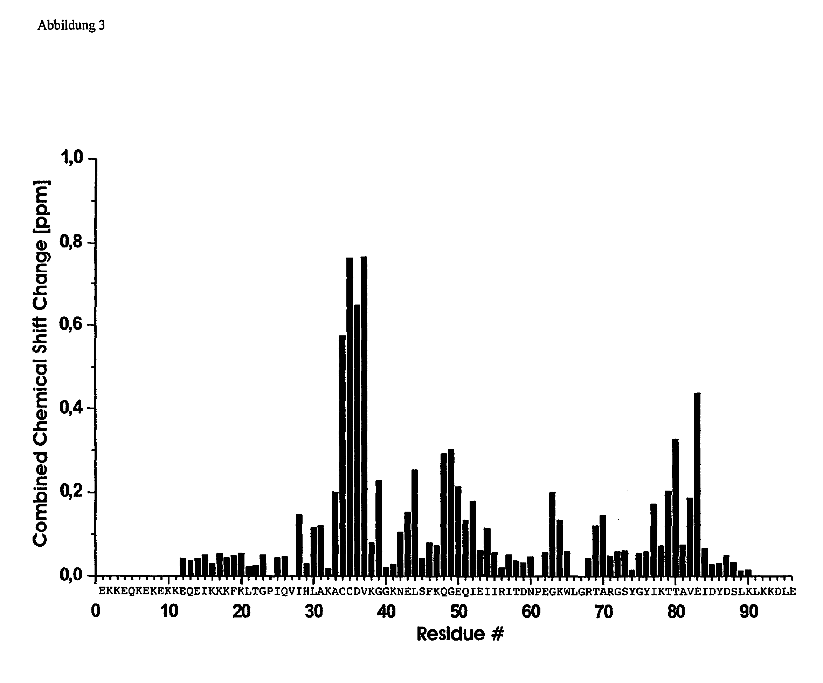

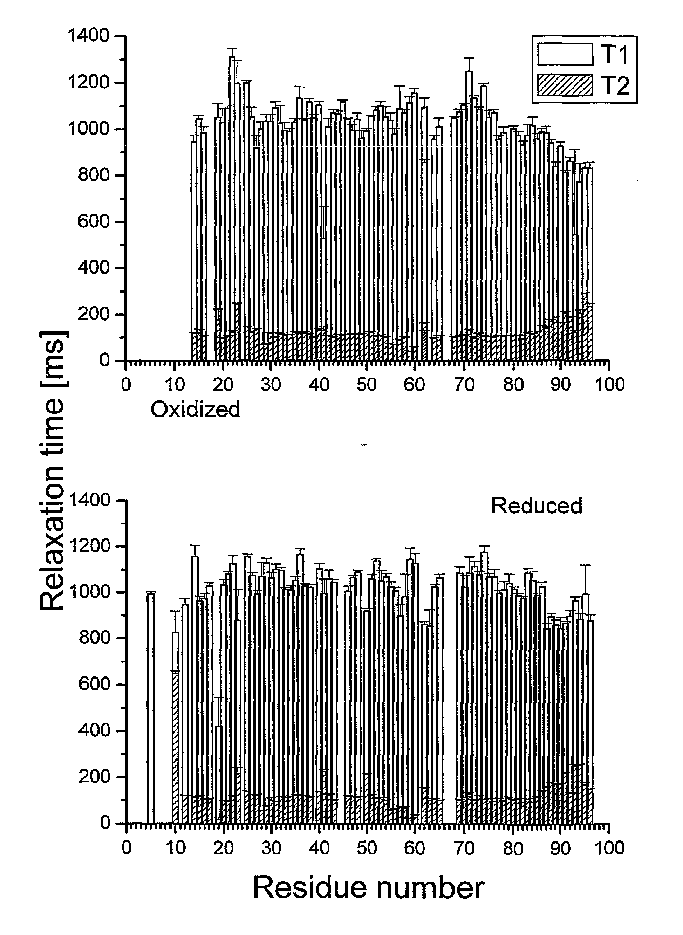

hier ist $\delta^1$H die chemische Verschiebung der Rückgrat-Protonen, $\delta^{15}$N die chemische Verschiebung der Rückgrat-Stickstoffatome. Die deutlichsten Unterschiede sind in der Region um die beiden Cystein-Reste erkennbar (s. Abb. 3).

Basierend auf der Rückgratzuordnung wurden die Relaxationseigenschaften der Rückgrat-NH-Gruppen der beiden Formen des Proteins gemessen. Diese erlauben eine Aussage über die interne Flexibilität der Domäne und die Bestimmung des Oligomerisationsgrades des Proteins unter den gegeben Puffer- und Konzentrationsbedingungen.

Zu diesem Zweck wurde eine Serie von $^{15}$N $T_1$- und $T_2$-Relaxationsexperimenten (Farrow et al., Biochemistry 1997, 36, 2390) gemessen ($T_1$ und $T_2$ sind charakteristische Relaxationszeiten). Zur Messung von $T_1$ wurde der Delay (ein experimenteller Parameter) in sukzessiven Experimenten auf 14, 70, 140, 210, 280, 420, 560, 840, 1.260, 2.800 und 7.000 $\mu$s gesetzt, in den analogen $T_2$-Experimenten wurde der entsprechende Delay auf 6, 12, 24, 36, 48, 60, 120, 180, 240, 300 und 360 $\mu$s gesetzt. Die resultierenden Spektren sind einem $^{15}$N-HSQC ähnlich. Für jeden Rest des Proteins wird die Signalintensität in jedem Spektrum bestimmt und gegen den Delay aufgetragen.

Durch Regression mit einer einfach exponentiellen Funktion werden für jeden Rest die $T_1$- und $T_2$-Relaxationszeiten bestimmt, die erhaltenen Werte sind in Abbildung 4 gezeigt. Für die Regression wurde das Programm Sparky benutzt (Version 3.1, T.D. Goddard und D. G. Kneller, SPARKY 3, University of California, San Francisco).

Aus der weitgehend einheitlichen Verteilung der $T_1$- und $T_2$-Relaxationszeiten innerhalb der beiden Formen der ADAP hSH3-1-Domäne kann geschlossen werden, dass das Protein jeweils eine stabile Domäne mit geringer interner Mobilität bildet.

Weiterhin kann aus den Werten der $T_1$- und $T_2$-Relaxationszeiten der Oligomeristaionsgrad der Proteine abgeleitet werden. Dazu wird jeweils der Mittelwert der $T_1$- und $T_2$-Relaxationszeiten berechnet und mit Hilfe der nachstehenden Formel die Korrelationszeit der Rotation der Proteine berechnet. Die Korrelationszeit ist ein Maß für das Molekulargewicht eines Körpers und erlaubt daher Rückschlüsse auf eine mögliche Oligomerisierung der Proteine(Gryk et al., J. Mol. Biol. 1998, 280, 879):

$$\tau_C = \frac{\left\{\left[6\left(T_1/T_2\right) - 7\right]\frac{1}{4}\right\}^{1/2}}{\Omega_N \bullet 2\pi} \quad (1)$$

hier sind $T_1$ und $T_2$ die mittleren $^{15}N$-Relaxationszeiten und $\Omega_N$ ist die Larmorfrequenz für $^{15}N$ bei einer gegebenen magnetischen Feldstärke (hier ist $\Omega_N = 60.827865$ MHz).

Tabelle 1 zeigt die Werte für die mittleren $T_1$- und $T_2$-Relaxationszeiten der beiden Formen der ADAP hSH3-1-Domäne sowie die daraus berechneten Korrelationszeiten $\tau_C$:

|  | $T_1$ [ms] | $T_2$ [ms] | $\tau_C$ [ns] |
|---|---|---|---|
| Reduziert | 1.010,92 | 126,44 | **8,4** |
| Oxidiert | 1.012,56 | 122,39 | **8,5** |

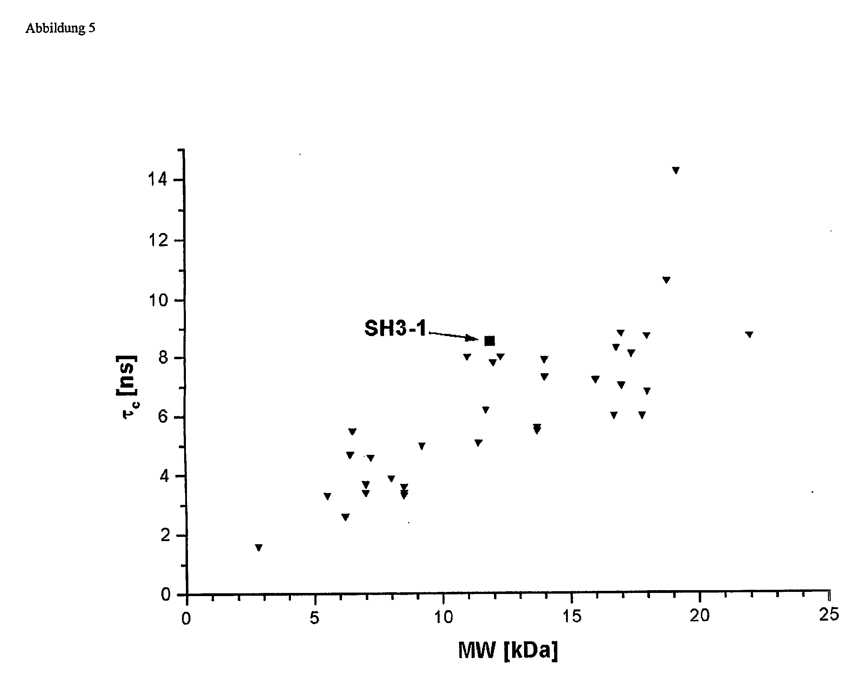

Im Rahmen der Messgenauigkeit sind die Korrelationszeiten für beiden Formen der Domäne gleich. Das erlaubt den Schluss, das beide Formen auch den gleichen Oligomerisationsgrad haben. Der Vergleich mit Korrelationszeiten anderer Proteine, deren Oligomeristaionsgrad bekannt ist, zeigt, dass die ADAP hSH3-1-Domäne sowohl in der reduzierten als auch in der oxidierten Form als Monomer vorliegt (s. Abbildung 5). Dies ist selbst bei den unphysiologisch hohen Konzentrationen der Fall, unter denen die NMR-Experimente durchgeführt wurden. Man darf mit Sicherheit davon ausgehen, dass die Domäne bei niedrigeren Konzentrationen ebenfalls als Monomer vorliegt.

Bestimmung des Redox-Potentials:

Das Redox-Potential der Umwandlung der ADAP hSH3-1-Domäne von der reduzierten in die oxidierte Form wurde durch eine NMR-Messung bestimmt. Das Protein wurde in der oxidierten Form vorgelegt (Konzentration 0,1 mM). Der Standard-NMR-Puffer (s.o.) wurde zusätzlich mit 0,2 mM oxidiertem Glutathion (GSSG) versetzt, sukzessive wurde dann reduziertes Glutathion (GSH) zugesetzt. Die Konzentrationen sind in der Tab. genannt:

| Exp.-Nr. | 1 | 2 | 3 | 4 | 5 | 6 | 7 | 8 | 9 | 10 | 11 |
|---|---|---|---|---|---|---|---|---|---|---|---|
| GSH [mM] | 0,2 | 0,5 | 1,0 | 1,5 | 2,0 | 2,5 | 3,0 | 5,0 | 7,5 | 10,0 | 15,0 |
| GSSG [mM] | 0,2 | 0,2 | 0,2 | 0,2 | 0,2 | 0,2 | 0,2 | 0,2 | 0,2 | 0,2 | 0,2 |

Über diese Serie von $^{15}N$-HSQC-Experimenten wurde dann die Intensität geeigneter Signale verfolgt. Geeignet sind hier solche Reste, deren Signale in den Spektren der ganz oxidierten und ganz reduzierten Form nicht überlappen und die auch nicht mit Signalen anderer Reste überlappen. Ausgewählt wurden daher die Reste Cys 34, Cys 35, Gln 48, Gly 49 und Thr 80.

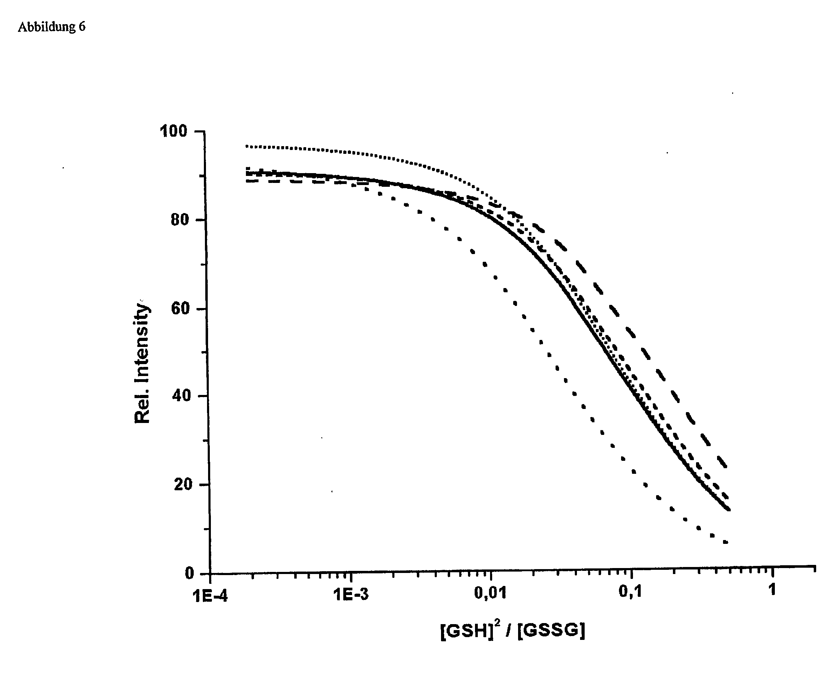

Abb. 6 zeigt den Verlauf der Signalintensitäten dieser Reste, aufgetragen gegen $[GSH]^2/[GSSG]$. Hierfür wurde die jeweils höchste Intensität willkürlich auf 100 skaliert. Die verschiedenen Reste zeigen ein vergleichbares Verhalten.

Die Regression liefert einen Wert von 0,05 für den Wendepunkt der Kurven. Daraus lässt sich mit Hilfe der Nernst'schen Gleichung das Redox-Potential der Domäne berechnen:

$$E = E_0 - \frac{RT}{zF} \ln \frac{[GSH]^2}{[GSSG]}$$

hier ist $E_0 = -240$ mV (Standard-Potential für GSH/GSSG), $R = 8,314$ JK$^{-1}$mol$^{-1}$ (ideale Gaskonstante), $T = 300$ K, $z = 2$ (Anzahl der übertragenen Elektronen) und $F = 9,648 \cdot 10^4$ (Faraday-Konstante). Als Redox-Potential wird $-204$ mV berechnet.

Dieser Wert liegt im Bereich des intrazellulären Milieus, wie es in verschiedenen Stadien der Zellreifung vorliegt. In ruhenden Zellen liegt das GSH/GSSG-Redox-Potential bei ca. -150 mV, unter diesen Bedingungen wird die hSH3-1-DOmäne bevorzugt im reduzierten Zustand vorliegen. In sich teilenden Zellen liegt das Potential bei ca. -200 mV, in apoptotischen Zellen kann es auch bei -250 mV liegen. Hier wird die hSH3-1-Domäne im oxidierten Zustand vorliegen.

Bestimmung der Schmelzpunkte

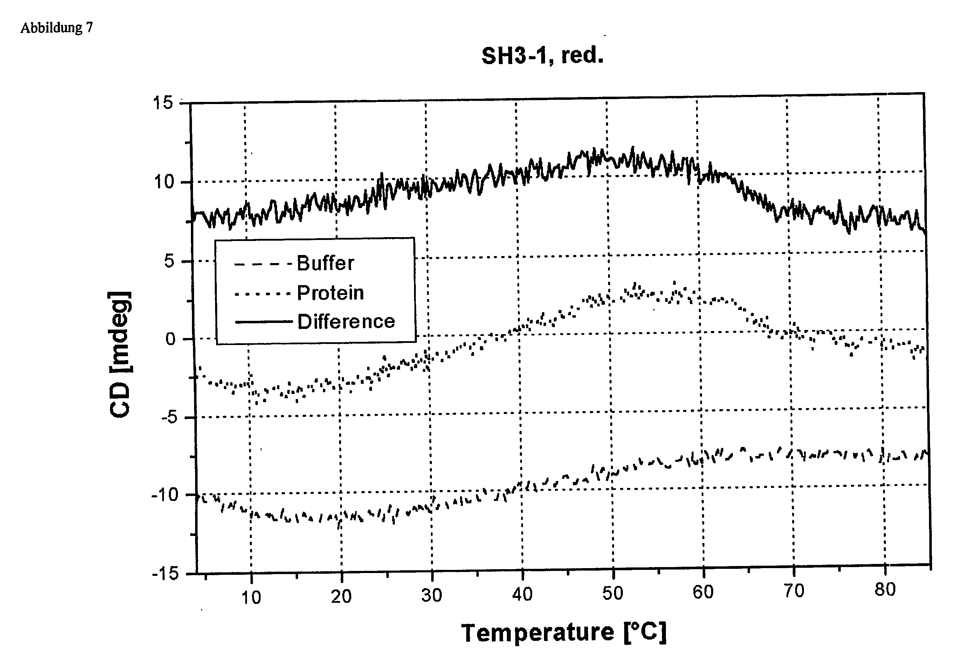

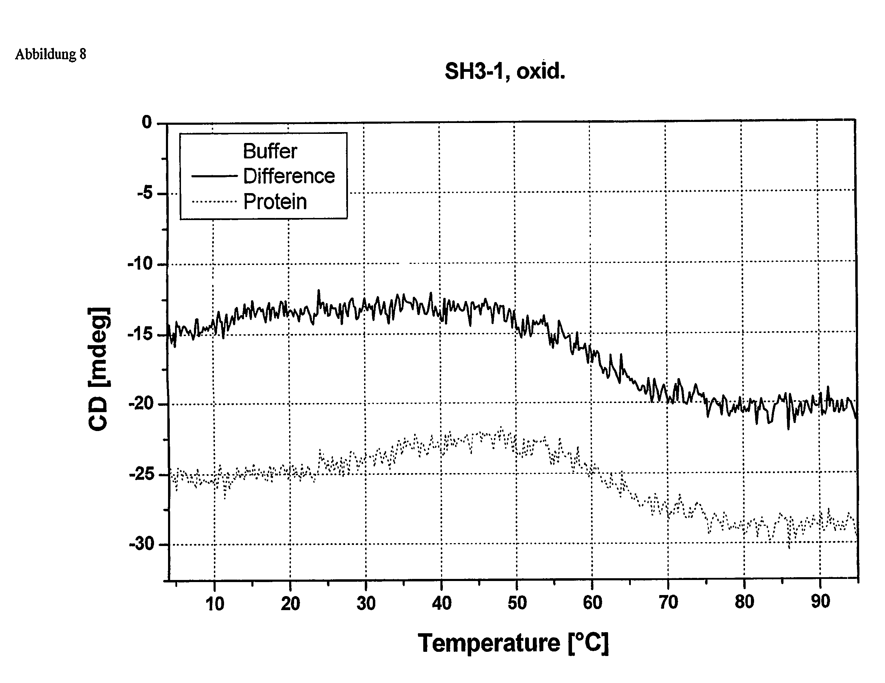

Mit Hilfe von CD-Spektroskopie wurde die TemperaturStabilität der reduzierten und oxidierten Form der ADAP hSH3-1 Domäne untersucht. Dazu wurde jeweils eine 15 μM Probe in NMR-Puffer in ein CD-Spektrometer (Jasco 720) eingebracht. Das CD-Signal wurde bei 221 nm verfolgt, während die Temperatur der Probe kontinuierlich von +4° auf +95° C erhöht wurde. Die Temperaturkurve des Puffers wurde von der so erhaltenen Kurve abgezogen. Die Ergebnisse sind in den Abb. 7 und 8 dargestellt. Beide Formen der ADAP hSH3-1 Domäne haben einen Schmelzpunkt von ca. 60° C und zeigen damit eine hohe thermische Stabilität.

Basierend auf der Auswertung der NOESY-Spektren (s.o.) wurden die Strukturen der reduzierten und oxidierten Form der ADAP hSH3-1-Domäne berechnet. Die Berechnungen wurden mit Hilfe des Programms CNS 1.1 ("Crystallography and NMR System (CNS): A new software system for macromolecular structure determination". Brunger A.T., Adams P.D., Clore G.M., Delano W.L., Gros P., Grosse-Kunstleve R.W., Jiang J.-S., Kuszewski J., Nilges N., Pannu N.S., Read R.J., Rice L.M., Simosnson T., and Warren G.L. ACTA CRYST. D54, 905-921 (1998)) auf UNIX-basierten Rechnern durchgeführt.

Folgende experimentelle Daten waren Grundlagen für die Berechnungen:

- Abstandsinformationen, basierend auf der Zuordnung der NOESY-Spektren.
  Für die reduzierte Form konnten 1119 Abstandsrestraints bestimmt werden, für die oxidierte Form waren es 999 Restraints. Eine genaue Auflistung und ein Vergleich der Restraints für beide Formen wurde in Tabelle 2 zusammengestellt.
- Restraints für die Rückgrat-Diederwinkel $\phi$ und $\psi$, diese Restraints basieren auf einer statistischen Auswertung der chemischen Verschiebungen der C$\alpha$- und C$\beta$-Kohlenstoffatome für die einzelnen Reste (TALOS, (Cornilescu et al., J. Biomol. NMR 1999, 13, 289)).
- Wasserstoffbrückenbindungen; Donor-NH-Gruppen wurden durch Austausch des wässrigen Puffers gegen $D_2O$ bestimmt, Akzeptor-Sauerstoffatome konnten während mehrerer Runden der Strukturberechnung identifiziert werden.

Die Strukturberechnung wurde unter Verwendung eines CNS-Standard-Protokolls für Torsional Angle Dynamics durchgeführt. Die Parameter der Berechnung wurden wie folgt gesetzt:

Tabelle 2

| High Temperature annealing | |
|---|---|
| Start Temperature | 50000 |
| Number of Steps | 3000 |
| Van der Waals scale Factor | 0.1 |
| NOE scale Factor | 50 |
| Dihedral Angle Scale Factor | 100 |
| MD Timestep | 0.015 |
| **First Slow Cool Annealing** | |
| Temperature | 50000 |
| Number of Steps | 20000 |
| Van der Waals Scale Factor | 1 |
| NOE Scale Factor | 50 |
| Dihedral Angle Scale Factor | 100 |

(fortgesetzt)

| First Slow Cool Annealing | |
|---|---|
| MD Timestep | 0.015 |
| Temperature Step | 250 |
| **Second Slow Cool Annealing** | |
| Temperature | 3000 |
| Number of Steps | 10000 |
| Van der Waals Scale Factor | 1 |
| NOE Scale Factor | 50 |
| Dihedral Angle Scale Factor | 100 |
| MD Timestep | 0.005 |
| Temperature Step | 25 |
| **Final Minimization** | |
| NOE Scale Factor | 50 |
| Dihedral Angle Scale factor | 100 |
| Number of Steps | 200 |
| Number of Cycles | 10 |

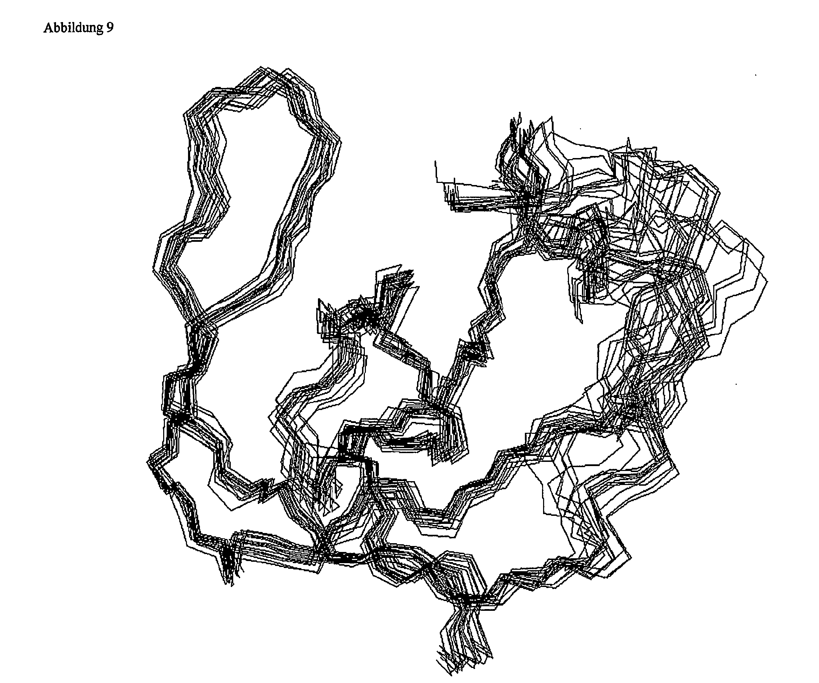

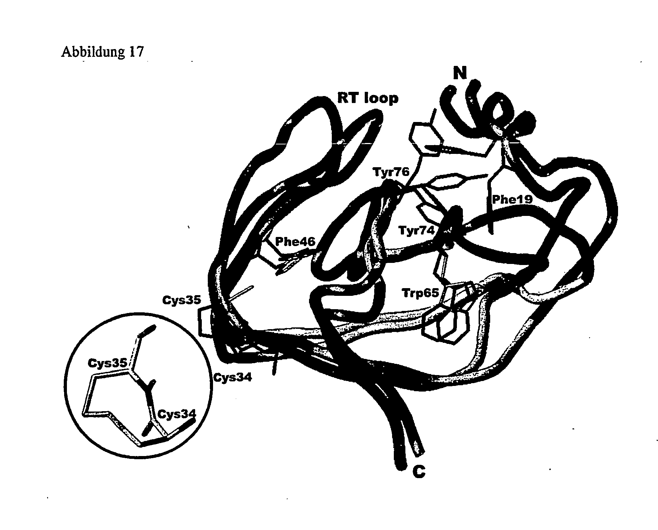

Zur Repräsentation der Lösungsstruktur der ADAP hSH3-1-Domäne wurden für beide Formen jeweils die 20 Strukturen mit der niedrigsten Gesamtenergie ausgewählt. Diese Ensembles sind in Abbildung 9 (reduziertes hSH3-1) und 10 (oxidiertes SH3-1) dargestellt. Ein Vergleich der Strukturen ist in Abbildung 17 gezeigt. Den gezeigten Strukturen liegen pdb-Dateien zugrunde, welche die Atomkoordinaten enthalten. Der Fachmann kann demgemäß aus der Abbildung die Atomkoordinaten automatisch herleiten.

Die beiden Ensembles können wie nachstehend näher charakterisiert werden (Tabelle 3):

Tabelle 3:

| | Reduzierte Form | Oxidierte Form |
|---|---|---|
| Abstandsrestraints | | |
| Gesamt | 1119 | 999 |
| "Long-Range" (> i,i+4) | 384 | 305 |
| Diederwinkel | 97 | 110 |
| Wasserstoffbrücken | 0 | 0 |
| Ramachandran-Statistik (%) | | |
| Bevorzugt | | 78.1 |
| Zusätzlich erlaubt | | 17.5 |
| Weiterhin erlaubt | | 2.6 |
| Verboten | | 1.8 |
| RMS-Daten [Å] | | |
| Sekundärstrukturelemente | | 0.29 |
| Geordnete Bereiche | | 0.41 |
| AS 12- 87 | | 0.66 |

Die Unterschiede in der Struktur wird durch folgendes Bsp. erläutert, in dem die Differenz (in Å) zwischen oxidierter und reduzierter Form durch Überlagerung zweier beispielhafter 3D-Strukturen ermittelt wurde (Tabelle 4).

Tabelle 4:

| Distanz der Ca-Atome in der Struktur von reduziertem und oxidiertem ADAP-SH3-1 | | | | | | | | | | |
|---|---|---|---|---|---|---|---|---|---|---|
| Oxidized | | | | | Reduced | | | | | Distance (Å) |
| Res. | N: | X | Y | Z | Res. | N: | X | Y | Z | |
| ASP | 59 | 4.718 | 14.227 | 2.374 | ASP | 59 | -3.628 | 15.013 | -0.888 | 9 |
| PRO | 61 | 5.219 | 19.339 | 6.728 | PRO | 61 | 0.068 | 13.593 | 3.521 | 8 |
| LYS | 38 | 10.314 | 3.783 | 16.198 | LYS | 38 | 7.478 | -1.467 | 15.887 | 6 |
| ASN | 60 | 3.547 | 17.556 | 3.794 | ASN | 60 | -1.865 | 16.576 | 2.1 | 6 |
| GLY | 39 | 7.743 | 5.279 | 13.845 | GLY | 39 | 4.758 | 1.159 | 15.589 | 5 |
| CYSS | 34 | 14.52 | -2.82 | 5.742 | CYS | 34 | 15.366 | 2.097 | 6.118 | 5 |
| GLY | 40 | 5.44 | 7.312 | 16.052 | GLY | 40 | 5.213 | 3.399 | 15.605 | 5 |
| LYS | 17 | -11.66 | 4.057 | 10.31 | LYS | 17 | -10.03 | 8.14 | 8.913 | 5 |
| LYS | 18 | -8.156 | 5.232 | 11.206 | LYS | 18 | -6.264 | 8.473 | 9.309 | 4 |
| GLU | 62 | 3.244 | 17.207 | 9.166 | GLU | 62 | 0.415 | 15.559 | 6.759 | 4 |
| PHE | 19 | -6.955 | 6.479 | 7.821 | PHE | 19 | -0.661 | 8.971 | 5.896 | 4 |
| LYS | 16 | -10.43 | 2.383 | 7.126 | LYS | 16 | -9.856 | 3.905 | 3.839 | 4 |
| GLN | 13 | -8.752 | -4.062 | 9.17 | GLN | 13 | -9.912 | -0.861 | 7.684 | 4 |
| GLU | 14 | -9.02 | -1.142 | 11.593 | GLU | 14 | -9.935 | 2.038 | 10.147 | 4 |
| THR | 58 | 1.5 | 13.099 | 0.694 | THR | 58 | -0.485 | 13.324 | -2.203 | 4 |
| LYS | 41 | 1.694 | 7.031 | 16.617 | LYS | 41 | 1.568 | 4.462 | 18.626 | 3 |
| GLN | 51 | 7.759 | -5.888 | 1.891 | GLN | 51 | 10.246 | -3.776 | 1.566 | 3 |
| VAL | 37 | 12.559 | 2.127 | 13.613 | VAL | 37 | 10.018 | 0.061 | 13.504 | 3 |
| GLY | 49 | 11.941 | -6.757 | 4.97 | GLY | 49 | 14.531 | -4.824 | 4.67 | 3 |
| LYS | 1 | -6.95 | -6.966 | 3.112 | LYS | 11 | -9.459 | -0.573 | 4.932 | 3 |
| PRO | 24 | -9.715 | 1.388 | -5.236 | PRO | 24 | -8.79 | 0.018 | -2.717 | 3 |
| LYS | 32 | 10.165 | -2.331 | 1.841 | LYS | 32 | 12.182 | -0.104 | 1.692 | 3 |
| ILE | 15 | -7.374 | 0.922 | 8.854 | ILE | 15 | -7.448 | 3.718 | 7.812 | 3 |
| GLN | 48 | 13.186 | -5.308 | 8.245 | GLN | 48 | 15.076 | -3.023 | 7.962 | 3 |
| CYSS | 35 | 15.483 | -1.339 | 9.111 | CYS | 35 | 14.985 | 1.38 | 9.833 | 3 |
| THR | 80 | 12.605 | 8.453 | 6.13 | THR | 80 | 11.226 | 8.791 | 8.492 | 3 |
| GLU | 50 | 8.246 | -6.211 | 5.649 | GLU | 50 | 10.798 | -5.471 | 4.925 | 3 |
| GLY | 23 | -12.33 | 3.67 | -3.649 | GLY | 23 | -11.31 | 2.537 | -1.784 | 2 |
| LEU | 21 | -9.22 | 5.128 | 2.425 | LEU | 21 | -8.345 | 6.977 | 1.445 | 2 |
| GLY | 63 | 1.987 | 14.411 | 6.936 | GLY | 63 | 4.147 | 14.922 | 6.956 | 2 |
| LYS | 78 | 7.862 | 9.66 | 7.806 | LYS | 78 | 6.045 | 9.135 | 8.855 | 2 |
| GLU | 83 | 13.999 | 3.652 | 1.253 | GLU | 83 | 12.736 | 3.154 | 1.316 | 2 |
| ILE | 52 | 5.563 | -2.802 | 2.217 | ILE | 52 | 7.127 | -1.65 | 2.024 | 2 |
| TYR | 76 | 2.2 | 6.277 | 8.58 | TYR | 76 | 0.617 | 5.49 | 8.064 | 2 |
| THR | 79 | 9.344 | 9.217 | 4.331 | THR | 79 | 8.442 | 8.954 | 5.906 | 2 |

(fortgesetzt)

| Distanz der Ca-Atome in der Struktur von reduziertem und oxidiertem ADAP-SH3-1 | | | | | | | | | | |
|---|---|---|---|---|---|---|---|---|---|---|
| Oxidized | | | | | Reduced | | | | | Distance (Å) |
| Res. | N: | X | Y | Z | Res. | N: | X | Y | Z | |
| LYS | 20 | -8.82 | 8.01 | 4.379 | LYS | 20 | -8.045 | 9.528 | 4.251 | 2 |
| ILE | 84 | 11.474 | 3.584 | -1.591 | ILE | 84 | 10.623 | 5.12 | -1.845 | 2 |
| LYS | 47 | 11.495 | -4.441 | 11.541 | LYS | 47 | 12.003 | -4.167 | 9.89 | 2 |
| ILE | 77 | 5.813 | 6.479 | 7.415 | ILE | 77 | 4.408 | 5.774 | 8.149 | 2 |
| ASN | 42 | -0.038 | 5.585 | 13.564 | ASN | 42 | 0.217 | 4.921 | 15.101 | 2 |
| GLY | 75 | 0.919 | 2.706 | 8.442 | GLY | 75 | 0.373 | 2.099 | 9.754 | 2 |
| ALA | 33 | 11.176 | -1.065 | 5.281 | ALA | 33 | 12.267 | -0.017 | 5.492 | 2 |
| ILE | 57 | 2.57 | 10.298 | -1.644 | ILE | 57 | 1.453 | 10.38 | -0.772 | 1 |
| ALA | 31 | 9.219 | 0.971 | 0.21 | ALA | 31 | 9.887 | 1.675 | -0.766 | 1 |
| THR | 22 | -11.75 | 5.511 | -0.387 | THR | 22 | -11.02 | 6.243 | -1.159 | 1 |
| SER | 45 | 5.635 | -0.784 | 12.861 | SER | 45 | 5.645 | -1.91 | 12.359 | 1 |
| GLN | 26 | -3.037 | 1.186 | -6.819 | GLN | 26 | -2.87 | 0.127 | -6.737 | 1 |
| ILE | 54 | 2.995 | 3.028 | -0.095 | ILE | 54 | 3.55 | 3.21 | -0.933 | 1 |
| VAL | 82 | 11.831 | 3.413 | 4.369 | VAL | 82 | 11.333 | 4.201 | 4.721 | 1 |
| SER | 73 | -1.044 | -3.403 | 7.478 | SER | 73 | -1.735 | -2.864 | 7.879 | 1 |
| PHE | 46 | 8.184 | -2.722 | 10.807 | PHE | 46 | 8.335 | -3.165 | 9.98 | 1 |
| ILE | 25 | -6.015 | 1.973 | -4.587 | ILE | 25 | -6.142 | 1.245 | -5.156 | 1 |
| ASP | 36 | 14.078 | -1.224 | 12.642 | ASP | 36 | 13.329 | -1.185 | 12.104 | 1 |
| ARG | 71 | -0.749 | -8.406 | 4.496 | ARG | 71 | -1.145 | -8.339 | 3.681 | 1 |
| GLU | 12 | -7.796 | -0.117 | 5.488 | GLU | 12 | -7.688 | -3.328 | 5.83 | 1 |
| ARG | 56 | -0.213 | 7.779 | -1.025 | ARG | 36 | -0.857 | 7.36 | -0.726 | 1 |
| GLU | 43 | 3.249 | 4.259 | 12.186 | GLU | 43 | 3.163 | 3.906 | 12.916 | 1 |
| ALA | 81 | 12.275 | 5.042 | 7.777 | ALA | 81 | 11.767 | 5.028 | 8.409 | 1 |
| LYS | 64 | 2.999 | 10.788 | 6.413 | LYS | 64 | 3.54 | 11.18 | 6.715 | 1 |
| LEU | 66 | 0.33 | 5.767 | 3.712 | LEU | 66 | 0.972 | 5.45 | 3.804 | 1 |
| GLY | 67 | 2.229 | 2.485 | 3.733 | GLY | 67 | 2.653 | 2.058 | 3.599 | 1 |
| GLU | 53 | 4.301 | -0.514 | -0.549 | GLU | 53 | 4.861 | -0.354 | -0.744 | 1 |
| GLY | 72 | -2.436 | -5.013 | 4.335 | GLY | 72 | -2.584 | -5.046 | 4.892 | 1 |
| LEU | 30 | 7.788 | -0.261 | -3.092 | LEU | 30 | 7.944 | -0.043 | -3.547 | 1 |
| TRP | 65 | 3.216 | 8.24 | 3.599 | TRP | 65 | 3.22 | 8.51 | 4.028 | 1 |
| TYR | 74 | -1.392 | 0.294 | 6.652 | TYR | 74 | -1.531 | 0.765 | 6.762 | 1 |
| ILE | 28 | 2.686 | 3.347 | -6.511 | ILE | 28 | 2.606 | 3.823 | -6.537 | 0 |
| LEU | 44 | 3.472 | 0.976 | 10.278 | LEU | 44 | 3.673 | 0.873 | 10.676 | 0 |
| ILE | 55 | -0.025 | 4.141 | -2.119 | ILE | 55 | -0.098 | 3.801 | -1.829 | 0 |
| THR | 69 | 2.748 | -4.48 | 4.279 | THR | 69 | 2.394 | -4.617 | 4.518 | 0 |
| VAL | 27 | 0.47 | 0.404 | -5.573 | VAL | 27 | 0.653 | 0.748 | -5.449 | 0 |

(fortgesetzt)

| Distanz der Ca-Atome in der Struktur von reduziertem und oxidiertem ADAP-SH3-1 | | | | | | | | | | |
|---|---|---|---|---|---|---|---|---|---|---|
| Oxidized | | | | | Reduced | | | | | Distance (Å) |
| Res. | N: | X | Y | Z | Res. | N: | X | Y | Z | |
| ALA | 70 | 2.195 | -7.627 | 2.216 | ALA | 70 | 2.413 | -7.806 | 2.446 | 0 |
| HIS | 29 | 6.166 | 2.329 | -5.358 | HIS | 29 | 6.062 | 2.519 | -5.638 | 0 |
| ARG | 68 | 1.052 | -1.122 | 3.716 | ARG | 68 | 0.838 | -1.277 | 3.579 | 0 |

Aus der Literatur ist bekannt, dass der Rückgratwinkel von vicinalen Cysteinen, die über eine Disulfidbrücke einen Achtringbilden, auch die cis-Konformation annehmen kann. Daher wurde im vorliegenden Fall die Strukturberechnung für die oxidierte Form der hSH3-1-Domäne sowohl mit einer vordefinierten trans- als auch cis-Bindung durchgeführt. Für die cis-Konformation ergibt sich bei gleichem Restraint-Satz und ansonsten identischen Bedingungen der Rechnung eine etwas höhere Gesamtenergie. Insbesondere sind im Bereich der Reste 33 bis 36 werden einige NOE-Restraints verletzt, die durch die trans-Konformation erfüllt werden können. Daher wird im weiteren von einer trans-Konformation dieser Bindung ausgegangen.

Mutagenisierung von Aminosäureresten in Bezug auf molekulare Bibliotheken:

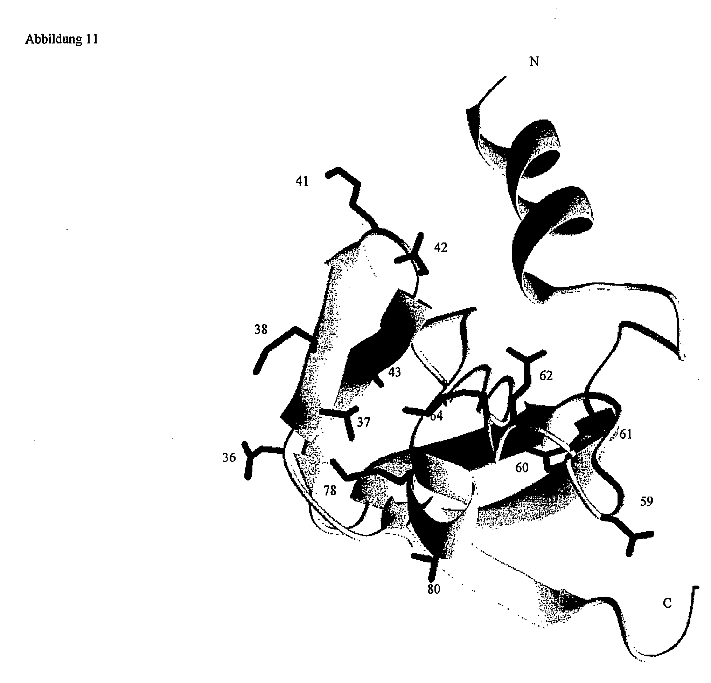

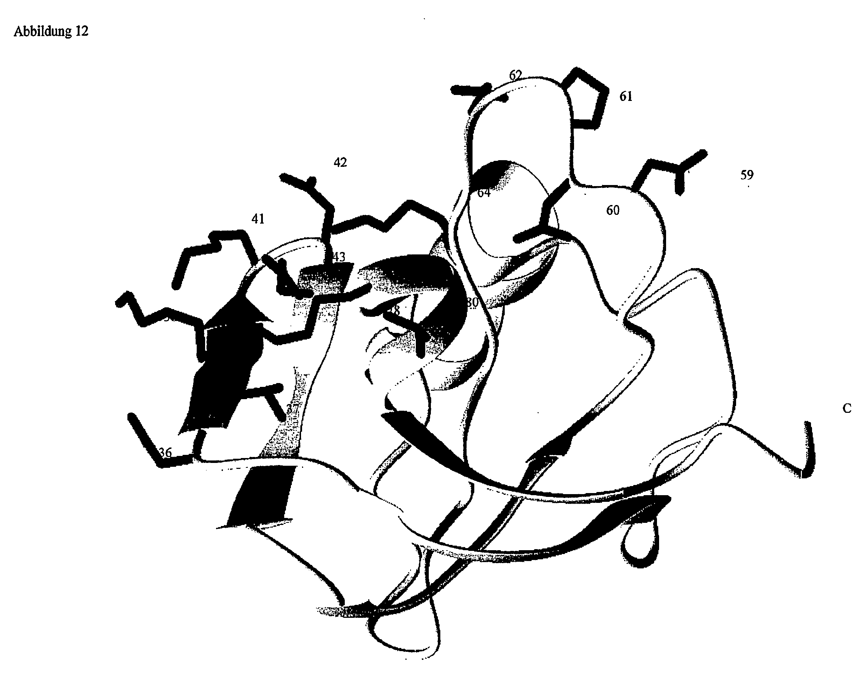

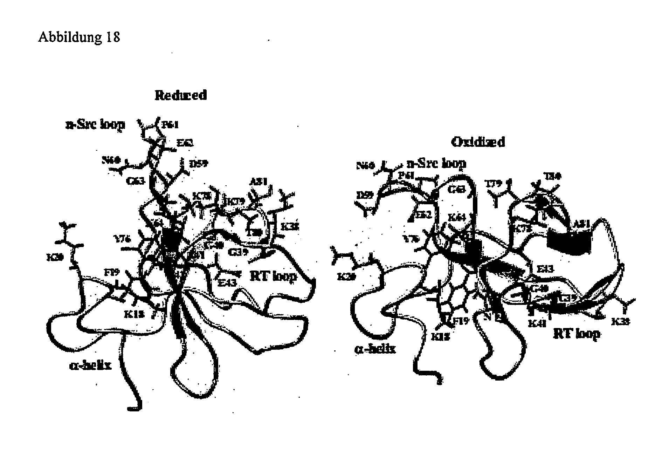

Auf der Basis des Strukturvergleichs wurden unter anderem folgende Aminosäuren ausgewählt, die einer Mutagenese unterzogen werden: 18, 20, 32, 38 bis 43, 59 bis 64, 78, 79, 80 und/oder 81 (Abbildung 11, 12 und 18). Die Aminosäuren an diesen Positionen erfüllen zumindest zwei der folgenden Kriterien:

1. Sie sind weitgehend Lösungsmittelexponiert, d.h. sie behindern aller Voraussicht nach nicht die Faltung des Proteins.
2. Sie sind in relativer räumlicher Nähe zu dem Cystein-Paar (< 20Å) und zeigen eine signifikante Änderung der NH-Gruppen-Resonanzen zwischen reduzierter und oxidierter Form. Dies gewährleistet die Einbindung der Cysteine in die möglichen Bindungsepitope, und damit ein differentiell unterschiedliches Verhalten der beiden Redox-Formen.
3. Zusammen genommen und unter Berücksichtigung der beiden Redox-Cysteine bilden diese Aminosäuren ein oberflächenexponiertes Epitop mit einer großen Anzahl möglicher Bindungsmodi.

Das Protokoll für die Randomisierung wird im Moment bearbeitet und es wird nach folgender Strategie vorgegangen: Einführung der randomisierten Stellen durch eine ZweiSchritt-Gen-Assemblierung oder eine Ein-Schritt-Gen-Synthese mit Hilfe der PCR-Reaktion. Die randomisierten Oligonukleotide werden dabei durch die Triplets NNK oder durch Trinukleotide (N: jede Base möglich, K: G oder T) codiert, was einerseits den Einbau aller 20 Aminosäuren erlaubt, andererseits aber nur das Auftreten des Amber-Stop-Kodons (im Falle des NNK Codes) erlaubt. Letzteres kann in E.coli XL1Blue supprimiert werden, da in diesem Stamm TAG als Glutamat gelesen wird. Bei Verwendung der Trinukleotide wird gezielt nur ein Satz von 19 Codons (keine Stop-Codons, kein Cystein) verwendet.
Im Falle der Verwendung des Phage Display soll die molekulare Bibliothek entweder als GenIII-oder GenVIII-Fusionsprotein exprimiert werden und die Phagen bzw. Phagemide nach Standard-Protokollen gewonnen werden. Insbesondere wurden randomisierte PCR-Fragmente über eine Zwei-Fragment-Assemblierung in die Vektoren pAK100 und pAK200 über SfiI-Schnittstellen eingefügt (Krebber et al., J. Immunol. Meth. 201, 35-55 (1997). Dabei wurden die Positionen 18, 20, 32, 38 bis 43, 59 bis 64, 78, 79, 80 und/oder 81 durch NNK-Mutagenese randomisiert und das entsprechende PCR-Fragment in die Vektoren kloniert. Als Beispiel für die Konstruktion einer Bibliothek wurde wie folgt vorgegangen:

**Primers used for construction of the library:**

```
pAKfyb1S Dir: 60-mer   (vorhanden !!!)
5'-CAGACGGCCCAGCCGGCCATGGCCGACTACAAAGACGAAAAGAAAGAACAAGAAATAAAG-3'
                                                   47.5-60°C
```

```
fyb1Lib1d : 5'-
GAAAAGAAAGAACAAGAAATAAAGAAGNNKTTTNNKCTAACAGGCCCTATTCAAG-3'   55-mer
60°C                                  50.6°C
```

```
fyb1Lib2'r:  68-mer
5'-
CTCGATCTGCTCCCCCTGCTTGAAGCTCAGMNNMNNMNNMNNMNNMNNMNNGACATCACAACAAGCTTTTG-
3'
```

```
fyb1Lib3'd:  69-mer
5'-
GCAGGGGGAGCAGATCGAGATCATCCGCATCACANNKNNKNNKNNKNNKNNKTGGTTGGGCAGAACAGC-
3'
```

```
fyb1Lib4r : 5'-GAATCATAGTCAATCTCTACMNNMNNMNNMNNAATATAGCCATATGAACCCC-3'
 52-mer                   54°C                             56°C
```

```
fyb1Lib5r : 5'-GTGATGCGGATGATTTCAATTTGCTCTCCTTGCTTGAAGCTCAG-3'  44-mer
```

```
fyb1Lib6r : 38-mer (reverse for the short variant)
5'-CCCTCGGCCCCCGAGGCCGAATCATAGTCAATCTCTAC-3'
```

**Short form 5Mutant (aa 494-573):**

```
                                               FLAG
                    A  Q  P  A  M  A  D  Y  K  D  E  K  K  E  Q
                        SfiI
pAKfyb1S Dir:5'-CAGACGGCCCAGCCGGCCATGGCCGACTACAAAGACGAAAAGAAAGAACAA->
                               fyb1Lib1d : 5'-GAAAAGAAAGAACAA->
1442  GAAAAGAAGAGGTTAGAGCTGGAGAAAAAGGAACAGAAAGAGAAAGAAAAGAAAGAACAA

  501    E  I  K  K ] K18 F  K20 L  T  G  P  I  Q  V  I  H  L  A  K  A
         ->GAAATAAAG-3' 60°C
         ->GAAATAAAGAAGNNKTTTNNKCTAACAGGCCCTATTCAAG-3' 56°C  55-mer
   1502  GAAATAAAGAAGAAATTTAAACTAACAGGCCCTATTCAAGTCATCCATCTTGCAAAAGCT
                                              fyb1Lib2'r: 3'-CAAAAGCT<-

  521    C  C  D  V  K38 G  G  K  N  E43 L  S  F  K  Q  G  E  Q  I  E
                                    fyb1Lib3'd: 5'-GCAGGGGGAGCAGATCGAG->
   1562  TGTTGTGATGTCAAAGGAGGAAAGAATGAACTGAGCTTCAAGCAGGGGGAGCAGATCGAG
        <-TGTTGTGATGTCNNKNNKNNKNNKNNKNNKCTGAGCTTCAAGCAGGGGGAGCAGATCGAG-5'

         I  I  R  I  T  D59 N  P  E  G  K64 W  L  G  R  T  A  R  G  S
       ->ATCATCCGCATCACANNKNNKNNKNNKNNKNNKTGGTTGGGCAGAACAGC-3'
   1622  ATCATCCGCATCACAGACAACCCAGAAGGAAAATGGTTGGGCAGAACAGCAAGGGGTTCA
                                              56°C fyb1Lib4r:3'-GGGGTTCA<-

  561    Y  G  Y  I  K78 T  T  A81 V  E  I  D  Y  D  S  A  S  G  A  E
   1682  TATGGCTATATTAAAACAACTGCTGTAGAGATTGACTATGATTCTTTGAAACTGAAAAAA
        <-TATGGCTATATTNNKNNKNNKNNKNNKGTAGAGATTGACTATGATTC-5' 54°C
               54°C  fyb1Lib6r:5'-GTAGAGATTGACTATGATTCGGCCTCGGGGGCCGAGGG-
         3'
```

SfiI

Library construction :

PCR1 :

PCR2 :

NNK-randomization:

| N: | NNK | a-a | N: | NNK | a-a | N: | NNK | a-a |
|----|-----|-----|----|-----|-----|----|-----|-----|
| 1 | AAG | K | 12 | CCT | P | 23 | GTG | V |
| 2 | AAT | N | 13 | CGG | R | 24 | GTT | V |
| 3 | ACG | T | 14 | CGT | R | 25 | TAG | - |
| 4 | ACT | T | 15 | CTG | L | 26 | TAT | Y |
| 5 | AGG | R | 16 | CTT | L | 27 | TCG | S |
| 6 | AGT | S | 17 | GAG | E | 28 | TCT | S |
| 7 | ATG | M | 18 | GAT | D | 29 | TGG | W |
| 8 | ATT | I | 19 | GCG | A | 30 | TGT | C |
| 9 | CAG | Q | 20 | GCT | A | 31 | TTG | L |
| 10 | CAT | H | 21 | GGG | G | 32 | TTT | F |
| 11 | CCG | P | 22 | GGT | G | | | |

Table 2. IUPAC ambiguity codes

| Code | Description |
|------|-------------|
| M | AC |
| R | AG |
| W | AT |
| S | CG |
| Y | CT |
| K | GT |
| V | ACG |
| H | ACT |
| D | AGT |
| B | CGT |
| N | ACGT |

Alternative Display-Methoden sind ebenfalls möglich, z.B. das Ribosome Display, einem in vitro-Display-Verfahren, bei dem eine deutlich größere Vielfalt in der molekularen Bibliothek als mit dem Phage Display erzielt werden kann (Hanes & Plückthun, Proc. Nat. Acad. Sci. 94, 4937-4942 (1997); Roberts & Szostak 94, 12297-12302 (1997)).

Die erhaltenen molekularen Bibliotheken werden ähnlich wie Antikörper-Bibliotheken interaktionsrelevante Proteinvarianten enthalten, die eine Vielzahl unterschiedlicher Zielmolekülen erkennen können. Als Zielstrukturen werden eingesetzt: Proteine, DNA, Lipide, organische Moleküle (Fluorophore, insbesondere in der Fluoreszenzmikroskopie eingesetzte Substanzen), die an ein festes Trägermaterial gebunden werden (entweder kovalente Kopplung, oder affinitätsbasiert). Für das Panning werden wir in vitro-Bedingungen wählen, die entweder die oxidierte oder die reduzierte Form der Proteine einstellen. Konkret wird z.B. folgendes Protokoll angewendet (am Bsp. eines GST-Fusionsproteins): 100 mg des GST-Fusionsproteins, die an Glutathion-Sepharose 4B gebunden sind werden mit $10^8$-$10^{12}$ infektiösen Partikel bei 4 °C über Nacht entweder in Anwesenheit von 2 mM $H_2O_2$ oder 2mM DTT (bzw. β-Mercapto-Ethanol) in einem Volumen von 400 μl inkubiert. Anschließend wird für den mit $H_2O_2$-inkubierten Ansatz einige Male mit PBS, 0.1 % Tween20 und 2 mM $H_2O_2$ gewaschen, und anschließend mit PBS, 0.1 % Tween20 und 2 mM DTT (β-ME) eluiert. Bei diesem Schritt werden die Phagen freigesetzt, die nur in der oxidierten Form an das GST-Zielprotein binden. Anschließend können die noch gebundenen Phagen mit 100 mM glycine HCl, pH 2.2 eluiert werden. Für den mit DTT inkubierten Ansatz wird mit PBS, 0.1% Tween und 2 mM DTT (β-ME) gewaschen, bevor in PBS mit 2mM $H_2O_2$ eluiert wird. In diesem Schritt werden diesmal nur die Phagen eluiert, die ausschließlich in der reduzierten Form an das Ziel-Protein binden. Die übrigen bindenden Phagen werden im nächsten Schritt mit 100 mM glycine HCl, pH 2.2 eluiert. Alternativ kann eine "Preclearing"-Methode angewendet werden, bei der die Phagen unter oxidierenden (reduzierenden) Bedingungen vorinkubiert werden, die GST-beads mit unter diesen Bedingungen bindenden Phagen abzentrifugiert werden, um dann nach entsprechenden Wasch-Schritten unter reduzierenden (oxidierenden) Bedingungen inkubiert und bindende Phagen anschließend (nach weiteren Wasch-Schritten) mit 100 mM glycine HCl, pH 2.2 eluiert werden.

Gebundene und weiter zu charakterisierende Phagen werden in jedem der oben geschilderten Fälle zur Infektion von z.B. E.coli XL1-Blue-Zellen benutzt und die Phagen werden über Nacht in 2xYT-Medium in der Anwesenheit des Selektionsmarkers (Kanamycin, Tetracyclin, oder Ampicilin, je nach verwendetem Vektor) bei 37°C, 200 rpm amplifiziert. Nach mehreren solcher Selektionsrunden können positive Klone sequenziert und anschließend für weitere Experimente über eine geeignete Klonierungskasette in einen Expressionsvektor (pTFT74, pET28d modified) ligiert werden. Targtet-bindende Proteine der Bibliothek können anschließend in E.coli oder Insektenzellen exprimiert und über die vorhandenen Tags (His$_6$-tag, GST-Tag, Strep-Tag), sowie einer sich anschließenden Ionenaustausch- oder Gelfiltrations-Säule gereinigt werden.

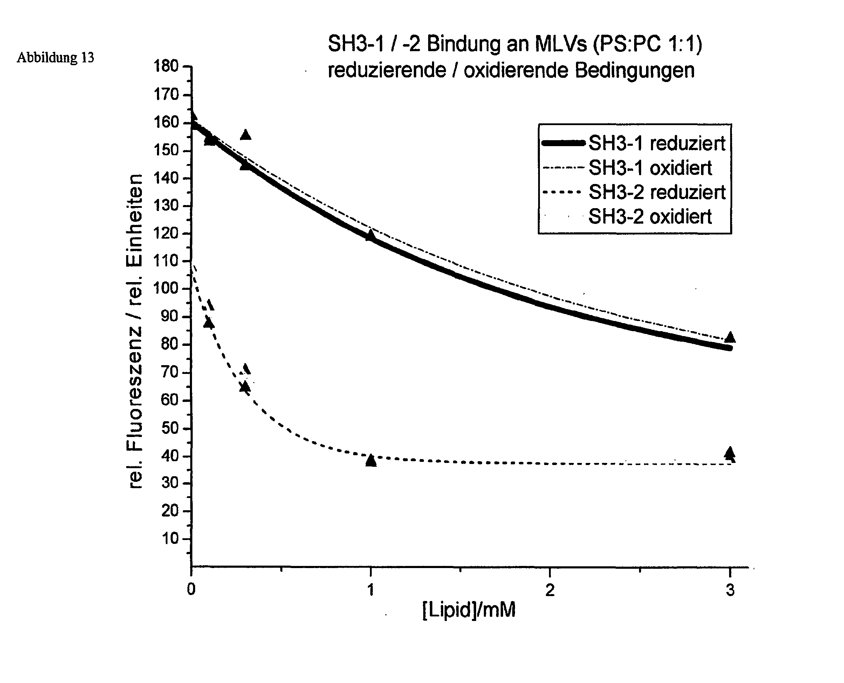

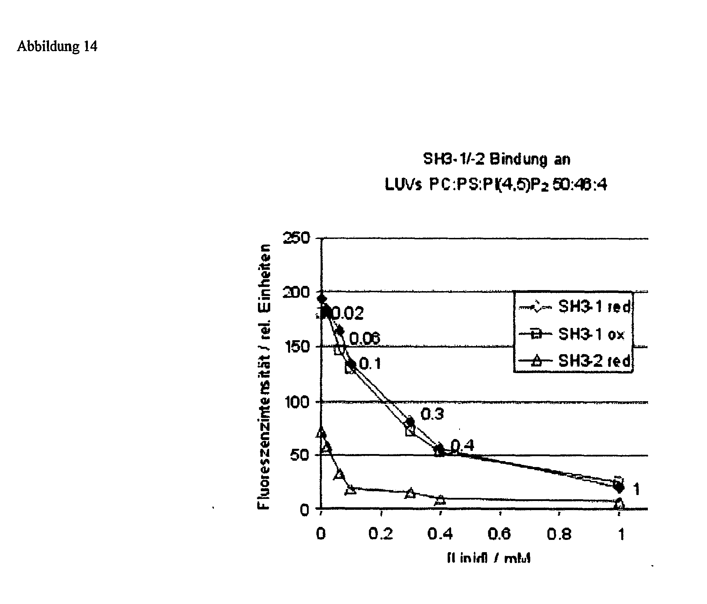

Lipid-Bindung: Die hSH3-1-Domäne bindet im Lipid-Overlay-Assay (Echelon) und im Liposom-Versuch an saure Lipide innerhalb von Lipid-Membranen (Liposomen) (Abbildung 13, 14). Basische Reste, die auf der Oberfläche der Domäne lokalisiert sind (Lys15, Lys16, Lys18, Lys20, Lys38, Lys41) sind in Anbetracht der Struktur für die Bindung zumindest mitverantwortlich. Auch aus Homolgiebetrachtungen zur hSH3-2-Domäne lassen sich die wahrscheinlichen Strukturelemente, die für die Lipidbindung verantwortlich sind, erschließen. Die Lipidbindungseigenschaften sind für zukünftige Experimente, welche diese Bindungsfunktion modifizieren, erfindungsrelevant. Eine weiterer möglicher Ansatz besteht in der Verknüpfung von Protein- und LipidBindungseigenschaften, die im Prinzip einen kooperativen, möglicherweise redox-regulierten Schalter darstellen können.

Für die sequenzhomologe ADAP hSH3-2-Domäne wurden die Bindungseigenschaften untersucht. Es konnte gezeigt werden, dass die ADAP hSH3-2-Domäne eine Lipid-Interaktionsdomäne ist (Abbildung 13,14). Sie bindet saure Lipide wie z. B. Phosphatidylinositole. Positiv geladene Reste an der Oberfläche der Domäne binden an polyvalente Lipide wie PIP$_2$ oder PIP$_3$ bevorzugt gegenüber monovalenten Posphatidylserin-Phospholipiden. Die N-terminale Helix des hSH3-2-Folds ist für diese Wechselwirkung notwendig. Außerdem tragen basische Reste des SH3-Scaffolds zur Lipid-Bindung bei.

Phosphorylierungen:

In einem in vitro-Kinase-Assay konnten für das Protein ADAP eine Reihe von Phosphorylierungsstellen identifiziert werden. Dazu gehört Tyr 86 (Seq. ID 1) in der hSH3-1-Domäne. Die $^{15}$N-HSQC-Spektren der phosphorylierten und unphosphorylierten Form der Domäne unterscheiden sich signifikant voneinander, was die Möglichkeit einer Strukturänderung impliziert.

Gezielte Herstellung Redox-sensitiver Muteine:

Die Muteine mit den Mutationen K32E, K32Q, V37K, Q48K und A81E wurden durch Orts-spezifische Mutagenese erzeugt und konnten in E.coli analog SEQ ID Nr.1 exprimiert werden. Einige mg des jeweils $^{15}$N-markierten Muteins wurden einer NMR-Redox-Titration analog der für SEQ ID Nr.1 beschriebenen Methode bestimmt. Die jeweiligen Redox-Titrationen sind in Abbildung 15 und 16 gezeigt. Ein erster Fit der Werte für das Redox-Potential ergibt Werte von -201 und -209 mV für die K32E bzw. A81E-Mutante. Es konnte also gezeigt werden, dass stabile Muteine mit leicht unterschiedlichem Redox-Potential erzeugt werden können.

Weitere Proteine mit CC-Motiven, die potentiell Redoxreguliert sind:

Aus der Datenbank der humanen Proteine der Swiss-Prot-Datenbank wurden alle Protein mit CC-Motiven, die in einer über das SMART-Domänen-Annotations-Programm definierten Domäne lagen und nicht mehr als 4 weitere Cysteine besitzen ausgewählt.

## Patentansprüche

1. Polypeptid umfassend ein Cys-Cys-Motiv, aufweisend ein reversibel einstellbares Redox-Potential im Bereich von -400 mV bis +200 mV, einsetzbar insbesondere zur Detektion von Redox-abhängigen Protein-Protein- oder Protein-Lipid-Wechselwirkungen und/oder zur Detektion von Molekülen, die in Abhängigkeit vom Redox-Potential von dem Polypeptid gebunden werden können, ausgewählt aus der Gruppe bestehend aus einem Polypeptid gemäß SEQ ID Nr. 1 und einem Polypeptid gemäß SEQ ID Nr. 1 wobei die Positionen 18, 20, 32, 38 bis 43, 48, 50, 59 bis 64, 78, 79, 80 und/oder 81, insbesondere 32, 48 und/oder 81, durch eine der 20 natürlichen Aminosäuren ersetzt sind, ausgewählt aus der Gruppe umfassend Gly, Ala, Val, Leu, Ile, Phe, Tyr, Trp, Pro, Asp, Glu, Asn, Gln, Ser, Thr, Cys, Met, Lys, Arg oder His.

2. Polypeptid-Bibliothek umfassend Polypeptide nach Anspruch 1.

3. Protein-Erkennungsdomäne umfassend ein Polypeptid nach Anspruch 1.

4. Verwendung eines Polypeptides gemäß Anspruch 1 zur randomisierten oder zufälligen in-vitro Mutagenese.

5. Verwendung nach dem vorhergehenden Anspruch
   **dadurch gekennzeichnet, dass**
   die Mutagenese mittels Phage-Display, mittels kombinatorischer Mutagenese, mittels in-vitro Systemen zur Proteinbiosynthese oder als gesteuerte Evolution und/oder kombinatorische Methode auf Nukleinsäurebasis erfolgen.

6. Verfahren zur Detektion und/oder Gewinnung von an Targetmolekülen bindenden Polypeptiden in-vitro umfassend folgende Schritte

   a) Immobilisierung eines potentiellen Targetmoleküls auf einem Träger,
   b) Inkontaktbringen von Peptiden einer Polypeptid-Bibliothek nach Anspruch 2 mit den immobilisierten Targetmolekülen,
   c) Eliminieren der nicht an die Targetmoleküle gebundenen Polypeptide,
   d) Eluieren der gebundenen Polypeptide unter stringenten Bedingungen, wobei die bindenden Polypeptide gewonnen werden.

7. Verfahren nach einem der vorhergehenden Ansprüche,
   **dadurch gekennzeichnet, dass**
   das detektierte bindende Polypeptid mit einem pharmazeutisch akzeptablen Träger formuliert wird.

8. Verfahren zur Herstellung einer pharmazeutischen Formulierung umfassend das Verfahren gemäß Anspruch 6 und weiterhin das Inkontaktbringen der identifizierten bindenden Polypeptide mit einem pharmazeutisch akzeptablen Träger.

9. Verwendung eines Polypeptids gemäß Anspruch 1 zusammen mit einer Membran-Translokationssequenz oder einem GFP-Konstrukt zur Identifizierung von Redox-abhängigen Protein-Protein- oder Protein-Lipid-Wechselwirkungen.

10. Verfahren zur in-vitro Detektion von Targetmolekülen, deren Inhibition Redox-Potential-abhängig schaltbar ist, umfassend die Schritte:

   - Bereitstellung einer Probe umfassend mindestens einen Kandidaten des Targetmoleküls,
   - Inkontaktbringen eines Polypeptids gemäß Anspruch 1 oder der Polypeptid-Bibliothek nach Anspruch 2 mit der Probe und
   - Detektion der Wechselwirkung, insbesondere der Bindung zwischen dem Targetmolekül und dem Polypeptid.

11. Verfahren nach einem der vorhergehenden Ansprüche, weiterhin umfassend die Formulierung des detektierten Targetmoleküls in einer pharmazeutisch akzeptablen Form.

**Claims**

1. A polypeptide comprising a Cys-Cys motif having a reversibly adjustable redox potential in the range of from -400 mV to +200 mV, which can be used particularly for detecting redox-dependent protein-protein or protein-lipid interactions and/or for detecting molecules which can be bound by the polypeptide in dependence on the redox potential, selected from the group consisting of a polypeptide in accordance with SEQ ID NO: 1 and a polypeptide in accordance with SEQ ID NO: 1 wherein the positions 18, 20, 32, 38 to 43, 48, 50, 59 to 64, 78, 79, 80 and/or 81, particularly 32, 48 and/or 81, are replaced with one of the twenty natural amino acids selected from the group comprising Gly, Ala, Val, Leu, Ile, Phe, Tyr, Trp, Pro, Asp, Glu, Asn, Gln, Ser, Thr, Cys, Met, Lys, Arg or His.

2. A polypeptide library comprising polypeptides according to claim 1.

3. A protein recognition domain comprising a polypeptide as claimed in claim 1.

4. Use of a polypeptide as claimed in any claim 1 for randomized or random *in vitro* mutagenesis.

5. The use according to the preceding claim,
   **characterized in that**
   said mutagenesis is effected using phage display, combinatorial mutagenesis, *in vitro* systems for protein biosynthesis, or as a controlled evolution and/or combinatorial method on a nucleic acid basis.

6. A method of detecting and/or obtaining polypeptides binding to target molecules *in vitro,* comprising the following steps:

   a) immobilizing a potential target molecule on a carrier,
   b) contacting peptides of the polypeptide library according to claim 2 with the immobilized target molecules,
   c) eliminating the polypeptides not bound to the target molecules,
   d) eluting the bound polypeptides under stringent conditions, thereby obtaining the binding polypeptides.

7. The method according to any of the preceding claims,
   **characterized in that**
   the detected binding polypeptide is formulated with a pharmaceutically acceptable carrier.

8. A method for the production of a pharmaceutical formulation, comprising the method according to claim 6 and, in addition, contacting the identified binding polypeptides with a pharmaceutically acceptable carrier.

9. Use of a polypeptide as claimed in claim 1, together with a membrane translocation sequence or a GFP construct for the identification of redox-dependent protein-protein or protein-lipid interactions.

10. A method for the *in vitro* detection of target molecules whose inhibition is switchable in dependence on the redox potential, comprising the steps of:

- providing a sample comprising at least one target molecule candidate;
- contacting a polypeptide as claimed in claim 1 or the polypeptide library as claimed in claim 2 with the sample; and
- detecting the interaction, particularly binding between the target molecule and the polypeptide.

11. The method according to any of the preceding claims, further comprising formulating the detected target molecule in a pharmaceutically acceptable form.

**Revendications**

1. Polypeptide comprenant un motif Cys-Cys, présentant un potentiel redox variable de façon réversible dans une gamme de - 400 mV à + 200 mV, utilisable notamment pour détecter des interactions protéine-protéine ou protéine-lipide en fonction du potentiel redox et/ou pour détecter des molécules qui peuvent être liées par le polypeptide en fonction du potentiel redox, choisi parmi le groupe comprenant un polypeptide correspondant au SEQ ID n°1 et un polypeptide correspondant au SEQ ID n° 1 dans lequel les positions 18, 20, 32, 38 à 43, 48, 50, 59 à 64, 78, 79, 80, et/ou 81, notamment 32, 48, et/ou 81 sont remplacées par un des 20 acides aminés naturels choisis parmi le groupe comprenant Gly, Ala, Val, Leu, Ile, Phe, Tyr, Trp, Pro, Asp, Glu, Asn, Gln, Ser, Thr, Cys, Met, Lys, Arg ou His.

2. Banque de polypeptides comprenant le polypeptide selon la revendication 1.

3. Domaine de reconnaissance de protéine comprenant un polypeptide selon la revendication 1.

4. Utilisation d'un polypeptide selon la revendication 1 pour une mutagenèse in-vitro randomisée ou aléatoire.

5. Utilisation selon la revendication précédente,
   **caractérisée en ce que**
   la mutagenèse s'effectue au moyen d'une présentation par phage, au moyen d'une mutagenèse combinatoire, au moyen de systèmes in-vitro permettant la biosynthèse de protéines ou à titre d'évolution contrôlée et/ou de méthode combinatoire à base d'acide nucléique.

6. Procédé de détection et/ou de préparation de polypeptides se liant à des molécules cibles in-vitro comprenant les étapes suivantes :

   a) immobilisation d'une molécule cible potentielle sur un support,
   b) mise en contact de peptides d'une banque de polypeptides selon la revendication 2 avec les molécules cibles immobilisées,
   c) élimination des polypeptides non liés aux molécules cible,
   d) élution des polypeptides liés dans des conditions stringentes, dans laquelle les polypeptides liants sont obtenus.

7. Procédé selon l'une quelconque des revendications précédentes,
   **caractérisé en ce que**
   le polypeptide liant détecté est formulé avec un support pharmaceutiquement acceptable.

8. Procédé de préparation d'une formulation pharmaceutique comprenant le procédé selon la revendication 6 et en plus la mise en contact des polypeptides liants identifiés avec un support pharmaceutiquement acceptable.

9. Utilisation d'un polypeptide selon la revendication 1 avec une séquence de translocation de membrane ou un produit de synthèse GFP pour identifier des interactions protéine-protéine ou protéine-lipide en fonction du potentiel redox.

10. Procédé de détection in-vitro de molécules cibles, dont l'inhibition est commutable en fonction du potentiel redox, comprenant les étapes suivantes :

   - préparation d'une sonde comprenant au moins un candidat de la molécule cible,

- mise en contact d'un polypeptide selon la revendication 1 ou de la banque de polypeptides selon la revendication 2 avec la sonde et
- détection de l'interaction, notamment de la liaison entre la molécule cible et le polypeptide.

11. Procédé selon l'une quelconque des revendications précédentes, comprenant en plus la formulation de la molécule cible détectée dans une forme pharmaceutiquement acceptable.

EP 1 856 144 B1

Abbildung 1

73

Abbildung 2

Abbildung 3

EP 1 856 144 B1

Abbildung 5

Abbildung 6

Abbildung 7

## SH3-1, red.

EP 1 856 144 B1

Abbildung 8

## SH3-1, oxid.

EP 1 856 144 B1

Abbildung 9

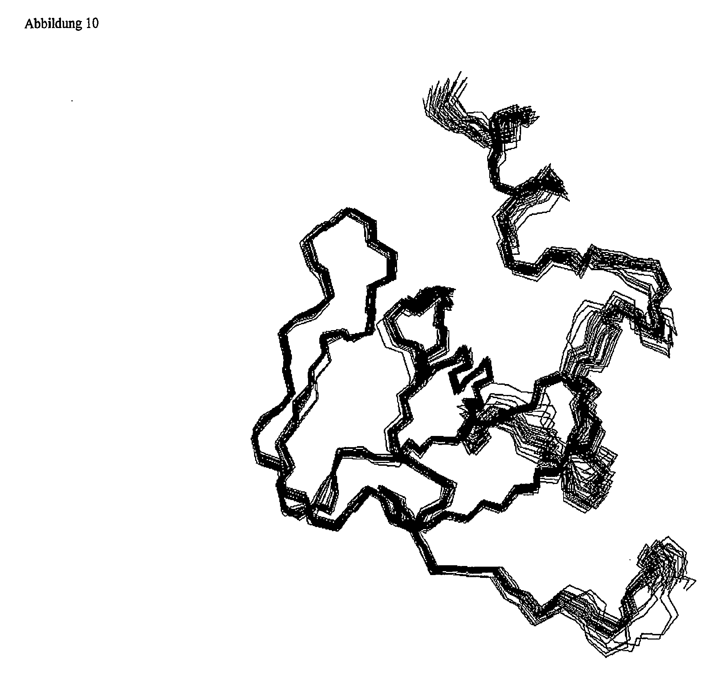

Abbildung 10

Abbildung 11

83

Abbildung 12

Abbildung 13

SH3-1 / -2 Bindung an MLVs (PS:PC 1:1)
reduzierende / oxidierende Bedingungen

Abbildung 14

EP 1 856 144 B1

SH3-1/-2 Bindung an
LUVs PC:PS:PI(4,5)P$_2$ 50:46:4

Abbildung 15

EP 1 856 144 B1

Abbildung 16

Abbildung 17

Abbildung 18

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 173494 A
- WO 8601533 A
- US 5283317 A
- WO 9410300 A, Brent

**In der Beschreibung aufgeführte Nicht-Patentliteratur**

- **Bowie et al.** *Science,* 1990, vol. 247, 1306-1310
- Current Protocols in Immunology. John Wiley & Sons, 1994
- **Ronald H. ; Hoess.** Protein Design and Phage Display. *Chem. Rev.,* 2001, vol. 101, 3205-3218
- **Hanes ; Plückthun.** *Proc. Nat. Acad. Sci.,* 1997, vol. 94, 4937-4942
- **Roberts ; Szostak.** *PROC. NAT. ACAD. SCI.,* 1997, vol. 94, 12297-12302
- **Behrens et al.** *Curr Gene Ther.,* Oktober 2003, vol. 3 (5), 486-94
- **Rojas et al.** *Nat Biotechnol.,* 1998, vol. 16, 370-375
- **Ronald H. Hoess.** Protein Design and Phage Display. *Chem. Rev.,* 2001, vol. 101, 3205-3218
- **Zervos et al.** *Cell,* 1993, vol. 72, 223-232
- **Madura et al.** *J. Biol. Chem.,* 1993, vol. 268, 12046-12054
- **Bartel et al.** *Biotechniques,* 1993, vol. 14, 920-924
- **Iwabuchi et al.** *Oncogene,* 1993, vol. 8, 1693-1696
- **Spence et al.** *J. Am. Chem. Soc.,* 2004, vol. 126, 15287-15294
- **Lowery et al.** *Angew. Chem.,* 2004, vol. 43, 6320-6322
- **Grzesiek et al.** *J. Magn. Res.,* 1992, vol. 99, 201
- **Grzesiek et al.** *J. Am. Chem. Soc.,* 1992, vol. 114, 6291
- **P. Kraulis.** ANSIG: A Program for the Assignment of Protein 1H 2D NMR spectra by Interactive Graphics. *J. Magn. Reson.,* 1989, vol. 24, 627-633
- **Hajduk et al.** *J. Med. Chem.,* 1997, vol. 40, 3144
- **Farrow et al.** *Biochemistry,* 1997, vol. 36, 2390
- **Gryk et al.** *J. Mol. Biol.,* 1998, vol. 280, 879
- **Brunger A.T. ; Adams P.D. ; Clore G.M. ; Delano W.L. ; Gros P. ; Grosse-Kunstleve R.W. ; Jiang J.-S. ; Kuszewski J. ; Nilges N. ; Pannu N.S.** Crystallography and NMR System (CNS): A new software system for macromolecular structure determination. *ACTA CRYST,* 1998, vol. D54, 905-921
- **Cornilescu et al.** *J. Biomol. NMR,* 1999, vol. 13, 289
- **Krebber et al.** *J. Immunol. Meth.,* 1997, vol. 201, 35-55